# EUROPEAN PATENT SPECIFICATION

(11) **EP 4 081 242 B1**
(45) Date of publication and mention of the grant of the patent: **10.09.2025**
(21) Application number: 20842695.7
(22) Date of filing: 18.12.2020
(51) Int. Cl.: A61K 38/25, G01N 33/50, A61B 5/00, A61K 45/06, A61B 5/021, A61B 5/029, A61B 5/02, A61P 9/04

(54) **TREATMENT OF ACUTE HEART FAILURE**
BEHANDLUNG VON AKUTEM HERZVERSAGEN
TRAITEMENT DE L'INSUFFISANCE CARDIAQUE AIGUË

(30) Priority: 19.12.2019 GB 201918853
(43) Date of publication of application: 02.11.2022
(73) Proprietor: ANACARDIO R&D AB, 171 65 Solna (SE)
(72) Inventor: LUND, Lars H., 11437 Stockholm (SE)
(74) Representative: AWA Sweden AB
(86) International application number: PCT/EP2020/086960
(87) International publication number: WO 2021/123119

(56) References cited:
- EP-A1- 1 297 839
- BEDENDI IVANO ET AL: "Cardiac effects of ghrelin and its endogenous derivatives des-octanoyl ghrelin and des-Gln14-ghrelin", EUROPEAN JOURNAL OF PHARMACOLOGY, vol. 476, no. 1-2, 27 October 2021 (2021-10-27), NL, pages 87 - 95, XP055855329, ISSN: 0014-2999, Retrieved from the Internet <URL:https://iris.unito.it/retrieve/handle/2318/125949/18483/ghrelin.pdf> DOI: 10.1016/S0014-2999(03)02083-1
- PEI XIAO M ET AL: "Protective effects of desacyl ghrelin on diabetic cardiomyopathy", ACTA DIABETOLOGICA, SPRINGER INTERNATIONAL, BERLIN, DE, vol. 52, no. 2, 6 September 2014 (2014-09-06), pages 293 - 306, XP035475108, ISSN: 0940-5429, [retrieved on 20140906], DOI: 10.1007/S00592-014-0637-4
- EID REFAAT A ET AL: "Cardioprotective effect of ghrelin against myocardial infarction-induced left ventricular injury via inhibition of SOCS3 and activation of JAK2/STAT3 signaling", BASIC RESEARCH IN CARDIOLOGY, STEINKOPFF, DARMSTADT, DE, vol. 113, no. 2, 1 February 2018 (2018-02-01), pages 1 - 16, XP036451658, ISSN: 0300-8428, [retrieved on 20180201], DOI: 10.1007/S00395-018-0671-4
- MAO YUANJIE ET AL: "One dose of oral hexarelin protects chronic cardiac function after myocardial infarction", PEPTIDES, vol. 56, 18 April 2014 (2014-04-18), pages 156 - 162, XP029032589, ISSN: 0196-9781, DOI: 10.1016/J.PEPTIDES.2014.04.004
- NAGAYA NORITOSHI ET AL: "Ghrelin improves left ventricular dysfunction and cardiac cachexia in heart failure", CURRENT OPINION IN PHARMACOLOGY, ELSEVIER SCIENCE PUBLISHERS, NL, vol. 3, no. 2, 1 April 2003 (2003-04-01), pages 146 - 151, XP002311021, ISSN: 1471-4892
- BEIRAS-FERNANDEZ A ET AL: "Altered myocardial expression of ghrelin and its receptor (GHSR-1a) in patients with severe heart failure", PEPTIDES, ELSEVIER, AMSTERDAM, NL, vol. 31, no. 12, 1 December 2010 (2010-12-01), pages 2222 - 2228, XP027451693, ISSN: 0196-9781, [retrieved on 20100908], DOI: 10.1016/J.PEPTIDES.2010.08.019
- KURMANI SAMEER ET AL: "Acute Heart Failure: Definition, Classification and Epidemiology", CURRENT HEART FAILURE REPORTS, CURRENT SCIENCE INC., PHILADELPHIA, PA, US, vol. 14, no. 5, 7 August 2017 (2017-08-07), pages 385 - 392, XP036319097, ISSN: 1546-9530, [retrieved on 20170807], DOI: 10.1007/S11897-017-0351-Y

## Description

The invention relates generally to the treatment of Acute Heart Failure (AHF).

AHF is a distinctly identifiable heart disease that involves a rapid loss of heart function in a patient, which can often lead to death. AHF is the most common cause of hospitalization (Ambrosy *et al.,* 2014). Two particularly problematic types of AHF are acute decompensated heart failure (ADHF) and acute decompensated chronic heart failure (ADCHF).

In ADHF and ADCHF, in-hospital mortality ranges from 5-10%, and median length of hospital stay is 5-8 days. Over 50% of patients are discharged with unresolved symptoms, and within 30 or 60 days in different studies, half have again worsening symptoms, one fourth are re-hospitalized and over 10% have died (Baker et al., 2003; Curtis et al., 2008; Gheorghiade et al., 2006; Go et al., 2014; and Polanczyk et al., 2000). Mortality at 1 year in population wide registries is 25-35% (Lund, 2017). After an improvement in outcomes in the late 1990s, prognosis in ADCHF has not improved since 2000 (Baker et al., 2003; Curtis et al., 2008; Polanczyk et al., 2000; and Thorvaldsen et al., 2016). Costs to society for heart failure are projected to increase 3-fold between 2010 and 2030 and most of this cost is related to ADCHF (Heidenreich et al., 2013).

It is considered that ADHF often develops in the context of pre-existing cardiomyopathy (Kurmani et al., 2017) and Pei et al. (2015) disclose that desacyl (i.e. un-acylated) ghrelin protects against diabetic cardiomyopathy. Desacyl ghrelin is purposely selected as acetyl ghrelin has been described with a negative impact on metabolic parameters critical in these patients. There is currently no available therapy to reduce mortality or re-hospitalization for patients with AHF, ADHF or ADCHF. In some circumstances, diuretics are used for symptom relief.

One particular challenge for the treatment is AHF is that that condition leads to the patient being very sensitive to physiological changes, such as heart arrhythmias, hypotension and ischemia. That sensitive state means that a number of drugs (such as inotrope drugs) cause problematic physiological changes contributing to worse outcomes in AHF.

Accordingly, AHF, and in particular ADHF and ADCHF, are difficult and expensive to treat, which leads to a financial burden on health services. Therefore, there is a great demand to develop new and effective treatments for AHF, and for ADHF and ADCHF in particular.

Against this background, the inventors have developed a new treatment for AHF, and in particular ADHF and ADCHF, using acylated ghrelin.

Accordingly, a first aspect of the invention provides an acylated ghrelin molecule for use in the treatment of Acute Heart Failure (AHF) in an individual.

AHF occurs in an individual when the function of the individual's heart (such as the function of the heart in maintaining the flow of blood) rapidly deteriorates over a short period of time. An individual can be diagnosed as having AHF if the individual exhibits the rapid onset of symptoms associated with heart failure, which include a shortness of breath (also called dyspnea), fatigue, coughing, swollen tissues (also called edema), dizziness, and/or light-headedness. If an individual with AHF is not treated, the individual's heart might cease to function, leading to the individual dying. One skilled in medicine would be able to identify if an individual has AHF.

Ghrelin is a 28 amino acid peptide hormone. Generally, ghrelin is known to be secreted into the bloodstream from cells in an individual's gastrointestinal tract, and functions to regulate appetite as well as the distribution and rate of energy use. It has been considered that ghrelin act as an inotrope agent, and exhibit side effects that would be highly dangerous for a patient with AHF, including increased heart arrhythmias, hypotension and ischemia (Abraham *et al.,* 2005; Cuffe *et al.,* 2002; Mebasaa *et al,* 2007; and Packer *et al.,* 2013). Due to these expected dangerous side effects, ghrelin has never been considered suitable as a treatment for AHF.

Against that background, the inventors have now surprisingly identified that although ghrelin is an inotrope it does not exhibit the expected side effects, and is suitable and very effective for treating AHF. The inventors have demonstrated the mechanism by which ghrelin functions, which showed that it increases heart muscle contractility but does so by increasing sensitivity to existing Ca²⁺ rather than increasing Ca²⁺ concentrations in the heart muscle cells, so will not have the previously expected dangerous side effects for AHF patients associated with inotrope drugs (in particular, heart arrhythmias, hypotension and ischemia). The inventors also conducted a human study of ghrelin treatment in patients with advanced heart failure, which is closely related to AHF but is less severe, which showed that ghrelin was effective in improving symptoms and cardiac output, and did not worsen survival of patients. Advanced heart failure patients were used instead of AHF patients for this study for ethical reasons, as it was safer to initially test ghrelin on human patients with a less severe heart failure condition.

The term "treatment" would be understood by those skilled in medicine.

By "treatment" we include any treatment of AHF in an individual, particularly a human, and optionally include one or more of the following effects:
(i) inhibiting AHF, for example slowing, reducing or arresting the development of AHF;
(ii) relieving AHF, for example causing regression of AHF in an individual having the AHF;
(iii) curing AHF, for example returning an individual having the AHF, to a state of health in which the AHF, is no longer detectable; and/or
(iv) when AHF is resolved, reducing and/or inhibiting the progression and/or onset of chronic Heart Failure (HF).

In an embodiment, the individual is suspected of having AHF.

Due to the rapid onset of AHF, in some circumstances it might be necessary to administer a treatment, such an acylated ghrelin, before a diagnosis of AHF is confirmed, so when AHF is just suspected. Accordingly, by "suspected of having AHF" we include that the individual displays one or more of the signs and/or symptoms of AHF, but that that individual has not yet received a diagnosis of AHF.

In an embodiment, the individual is administered with a therapeutically effective amount of the acylated ghrelin molecule.

One skilled in medicine would understand what is a "therapeutically effective amount" of acylated ghrelin. By "therapeutically effective amount" we include the amount of acylated ghrelin that is sufficient to effect beneficial or desired results, including clinical results, associated with the treatment of AHF.

In one embodiment, the AHF is AHF of the left side of the heart or AHF of the right side of the heart. Preferably, the AHF is of the left side of the heart.

As would be known to those skilled in medicine, the heart is anatomically separated into a left side and a right side. Each side of the heart is separated into two chambers, a ventricle and an atrium. Accordingly, the right side of the heart comprises the right ventricle and right atrium, and the left side of the heart comprises the left ventricle and left atrium. Blood low in oxygen enters the right atrium from the superior and inferior venae cavae, and then passes into the right ventricle. The blood is then pumped to the lungs via the pulmonary circulation where it receives oxygen. The blood high in oxygen then enters the left atrium and passes to the left ventricle, from where it is pumped via the aorta to the systemic circulation. Accordingly, by "AHF of the right side of the heart", we include that the right atrium and/or the right ventricle is the subject of the AHF. By "AHF of the left side of the heart", we include that the left atrium and/or the left ventricle is the subject of the AHF.

In an embodiment, the AHF is selected from the group comprising or consisting of: Acute Decompensated Heart Failure (ADHF); AHF associated with hypertension; tachycardia-mediated AHF; AHF associated with pulmonary edema; cardiogenic shock AHF; or severe cardiogenic shock AHF.

Acute Decompensated Heart Failure (ADHF) occurs in an individual with existing heart failure, of an individual predisposed to heart failure, when that individual exhibits a rapid worsening of heart function over a short period of time, which can be characterised by new heart failure symptoms and/or signs, worsening of existing heart failure symptoms and/or signs and/or a rapid increase in the number of additional heart failure symptoms and/or signs. The particularly relevant symptoms and signs are shortness of breath, swollen legs, dizziness, light-headedness, and/or fatigue. One skilled in medicine would be able to identify if an individual has ADHF.

AHF associated with hypertension is similar to the AHF as described above, but for which a predominant symptom or sign is hypertension. One skilled in medicine would be able to identify if an individual has AHF associated with hypertension.

Tachycardia-mediated AHF is similar to the AHF as described above, but for which a predominant symptom or sign is tachycardia. One skilled in medicine would be able to identify if an individual has tachycardia-mediated AHF.

AHF associated with pulmonary edema is similar to the AHF as described above, but for which a predominant symptom or sign is a pulmonary edema. One skilled in medicine would be able to identify if an individual has AHF associated with pulmonary edema.

Cardiogenic shock AHF is similar to the AHF as described above, but for which a predominant symptom or sign is cardiogenic shock. One skilled in medicine would be able to identify if an individual has cardiogenic shock AHF.

Severe cardiogenic shock AHF is similar to the AHF as described above, but for which a predominant symptom or sign is severe cardiogenic shock. One skilled in medicine would be able to identify if an individual has severe cardiogenic shock AHF.

In a preferred embodiment, the Acute Decompensated Heart Failure (ADHF) is Acute Decompensated Chronic Heart Failure (ADCHF) (also known as Acute Decompensated Congestive Heart Failure) or congestive ADHF, more preferably Acute Decompensated Chronic Heart Failure (ADCHF).

Acute Decompensated Chronic Heart Failure (ADCHF) occurs in an individual with existing Chronic Heart Failure (CHF). CHF is characterised by the heart function in an individual gradually worsening over a long time (for example, at least months and often years), which can be diagnosed by the gradual worsening, and/or appearance, of symptoms and/or signs of heart failure. ADCHF is particularly relevant to individuals with CHF which is characterised by the symptoms: dyspnea, edema and/or fatigue. ADCHF occurs when an individual with CHF exhibits a rapid worsening of heart function over a short period of time (for example, over a period of a few weeks to a few days). The prognosis for ADCHF is particularly poor because the patient is usually receiving treatment for CHF, but deteriorates despite that treatment. One skilled in medicine would be able to identify if an individual has ADCHF.

AHF (and, in particular, ADHF and ADCHF) differs from CHF because CHF patient are stable at rest without dyspnea (shortness of breath), and have adequate cardiac output such that organ function is not deteriorating. Conversely, AHF (and, in particular, ADHF and ADCHF) patients have dyspnea and inadequate cardiac function even at rest, amongst other symptoms.

Other symptoms present in AHF (and, in particular, ADHF and ADCHF) patients but not in CHF patients are hypoxemia, worsening edema, acute weight gain, elevated (such as rising level of) biomarkers (such as BNP and NT-proBNP), worsening renal function (as measured by a worsening estimated glomerular filtration rate), low blood pressure, dizziness, and/or light-headedness.

Congestive ADHF is similar to AHF and ADHF, and an individual with congestive ADHF would exhibit similar symptoms. However, in congestive ADHF the individual is particularly susceptible to fluid retention, which leads to dyspnea, fatigue and edemas.

In another preferred embodiment, the Acute Decompensated Chronic Heart Failure (ADCHF) is severe ADCHF.

Severe ADCHF occurs in an individual with CHF in which heart function is worsening at a very rapid rate (for example, over a period of a few days to a few hours). Severe ADCHF is characterised as the individual exhibiting hypotension, shock, arrhythmias and widespread ischemia. The mortality in individuals with severe ADCHF is high, at over 50%. The prognosis for ADCHF is particularly poor because the patient is usually receiving treatment for CHF, but deteriorates rapidly despite that treatment. Patients with severe ADCHF are unlikely to survive if treated with treatments known in the art (such as diuretics, oxygen, currently existing inotropes and/or vasodilators). One skilled in medicine would be able to identify if an individual has severe ADCHF.

In an embodiment, the individual has had CHF. One skilled in medicine would be able to ascertain if the individual has had CHF, for example on the basis of the medical history of the individual. In some circumstances, the individual might previously have had, or been diagnosed with, CHF and had been considered to have, or had, fully recovered. By "fully recovered" we include that the individual does not have any CHF symptoms.

In an embodiment, the individual has had advanced heart failure. Advanced heart failure is an advanced form of chronic heart failure so is sometimes referred to as advanced chronic heart failure. Advanced heart failure and advanced chronic heart failure are borderline stable forms of the disease. Unlike a patient with AHF, a patient with advanced heart failure or advanced chronic heart failure will usually be cared for at home rather than at hospital. One skilled in medicine would be able to ascertain if the individual has had advanced chronic heart failure, for example on the basis of the medical history of the individual. In some circumstances, the individual might previously have had, or been diagnosed with, advanced chronic heart failure and had been considered to have, or had, recovered. By "recovered" we include: fully recovered, in which the individual does not have any advanced heart failure symptoms; and partially recovered, in which the individual has a reduced number of advanced heart failure symptoms and/or reduced severity of advanced heart failure symptoms. In other circumstances, the individual might have had, or be diagnosed as having, advanced chronic heart failure immediately prior to the onset or diagnosis of AHF, by which we include that the individual had one or more advanced heart failure symptoms immediately prior to the presentation of one or more AHF symptoms. Put in another way, the advanced chronic heart failure deteriorates to AHF. It will be appreciated that the embodiments of the invention can also apply to the treatment of an individual that currently has advanced heart failure, without the individual additionally having AHF.

In an embodiment, the AHF is ADCHF or severe ADCHF and the individual has had Chronic Heart Failure (CHF) for about one year or more; for example: about 13 months or more; about 14 months or more; about 15 months or more; about 16 months or more; about 17 months or more; about 18 months or more; about 19 months or more; about 20 months or more; about 21 months or more; about 22 months or more; about 23 months or more; about two years or more; about 25 months or more; about 26 months or more; about 27 months or more; about 28 months or more; about 29 months or more; about 30 months or more; about 31 months or more; about 32 months or more; about 33 months or more; about 34 months or more; about 35 months or more; about three years or more; about 37 months or more; about 38 months or more; about 39 months or more; about 40 months or more; about 41 months or more; about 42 months or more; about 43 months or more; about 44 months or more; about 45 months or more; about 46 months or more; about 47 months or more; about four years or more; about 49 months or more; about 50 months or more; about 51 months or more; about 52 months or more; about 53 months or more; about 54 months or more; about 55 months or more; about 56 months or more; about 57 months or more; about 58 months or more; about 59 months or more; about five years or more; about 66 months or more; about six years or more; about 78 months or more; about seven years or more; about 90 months or more; about 8 years or more; about 102 months or more; about nine years or more; about 114 months or more; or about or ten years or more, preferably about two years or more or about three years or more. In an alternative embodiment, the AHF is ADCHF or severe ADCHF and the individual has had Chronic Heart Failure (CHF) for about one year or less; for example: about 11 months or less; about ten months or less; about nine months or less; about eight months or less; about seven months or less; about six months or less; about five months or less; about four months or less; about three months or less; about two months or less; or about one month or less.

One skilled in medicine would be able to ascertain how long an individual has had CHF prior to the onset of ADCHF or severe ADCHF, for example on the basis of the medical history of the individual. Accordingly, by "AHF is ADCHF or severe ADCHF and the individual has had Chronic Heart Failure (CHF)" we include:
that the individual has had CHF, or one or more symptom of CHF, for a period of time prior to the onset of ADCHF or severe ADCHF; and/or
that the individual has been diagnosed with CHF for a period of time prior to the onset of ADCHF or severe ADCHF; and/or
that the individual has had CHF, or one or more symptom of CHF, for a period of time prior to the diagnosis of ADCHF or severe ADCHF; and/or
that the individual has been diagnosed with CHF for a period of time prior to the diagnosis of ADCHF or severe ADCHF.

In an embodiment, the individual has previously had AHF, preferably ADCHF. One skilled in medicine would be able to ascertain if the individual has previously had AHF, for example on the basis of the medical history of the individual. In some circumstances, the individual might previously have had, or been diagnosed with, AHF and had been considered to have, or had, recovered. However, following the individual having previously recovered from AHF, the individual is now having an AHF relapse.

Accordingly, "by the individual has previously had AHF" we include:
that the individual previously fully recovered from AHF (for example, the individual previously did not have any AHF symptoms and/or was no longer diagnosed as having AHF), but now the individual has relapsed (for example, the individual now has one or more AHF symptoms and/or is again diagnosed as having AHF); and/or
that the individual previously partially recovered from AHF (for example, the individual previously had a reduced number of AHF symptoms and/or reduced severity of AHF symptoms), but now the individual has relapsed (for example, the individual now has an increased number of AHF symptoms and/or increased severity of AHF symptoms).

In an embodiment, the AHF is not associated with a myocardial infarction.

A myocardial infarction is also known as a heart attack. A myocardial infarction occurs when the flow of blood to a part of the heart decreases or stops, which damages the heart. One skilled in medicine would be able to identify if an individual has had a myocardial infarction, and whether the AHF is associated with the myocardial infarction. Accordingly, by "the AHF is not associated with a myocardial infarction" we include: that the AHF is not caused by a myocardial infarction; and/or the onset, or diagnosis, of the AHF is not linked to a myocardial infarction.

In a preferred embodiment, the individual has not been diagnosed as having had a myocardial infarction and/or the individual is not suspected of having had a myocardial infarction.

In an embodiment, the individual has not had a myocardial infarction for about one month or less; for example: about three weeks or less; about two weeks or less; about one week or less; about six days or less; or about five days or less.

In an embodiment, the AHF is not *de novo* AHF. In an alternative embodiment, the AHF is *de novo* AHF.

*De novo* AHF is AHF in an individual with no previous history of heart failure, and occurs when that individual exhibits a rapid worsening of heart function over a short period of time due to an acute cause (for example, myocarditis). One skilled in medicine would be able to identify if an individual has *de novo* AHF.

In an embodiment, after the administration of the ghrelin molecule the individual exhibits one or more parameters from the group comprising or consisting of: an increased heart output; and/or an increased heart contraction; and/or an increased heart stroke volume; and/or an increased ventricular function; and/or an increased ventricular ejection fraction; and/or reduced troponin I phosphorylation; and/or an increased calcium sensitivity; and/or reduced intracellular cAMP; and/or improved renal function; and/or improved estimated glomerular filtration rate (eGFR); and/or improved dyspnea; and/or improved edema; and/or reduced biomarkers; and/or reduced hypotension; and/or resolution of cardiogenic shock; and/or reduced dizziness; and/or reduced light-headedness; and/or a reduced arterio venous oxygen (AVO2) difference; and/or an increase in pulmonary blood flow (PBF); and/or a reduced estimated systemic vascular resistance (eSVR); and/or reduced pulmonary capillary wedge pressure; and/or reduced left ventricular end-diastolic pressure; and/or reduced left ventricular end-diastolic volume; and/or reduced pulmonary artery pressure; and/or reduced central venous pressure.

By "individual exhibits" we include that changes in the specified parameters (such as heart output) of the individual are detectable and/or observable, for example after the administration of the ghrelin molecule an increased heart output of the individual is detectable and/or observable. One skilled in medicine would be able to identify if the individual exhibits any of the aforementioned parameters.

Any change in the parameter that the individual exhibits following the administration of the ghrelin molecule is usually compared to a measurement of the same parameter prior to the administration of the ghrelin molecule. For example, the heart output in the individual after the administration of the ghrelin molecule will be increased when compared to the heart output of the individual before the administration of the ghrelin molecule.

In a preferred embodiment, after the administration of the ghrelin molecule the individual exhibits one or more parameters from the group comprising or consisting of: an increased heart output; and/or a reduced artero venous oxygen (AVO2) difference; and/or an increase in pulmonary blood flow (PBF); and/or a reduced estimated systemic vascular resistance (eSVR). These specific parameters are particularly relevant to AHF, so it is especially relevant if the individual exhibits any one of them as it indicates that the ghrelin is having a desired effect. In a more preferred embodiment, after the administration of the ghrelin molecule the individual exhibits an increased heart output.

In an embodiment, the ghrelin molecule causes a change in one or more parameters in the individual, wherein the one or more parameters are from the group comprising or consisting of: an increased heart output; and/or an increased heart contraction; and/or an increased heart stroke volume; and/or an increased ventricular function; and/or an increased ventricular ejection fraction; and/or reduced troponin I phosphorylation; and/or an increased calcium sensitivity; and/or reduced intracellular cAMP; and/or improved renal function; and/or improved estimated glomerular filtration rate (eGFR); and/or improved dyspnea; and/or improved edema; and/or reduced biomarkers; and/or reduced hypotension; and/or resolution of cardiogenic shock; and/or reduced dizziness; and/or reduced light-headedness; and/or a reduced arterio venous oxygen (AVO2) difference; and/or an increase in pulmonary blood flow (PBF); and/or a reduced estimated systemic vascular resistance (eSVR); and/or reduced pulmonary capillary wedge pressure; and/or reduced left ventricular end-diastolic pressure; and/or reduced left ventricular end-diastolic volume; and/or reduced pulmonary artery pressure; and/or reduced central venous pressure.

By "the ghrelin molecule causes a change" we include that the specified parameters are altered and/or modified in the individual following the administration of the ghrelin molecule. One skilled in medicine would be able to identify if the ghrelin molecule causes a change in any of the aforementioned parameters.

In a preferred embodiment, the ghrelin molecule causes a change in one or more parameters in the individual, wherein the one or more parameters are from the group comprising or consisting of: an increased heart output; and/or a reduced arterio venous oxygen (AVO2) difference; and/or an increase in pulmonary blood flow (PBF); and/or a reduced estimated systemic vascular resistance (eSVR). In a preferred embodiment, the ghrelin molecule causes a change in the individual, which is an increased heart output.

Ways in which to measure the parameters would be known to one skilled in medicine.

In an embodiment, the heart output is increased if:
the heart output volume is about 1.5 litres/minute or more; for example: about 1.6 litres/minute or more; about 1.7 litres/minute or more; about 1.8 litres/minute or more; about 1.9 litres/minute or more; about 2 litres/minute or more; about 2.1 litres/minute or more; about 2.2 litres/minute or more; about 2.3 litres/minute or more; about 2.4 litres/minute or more; about 2.5 litres/minute or more; about 2.6 litres/minute or more; about 2.7 litres/minute or more; about 2.8 litres/minute or more; about 2.9 litres/minute or more; about 3 litres/minute or more; about 3.1 litres/minute or more; about 3.2 litres/minute or more; about 3.3 litres/minute or more; about 3.4 litres/minute or more; about 3.5 litres/minute or more; about 3.6 litres/minute or more; about 3.7 litres/minute or more; about 3.8 litres/minute or more; about 3.9 litres/minute or more; about 4 litres/minute or more; about 4.1 litres/minute or more; about 4.2 litres/minute or more; about 4.3 litres/minute or more; about 4.4 litres/minute or more; about 4.5 litres/minute or more; about 4.6 litres/minute or more; about 4.7 litres/minute or more; about 4.8 litres/minute or more; about 4.9 litres/minute or more; about 5 litres/minute or more; about 5.1 litres/minute or more; about 5.2 litres/minute or more; about 5.3 litres/minute or more; about 5.4 litres/minute or more; about 5.5 litres/minute or more; about 5.6 litres/minute or more; about 5.7 litres/minute or more; about 5.8 litres/minute or more; about 5.9 litres/minute or more; about 6 litres/minute or more; about 6.5 litres/minute or more; about 7 litres/minute or more; about 7.5 litres/minute or more; about 8 litres/minute or more; about 8.5 litres/minute or more; about 9 litres/minute or more; about 9.5 litres/minute or more; or about 10 litres/minute or more, preferably about 5.2 litres/minute or more; and/or
the heart output volume increases by about 0.1 litres/minute or more; for example: about 0.15 litres/minute or more; about 0.2 litres/minute or more; about 0.25 litres/minute or more; about 0.3 litres/minute or more; about 0.35 litres/minute or more; about 0.4 litres/minute or more; about 0.45 litres/minute or more; about 0.5 litres/minute or more; about 0.55 litres/minute or more; about 0.6 litres/minute or more; about 0.65 litres/minute or more; about 0.7 litres/minute or more; about 0.75 litres/minute or more; about 0.8 litres/minute or more; about 0.85 litres/minute or more; about 0.9 litres/minute or more; about 0.95 litres/minute or more; about 1 litre/minute or more; about 1.05 litres/minute or more; about 1.1 litres/minute or more; about 1.15 litres/minute or more; about 1.2 litres/minute or more; about 1.25 litres/minute or more; about 1.3 litres/minute or more; about 1.35 litres/minute or more; about 1.4 litres/minute or more; about 1.45 litres/minute or more; about 1.5 litres/minute or more; about 1.55 litres/minute or more; about 1.6 litres/minute or more; about 1.65 litres/minute or more; about 1.7 litres/minute or more; about 1.75 litres/minute or more; about 1.8 litres/minute or more; about 1.85 litres/minute or more; about 1.9 litres/minute or more; about 1.95 litres/minute or more; about 2 litres/minute or more; about 2.5 litres/minute or more; about 3 litres/minute or more; about 4 litres/minute or more; or about 5 litres/minute or more, preferably about 0.5 litres/minute or more; more preferably about 1.15 litres/minute or more; and/or
the heart output volume increases by about 5% or more; for example: about 6% or more; about 7% or more; about 8% or more; about 9% or more; about 10% or more; about 11% or more; about 12% or more; about 13% or more; about 14% or more; about 15% or more; about 16% or more; about 17% or more; about 18% or more; about 19% or more; about 20% or more; about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; about 95% or more; about 100% or more; about 105% or more; about 110% or more; about 115% or more; about 120% or more; about 125% or more; about 130% or more; about 135% or more; about 140% or more; about 145% or more; about 150% or more; about 155% or more; about 160% or more; about 165% or more; about 170% or more; about 175% or more; about 180% or more; about 185% or more; about 190% or more; about 195% or more; or about 200% or more, more preferably about 15% or more; even more preferably about 25% or more.

One example of how to measure heart output is using an inert gas rebreathing technique, such as by using the Innocor^{®} device (Innovision, Odense, Denmark). Alternative techniques include echocardiography, the Fick method, thermodilution, and by indicator dilution, which are techniques that would be known to one skilled in medicine.

In an embodiment, heart output is measured using an inert gas rebreathing technique.

In an alternative embodiment, the heart output is measured by echocardiography, or the Fick method, or thermodilution, or by indicator dilution.

In a preferred embodiment, the heart output is increased if the heart output volume is about 5.2 litres/minute or more.

In an alternative preferred embodiment, the heart output is increased if the heart output volume increases by about 0.5 litres/minute or more; more preferably about 1.15 litres/minute or more.

In some embodiments, heart output can be measured using surrogate parameters, such as blood pressure, and/or urine output, and/or a firm pulse, and/or a weak pulse, and/or warm extremities (such as arms and legs), and/or cold extremities (such as arms and legs), and/or levels of serum creatinine, and/or estimated glomerular filtration rate. One skilled in medicine would understand how measurements of those surrogate parameters could be used to demonstrate that an individual's heart output is increased.

In an embodiment, the heart contraction is increased if:
there is an increase of absolute percent fractional shortening of about 2% or more; for example: about 3% or more; about 4% or more; about 5% or more; about 6% or more; about 7% or more; about 8% or more; about 9% or more; about 10% or more; about 11% or more; about 12% or more; about 13% or more; about 14% or more; about 15% or more; about 16% or more; about 17% or more; about 18% or more; about 19% or more; about 20% or more; about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more, preferably about 3% or more; and/or
an increase of relative percent fractional shortening of about 20% or more; for example: about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more, preferably about 42% or more.

The terms "absolute percent fractional shortening" and "relative percent fractional shortening" would be known to one skilled in medicine. An increase in absolute percent fractional shortening can be the difference in percent fraction shortening after and before (such as after and before the administration of the ghrelin molecule) (for example, fractional shortening after minus the fractional shortening before). An increase in relative percent fractional shortening can be the difference between after and before (such as after and before the administration of the ghrelin molecule) divided by the value before (for example, (fractional shortening after minus fractional shortening before) divided by the fractional shortening before).

In a preferred embodiment, the heart contraction is increased if there is an increase of absolute percent fractional shortening of about 3% or more.

In an alternative preferred embodiment, the heart contraction is increased if there is an increase of relative percent fractional shortening of about 42% or more.

In an embodiment, the heart stroke volume is increased if:
the heart stroke volume is about 30 ml or more; for example: about 31 ml or more; about 32 ml or more; about 33 ml or more; about 34 ml or more; about 35 ml or more; about 36 ml or more; about 37 ml or more; about 38 ml or more; about 39 ml or more; about 40 ml or more; about 41 ml or more; about 42 ml or more; about 43 ml or more; about 44 ml or more; about 45 ml or more; about 46 ml or more; about 47 ml or more; about 48 ml or more; about 49 ml or more; about 50 ml or more; about 51 ml or more; about 52 ml or more; about 53 ml or more; about 54 ml or more; about 55 ml or more; about 56 ml or more; about 57 ml or more; about 58 ml or more; about 59 ml or more; about 60 ml or more; about, 61 ml or more; about 62 ml or more; about 63 ml or more; about 64 ml or more; about 65 ml or more; about 66 ml or more; about 67 ml or more; about 68 ml or more; about 69 ml or more; about 70 ml or more; about 71 ml or more; about 72 ml or more; about 73 ml or more; about 74 ml or more; about 75 ml or more; about 76 ml or more; about 77 ml or more; about 78 ml or more; about 79 ml or more; about 80 ml or more; about 85 ml or more; about 90 ml or more; about 95 ml or more; or about 100ml or more, preferably about 69 ml or more; more preferably about 79 ml or more; and/or
the heart stroke volume increases by about 3 ml or more; for example: about 4 ml or more; about 5 ml or more; about 6 ml or more; about 7 ml or more; about 8 ml or more; about 9 ml or more; about 10 ml or more; about 11 ml or more; about 12 ml or more; about 13 ml or more; about 14 ml or more; about 15 ml or more; about 16 ml or more; about 17 ml or more; about 18 ml or more; about 19 ml or more; about 20 ml or more; about 25 ml or more; about 30 ml or more; about 35 ml or more; about 40 ml or more; about 45 ml or more; or about 50 ml or more; and/or
the heart stroke volume increases by about 5% or more; for example: about 10% or more; about 15% or more; about 20% or more; about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more, more preferably about 15% or more, even more preferably about 25% or more.

In an embodiment, the heart stroke volume is increased if it is about 30 ml to about 100 ml; for example, about 40 ml to about 100 ml; about 50 ml to about 100 ml; about 60 ml to about 100 ml; about 70 ml to about 100 ml; about 80 ml to about 100 ml; or about 90 ml to about 100 ml.

In a preferred embodiment, the heart stroke volume is increased if the heart stroke volume is about 69 ml or more; more preferably about 79 ml or more.

In an alternative preferred embodiment, the heart stroke volume is increased if the heart stroke volume increases by about 15% or more, more preferably about 25% or more.

Heart stoke volume can be measured by echocardiography. In an embodiment, the heart stroke volume is measured by echocardiography.

Heart stroke volume can be calculated as heart output per minute divided by heart rate per minute. In one embodiment, the heart stroke volume is calculated as heart output per minute divided by heart rate per minute.

In an embodiment, the troponin I phosphorylation is reduced by about 20% or more; for example: about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more, preferably 50% or more.

In an embodiment, the intracellular cAMP is reduced by about 20% or more; for example: about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more, preferably 75% or more.

As discussed herein, the inventors have identified that the mechanism by which ghrelin functions is by reducing intracellular cAMP. Accordingly, it is particularly advantageous if intracellular cAMP is reduced.

In an embodiment, the ventricular ejection fraction is increased if:
there is an increase of absolute percentage of about 2% or more; for example: about 5% or more; about 10% or more; about 15% or more; about 20% or more; about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more preferably about 5% or more or about 10% or more; and/or
an increase of relative percentage of about 15% or more; for example: about 16% or more; about 17% or more; about 18% or more; about 19% or more; about 20% or more; about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more, preferably about 17% or more.

In a preferred embodiment, the ventricular ejection fraction is increased if there is an increase of absolute percentage of about 5% or more about 10% or more.

In a preferred embodiment, the ventricular ejection fraction is increased if there is an increase of relative percentage of about 25% or more.

In an embodiment, the ventricular ejection fraction is increased if there is an increase of relative percentage of about 20% to about 25%.

Ventricular ejection fraction can be measured by echocardiography. In an embodiment, the ventricular ejection fraction is measured by echocardiography.

The terms "absolute percentage" and "relative percentage" would be known to one skilled in medicine. An increase in absolute percentage can be the difference in percentage after and before (such as after and before the administration of the ghrelin molecule) (for example, percentage after minus the percentage before). An increase in relative percentage can be the difference between after and before (such as after and before the administration of the ghrelin molecule) divided by the value before (for example, (percentage after minus percentage before) divided by the percentage before).

In an embodiment, the improved estimated glomerular filtration rate (eGFR) is an increase of about 10% or more; for example, about 20% or more; about 30% or more; about 40% or more; about 50% or more; about 60% or more; about 70% or more; about 80 or more; or about 90% or more. Preferably, the improved estimated glomerular filtration rate (eGFR) is an increase of about 30% or more.

In an embodiment, the arterio venous oxygen (AVO2) difference is reduced by about 2% or more; for example: about 3% or more; about 4% or more; about 5% or more; about 6% or more; about 7% or more; about 8% or more; about 9% or more; about 10% or more; about 15% or more; about 20% or more; about 25% or more; about 30% or more; about 35% or more; about 40% or more; about 45% or more; about 50% or more; about 55% or more; about 60% or more; about 65% or more; about 70% or more; about 75% or more; about 80% or more; about 85% or more; about 90% or more; or about 95% or more preferably about 7% or more.

In an embodiment, the pulmonary blood flow (PBF) level is increased by about 0.1 litre/minute or more; for example: about 0.15 litres/minute or more; about 0.2 litres/minute or more; about 0.25 litres/minute or more; about 0.3 litres/minute or more; about 0.35 litres/minute or more; about 0.4 litres/minute or more; about 0.45 litres/minute or more; about 0.5 litres/minute or more; about 0.55 litres/minute or more; about 0.6 litres/minute or more; about 0.65 litres/minute or more; about 0.7 litres/minute or more; about 0.75 litres/minute or more; about 0.8 litres/minute or more; about 0.85 litres/minute or more; about 0.9 litres/minute or more; about 0.95 litres/minute or more; about 1 litre/minute or more; about 1.05 litres/minute or more; about 1.1 litres/minute or more; about 1.15 litres/minute or more; about 1.2 litres/minute or more; about 1.25 litres/minute or more; about 1.3 litres/minute or more; about 1.35 litres/minute or more; about 1.4 litres/minute or more; about 1.45 litres/minute or more; about 1.5 litres/minute or more; about 1.55 litres/minute or more; about 1.6 litres/minute or more; about 1.65 litres/minute or more; about 1.7 litres/minute or more; about 1.75 litres/minute or more; about 1.8 litres/minute or more; about 1.85 litres/minute or more; about 1.9 litres/minute or more; about 1.95 litres/minute or more; or about 2 litres/minute or more, preferably about 0.8 litre/minute or more or about 1 litre/minute or more.

In an embodiment, the estimated systemic vascular resistance (eSVR) is reduced by about 100 dyn*s/cm⁻⁵ or more; for example: about 120 dyn*s/cm⁻⁵ or more; about 140 dyn*s/cm⁻⁵ or more; about 160 dyn*s/cm⁻⁵ or more; about 180 dyn*s/cm⁻⁵ or more; about 200 dyn*s/cm⁻⁵ or more; about 220 dyn*s/cm⁻⁵ or more; about 240 dyn*s/cm⁻⁵ or more; about 260 dyn*s/cm⁻⁵ or more; about 280 dyn*s/cm⁻⁵ or more; about 300 dyn*s/cm⁻⁵ or more; about 320 dyn*s/cm⁻⁵ or more; about 340 dyn*s/cm⁻⁵ or more; about 360 dyn*s/cm⁻⁵ or more; about 380 dyn*s/cm⁻⁵ or more; about 400 dyn*s/cm⁻⁵ or more; about 420 dyn*s/cm⁻⁵ or more; about 440 dyn*s/cm⁻⁵ or more; about 460 dyn*s/cm⁻⁵ or more; about 480 dyn*s/cm⁻⁵ or more; or about 500 dyn*s/cm⁻⁵ or more, preferably about 300 dyn*s/cm⁻⁵ or more.

Systemic vascular resistance (eSVR) is a measurement that would be known to one skilled in medicine. It is a measurement of the resistance that the blood must overcome to reach tissues. It can be calculated by: (arterial pressure minus venous pressure) divided by cardiac output.

In an embodiment, the AHF is associated with one or more factors of the group comprising or consisting of: spontaneous worsening of chronic heart failure; an infection; and/or an allergic reaction; and/or a blood clot; and/or surgery; and/or cardiovascular disease; and/or lung disease; and/or cardiomyopathy; and/or sleep apnea; and/or alcohol consumption; and/or recreational drug consumption; and/or anaemia; and/or dyslipidemia; and/or an overactive thyroid; and/or Paget's disease; and/or hypertension (such as pulmonary hypertension); and/or prescription medication consumption; and/or smoking; and/or high blood pressure; and/or kidney dysfunction; and/or diabetes; and/or congenital heart defects; and/or lifestyle choices; and/or an irregular heartbeat; and/or rapid heartbeat; and/or slow heartbeat; and/or inflammation; and/or toxins; and/or autoimmune disease; and/or infiltrative disease; and/or connective tissue disease; and/or metabolic disease; and/or endocrine disease; and old age; and/or hereditary genetic mutations; and/or pregnancy.

One skilled in medicine would be able to identify if the AHF is associated with the abovementioned factors (for example, an infection). Accordingly, by "AHF is associated" we include: that the AHF is caused by the abovementioned factors; and/or the onset, or diagnosis, of the AHF is linked to the above mentioned factors.

In a preferred embodiment, the AHF is associated with one or more factors of the group comprising or consisting of: spontaneous worsening of chronic heart failure; and/or infection; and/or diabetes; and/or lifestyle choices (such as, obesity and/or a sedentary lifestyle).

In an alternative preferred embodiment, the AHF is associated with hypertension.

In an embodiment, the infection is a bacterial infection (such as bacterial pneumonia and/or bacterial sepsis), and/or a viral infection (such as viral pneumonia and/or viral sepsis), and/or a fungal infection, and/or a protozoal infection.

In an embodiment, the surgery is from the group comprising or consisting of: heart surgery; and/or heart bypass surgery (such as contrary artery heart bypass surgery); and/or angioplasty surgery; and/or andioplasty surgery; and/or heart valve repair surgery; and/or heart transplant surgery; and/or surgery to install a device (optionally wherein the device is a pacemaker, and/or heart pump, and/or defibrillator).

In an embodiment, the cardiovascular disease is one or more of the group comprising or consisting of: coronary heart disease; and/or valvular disease; and/or coronary artery disease; and/or atherosclerosis; and/or stroke; and/or peripheral vascular disease.

In an embodiment, the lung disease is asthma and/or chronic obstructive pulmonary disease (COPD).

In an embodiment, the prescription medication is one or more of the group comprising or consisting of: diabetes medication; and/or calcium channel blockers; and/or non-steroidal anti-inflammatory drugs (NSAIDs); and/or thiazolidinediones

In an embodiment, the lifestyle choices are one or more of the group comprising or consisting of: a poor diet; and/or a lack of exercise; and/or being overweight; and/or being obese; and/or a sedentary lifestyle.

In an embodiment, an overactive thyroid is associated with thyrotoxicosis.

In an embodiment, the AHF comprises one or more of the symptoms of the group comprising or consisting of: chest pain; and/or a cough; and/or shock (such as cardiogenic shock); and/or high blood pressure; and/or oliguria; and/or anuria; and/or fatigue; and/or shortness of breath (dyspnea); and/or hypoxemia; and/or rapid breathing (tachypnoea); and/or tachycardia; and/or ischemia; and/or edema (such as, a worsening edema); and/or impaired renal function (such as, worsening renal function); and/or low blood pressure; and/or organ failure (such as liver failure and/or kidney failure); and/or cold extremities; and/or numb extremities; and/or muscle fatigue; and/or nausea; and/or vomiting; and/or weight loss (such as anorexia); and/or pulmonary edema; and/or discomfort of the lower body; and/or peripheral swelling; and/or hypo-perfusion; and/or swelling of the lower body; and/or swelling of the heart; and/or weight gain (such as, sudden weight gain); and/or weight loss; and/or cachexia; and/or elevated neck blood vessels (such as an elevated neck blood vein and/or jugular venous distension); and/or hepatomegaly; and/or dizziness; and/or fainting (also known as syncope); and/or an altered mental state (for example, anxiety and/or confusion and/or depression); and/or loss of appetite; hypotension; and/or arrhythmia; and/or difficulty sleeping; and/or discomfort when lying flat; and/or sleep apnea.

In a preferred embodiment, the AHF comprises one or more of the symptoms of the group comprising or consisting of: shortness of breath (dyspnea) (such as, dyspnea at rest); and/or hypoxemia; and/or an edema (such as, a worsening edema); and/or sudden weight gain; and/or impaired renal function (such as, worsening renal function); and/or low blood pressure; and/or dizziness; and/or cardiogenic shock.

In an alternative preferred embodiment, the AHF comprises one or more of the symptoms of the group comprising or consisting of: shortness of breath (dyspnea); and/or discomfort when lying flat; and/or rapid breathing; and/or anxiety; and/or hypoxemia; and/or high blood pressure.

In a further alternative preferred embodiment, the AHF comprises one or more of the symptoms of the group comprising or consisting of: edema (such as, a worsening edema); weight gain; and/or elevated neck vein; and/or hepatomegaly.

In an embodiment, the lower body comprises one or more of the group comprising or consisting of: feet; and/or ankles; and/or legs (such as lower legs and/or upper legs); and/or lower back.

In an embodiment, sudden weight gain comprises an increase in weight of:
about 1 pound or more in one day; for example: about two pounds or more; about three pounds or more; about four pounds or more; about five pounds or more; about six pounds or more; about seven pounds or more; about eight pounds or more; about nine pounds or more; or about ten pounds or more in one day, preferably, two pounds or more in one day or three pounds or more in one day; and/or
about 1 pound or more in one week; for example: about two pounds or more; about three pounds or more; about four pounds or more; about five pounds or more; about six pounds or more; about seven pounds or more; about eight pounds or more; about nine pounds or more; about ten pounds or more; about 11 pound or more; about 12 pounds or more; about 13 pounds or more; about 14 pounds or more; about 15 pounds or more; about 16 pounds or more; about 17 pounds or more; about 18 pounds or more; about 19 pounds or more; about 20 pounds or more; about 21 pounds or more; about 22 pounds or more; about 23 pounds or more; about 24 pounds or more; about 25 pounds or more; about 26 pounds or more; about 27 pounds or more; about 28 pounds or more; about 29 pounds or more; or about 30 pounds or more in one week, preferably five pounds or more in one week.

In a preferred embodiment, sudden weight gain comprises an increase in weight of two pounds or more in one day.

In an alternative preferred embodiment, sudden weight gain comprises an increase in weight of three pounds or more in one day.

In a further alternative preferred embodiment, sudden weight gain comprises an increase in weight of five pounds or more in one week.

In an embodiment, the shortness of breath (dyspnea) is one or more of the group comprising or consisting of: shortness of breath at rest; and/or shortness of breath walking; and/or shortness of breath lying down; and/or exertional dyspnea; and/or orthopnea; and/or paroxysmal dyspnea; and/or nocturnal dyspnea.

In an embodiment, hypotension is a systolic blood pressure of about 40 mmHg or less; for example: about 45 mmHg or less; about 50 mmHg or less; about 55 mmHg or less; about 60 mmHg or less; about 65 mmHg or less; about 70 mmHg or less; about 75 mmHg or less; about 80 mmHg or less; about 85 mmHg or less; about 90 mmHg or less; about 95 mmHg or less; about 100 mmHg or less; about 105 mmHg or less; about 110 mmHg or less; about 115 mmHg or less; or about 120 mmHg or less, preferably about 90 mmHg or less.

In an embodiment, hypotension is a systolic blood pressure of about 40 mmHg to about 120 mmHg.

In an embodiment, cardiogenic shock is associated with one or more of the group comprising or consisting of:
blood pressure of about 45 mmHg or less, for example: about 50 mmHg or less; about 55 mmHg or less; about 60 mmHg or less; about 65 mmHg or less; about 70 mmHg or less; about 75 mmHg or less; about 80 mmHg or less; about 85 mmHg or less; about 90 mmHg or less; about 95 mmHg or less; about 100 mmHg or less;, preferably about 90 mmHg or less; and/or
a cardiac index of about three litres/minute/metre² or less, for example: about 2.5 litres/minute/metre² or less; about two litres/minute/metre² or less; about 1.5 litres/minute/metre² or less; or about one litres/minute/metre² or less, preferably about 2.5 litres/minute/metre² or less; and/or
a wedge pressure (such as a pulmonary capillary wedge pressure) of about 5 mmHg or more; for example: about 10 mmHg or more; about 15 mmHg or more; about 20 mmHg or more; about 25 mmHg or more; or about 30 mmHg or more, preferably about 15 mmHg or more or about 16 mmHg or more; and/or
cold extremities (for example, cold toes and/or cold fingers); and/or
oliguria; and/or
anuria.

In a preferred embodiment, cardiogenic shock is associated with blood pressure of about 90 mmHg or less, and one or more of the group comprising or consisting of: a cardiac index of about 2.5 litres/minute/metre² or less; and/or a wedge pressure of about 15 mmHg or more; cold extremities; and/or oliguria; and/or anuria.

In an embodiment, ischemia is in one or more organs, such as one or more organs of the group comprising or consisting of: the heart; and/or the brain; and or the kidneys, and/or the gastrointestinal tract; and/or the liver, preferably the heart.

Ischemia can occur due to insufficient blood flow, sometimes caused due to the heart pumping inadequately or an obstruction in the pulmonary system.

In an embodiment, the AHF is congestive ADHF and comprises the symptoms peripheral swelling and/or shortness of breath.

In an embodiment, the AHF is AHF associated with hypotension and comprises one or more symptoms of the group comprising or consisting of: shortness of breath; and/or an altered mental state; and/or oliguria; and/or anuria; and/or hypotension.

In an embodiment, the AHF is AHF associated with pulmonary edema and comprises one or more symptoms of the group comprising or consisting of: shortness of breath; and/or rapid breathing; and/or tachycardia; and/or pulmonary edema.

In an embodiment, the AHF is cardiogenic shock AHF and comprises one or more symptoms of the group comprising or consisting of: hypotension; hypo-perfusion; and/or oliguria; and/or cardiogenic shock; and/or an altered mental state; and/or oliguria; and/or anuria.

In an embodiment, the AHF is severe cardiogenic shock AHF and comprises one or more symptoms of the group comprising or consisting of: hypotension; hypo-perfusion; and/or anuria; and/or oliguria; and/or severe cardiogenic shock; and/or an altered mental state.

In an embodiment, the AHF is AHF of the right side of the heart and comprises the symptoms edema and/or elevated neck blood vessels.

In an embodiment, the AHF is severe ADCHF and comprises one or more symptoms of the group comprising or consisting of: hypotension; and/or shock; and/or an arrhythmia; and/or, widespread ischemia.

In an embodiment, the one or more symptoms develop over a period of about one month or less; for example: about three weeks or less; about two weeks or less; about seven days or less; about six days or less; about five days or less; about four days or less; about three days or less; about two days or less; or about one day or less, preferably about seven days or less, more preferably a period of about seven days to about one day.

By "one or more symptoms develop over a period" we include, that the number of symptoms increase and/or the severity of the symptoms increases over that time period.

In an embodiment, the individual is diagnosed as having AHF using one or more of the procedures of the group comprising or consisting of: an X-ray; and/or a blood test; and/or an electrocardiogram (ECG); and/or based on the medical history of the individual; and/or a positron emission tomography (PET) scan; and/or multigated acquisition (MUGA) scan; and/or scintigraphy; and/or an echocardiogram; and/or an angiogram; and/or hemodynamic measurement; and/or a computerised tomography (CT) scan; and/or a physical examination of symptoms; and/or measuring biomarkers (such as serum natriuretic peptides and/or plasma natriuretic peptides); and/or, a magnetic resonance imaging (MRI) scan.

One skilled in medicine would understand how to use the above procedures to diagnose an individual with AHF. For example, an X-ray can be used to detect an enlarged heart and/or fluid in the lungs or chest space. An ECG can be used to detect ischemia, tachycardia, bradycardia and/or an arrhythmia. A PET scan, a MUGA scan, a scintigraphy, an echocardiogram, an angiogram, a hemodynamic assessment, a CT scan, and/or MRI scan can be used to detect (and/or measure and/or quantify) cardiac output, pulmonary blood flow, stroke volume, ejection fraction, cardiac contractility, and/or cardiac injury.

In a preferred embodiment, the individual is diagnosed as having AHF using one or more of the procedures of the group comprising or consisting of: based on the medical history of the individual; and/or a physical examination of symptoms; and/or an echocardiogram; and/or measuring serum natriuretic peptides.

In an embodiment, the biomarkers are one or more selected of the group comprising or consisting of: serum natriuretic peptides; and/or sST2 cardiac biomarker; and/or mid-regional pro-adrenomedullin (MR-proADM); and/or lactate, preferably serum natriuretic peptides. Preferably, the individual is diagnosed as having AHF if one or more of the biomarkers of serum and/or plasma natriuretic peptides; and/or sST2 cardiac biomarker; and/or mid-regional pro-adrenomedullin (MR-proADM); and/or lactate are elevated.

By the biomarkers being "elevated", we mean that the biomarker in the individual is higher than would be expected in an individual without AHF. One skilled in medicine would understand how to detect those biomarkers, and at what levels those biomarkers could be considered elevated (such as based on Ponikowski 2016, ESC HF guidelines, European Heart Journal).

In an embodiment, the serum natriuretic peptides comprise B-type natriuretic peptide (BNP) and/or N-terminal pro-B-type natriuretic peptide (NT-proBNP); and/or mid-regional pro-atrial natriuretic peptide (MR-proANP).

In an embodiment, the individual is diagnosed as having AHF if:
the BNP is about 50 ng/litre or more, for example: about 60 ng/litre or more; about 70 ng/litre or more; about 80 ng/litre or more; about 90 ng/litre or more; about 100 ng/litre or more; about 110 ng/litre or more; about 120 ng/litre or more; about 130 ng/litre or more; about 140 ng/litre or more; or about 150 ng/litre or more; or about 160 ng/litre or more; or about 170 ng/litre or more; or about 180 ng/litre or more; or about 190 ng/litre or more; or about 200 ng/litre or more; or about 250 ng/litre or more; or about 300 ng/litre or more, preferably about 100 ng/litre or more; and/or
the NT-proBNP is about 250 ng/litre or more, for example: about 260 ng/litre or more; about 270 ng/litre or more; about 280 ng/litre or more; about 290 ng/litre or more; about 300 ng/litre or more; about 310 ng/litre or more; about 320 ng/litre or more; about 330 ng/litre or more; about 340 ng/litre or more; or about 350 ng/litre or more; or about 360 ng/litre or more; or about 370 ng/litre or more; or about 380 ng/litre or more; or about 390 ng/litre or more; or about 400 ng/litre or more; or about 425 ng/litre or more; or about 450 ng/litre or more; or about 500 ng/litre or more, preferably about 300 ng/litre or more.

In a preferred embodiment, the individual is diagnosed as having AHF if the BNP is about 100 ng/litre or more and/or the NT-proBNP is about 300 ng/litre or more.

In an embodiment, the individual is diagnosed as having AHF if the MR-proANP is about 80 pmol/L or more, for example: about 90 pmol/L or more; about 100 pmol/L or more; about 110 pmol/L or more; about 120 pmol/L or more; about 130 pmol/L or more; about 140 pmol/L or more; or about 150 pmol/L or more, preferably about 120 pmol/L or more.

In an embodiment, the hemodynamic measurement is one or more of the group comprising or consisting of: measurement of systolic blood pressure; and/or measurement of cardiac index; and/or measurement of pulmonary capillary wedge pressure.

Pulmonary capillary wedge pressure can be measured by a pulmonary artery catheter. In an embodiment, the pulmonary capillary wedge pressure is measured by a pulmonary artery catheter.

In an embodiment, the hemodynamic measurement is the measurement of systolic blood pressure and the systolic blood pressure is about 180 mmHg or more.

In an embodiment, the hemodynamic measurement is the measurement of systolic blood pressure and the systolic blood pressure is about 90 mmHg or less.

In an alternative embodiment, the hemodynamic measurement is the measurement of systolic blood pressure and the systolic blood pressure is about 90 mmHg to about 180 mmHg.

In an embodiment, the hemodynamic measurement is the measurement of cardiac index and the cardiac index is about five litres/minute/metre² or less; for example, about 4.5 litres/minute/metre² or less; about four litres/minute/metre² or less; about 3.5 litres/minute/metre² or less; about three litres/minute/metre² or less; about 2.5 litres/minute/metre² or less; about two litres/minute/metre² or less; about 1.5 litres/minute/metre² or less; or about one litres/minute/metre² or less, preferably about two litres/minute/metre² or less or about 1.8 litres/minute/metre² or less.

In an embodiment, the hemodynamic measurement is the measurement of pulmonary capillary wedge pressure and the pulmonary capillary wedge pressure is about 10 mmHg or more; for example: about 11 mmHg or more; about 12 mmHg or more; about 13 mmHg or more; about 14 mmHg or more; about 15 mmHg or more; about 16 mmHg or more; about 17 mmHg or more; about 18 mmHg or more; about 19 mmHg or more; about 20 mmHg or more; about 25 mmHg or more; about 30 mmHg or more; about 35 mmHg or more; or about 40 mmHg or more, preferably about 15 mm Hg or more.

In an embodiment, after administration of the ghrelin molecule the individual does not exhibit one or more parameters of the group comprising or consisting of: an increased heart rate; and/or tachycardia; and/or a decreased blood pressure; and/or hypotension; and/or an increased oxygen demand; and/or ischemia; and/or increased plasma troponin T; and/or heart arrhythmias; and/or affected calcium transients. One skilled in medicine would understand how to measure these parameters. For example, heart rate can be measured using pulse palpation, electrocardiogram (ECG) or telemetry. Blood pressure can be measured using an arterial cuff or an arterial line. As discussed here, ischemia can be measured using and ECG, amongst other methods. Ischemia can also be identified using clinical symptoms, such as chest pain and/or chest pressure, as well as increases in myocardial injury biomarkers (such a troponin T and/or troponin I).

By "individual does not exhibit" we include that changes in the specified parameters (such as heart rate) of the individual are not detectable and/or observable, and/or clinically meaningful, for example after the administration of the ghrelin molecule no increased heart rate of the individual is detectable and/or observable. One skilled in medicine would be able to identify if the individual does not exhibit any of the aforementioned parameters.

Any change in the parameter that the individual does not exhibit following the administration of the ghrelin molecule is usually compared to a measurement of the same parameter prior to the administration of the ghrelin molecule. For example, the heart rate in the individual after the administration of the ghrelin molecule is not increased when compared to the heart rate of the individual before the administration of the ghrelin molecule.

In a preferred embodiment, after administration of the ghrelin molecule the individual does not exhibit one or more of the group comprising or consisting of: hypotension; and/or ischemia; and/or heart arrhythmias; and/or tachycardia. In a more preferred embodiment, after administration of the ghrelin molecule the individual does not exhibit one or more of the group comprising or consisting of: hypotension; and/or ischemia; and/or heart arrhythmias. These specific parameters are the particularly problematic side effects for a patient with AHF, which would be expected to be caused by an inotrope. Therefore, it is particularly advantageous if they are not caused by ghrelin.

In an embodiment, the ghrelin molecule does not cause a change in one or more parameters in the individual, wherein the one or more parameters are from the group comprising or consisting of: an increased heart rate; and/or tachycardia; and/or a decreased blood pressure; and/or hypotension; and/or an increased oxygen demand; and/or ischemia; and/or increased plasma troponin T; and/or heart arrhythmias; and/or affected calcium transients.

By "the ghrelin molecule does not cause a change" we include that the specified parameters are not altered and/or modified in the individual following the administration of the ghrelin molecule. One skilled in medicine would be able to identify if the ghrelin molecule does not cause a change in any of the aforementioned parameters.

In a preferred embodiment, the ghrelin molecule does not cause a change in one or more parameters in the individual, wherein the one or more parameters are from the group comprising or consisting of: hypotension; and/or ischemia; and/or heart arrhythmias; and/or tachycardia. In a more preferred embodiment, the ghrelin molecule does not cause a change in one or more parameters in the individual, wherein the one or more parameters are from the group comprising or consisting of: hypotension; and/or ischemia; and/or heart arrhythmias.

Ways in which to measure the parameters would be known to one skilled in medicine.

In an embodiment, the heart rate is increased by:
about 5 heart beats/minute or more; for example: about 10 heart beats/minute or more; about 15 heart beats/minute or more; about 20 heart beats/minute or more; about 25 heart beats/minute or more; about 30 heart beats/minute or more; about 35 heart beats/minute or more; or about 40 heart beats/minute or more; and/or
is about 100 heart beats/minute or more; for example: about 105 heart beats/minute or more; about 110 heart beats/minute or more; about 115 heart beats/minute or more; about 120 heart beats/minute or more; about 125 heart beats/minute or more; about 130 heart beats/minute or more; about 135 heart beats/minute or more; or about 140 heart beats/minute or more.

In an embodiment, tachycardia is an increase in heart rate of:
about 10 heart beats/minute or more; for example: 15 heart beats/minute or more; about 20 heart beats/minute or more; about 25 heart beats/minute or more; about 30 heart beats/minute or more; about 35 heart beats/minute or more; or about 40 heart beats/minute or more, preferably about 20 heart beats/minute or more; and/or
a heart rate of about 100 heart beats/minute or more; for example: about 110 heart beats/minute or more; 115 heart beats/minute or more; about 120 heart beats/minute or more; about 125 heart beats/minute or more; about 130 heart beats/minute or more; about 135 heart beats/minute or more; or about 140 heart beats/minute or more, preferably about 100 heart beats/minutes or more.

In an embodiment, the tachycardia is sustained ventricular tachycardia and/or non-sustained ventricular tachycardia.

In an embodiment, the blood pressure is decreased by about 5 mmHg or more; for example: about 10 mmHg or more; about 15 mmHg or more; about 20 mmHg or more; about 25 mmHg or more; about 30 mmHg or more; about 35 mmHg or more; about 40 mmHg or more; about 45 mmHg or more; about 50 mmHg or more; about 55 mmHg or more; about 60 mmHg or more; about 65 mmHg or more; about 70 mmHg or more; about 75 mmHg or more; about 80 mmHg or more; about 85 mmHg or more; about 90 mmHg or more; about 95 mmHg or more; about 100 mmHg or more, preferably about 20 mmHg or more, more preferably 20 mmHg to 80 mmHg. In a preferred embodiment, the decrease in blood pressure is regardless of the starting blood pressure of the individual.

In an embodiment, hypotension is a blood pressure of about 90 mmHg or less; for example: about 85 mmHg or less; about 80 mmHg or less; about 75 mmHg or less; about 70 mmHg or less; about 65 mmHg or less; about 60 mmHg or less; about 55 mmHg or less; about 50 mmHg or less, preferably about 80 mmHg or less.

In an embodiment, the heart arrhythmia is selected from the group comprising or consisting of: ventricular arrhythmia; supra-ventricular arrhythmia; ectopic atrial tachycardia; atrial flutter; sinus bradycardia; atrial ventricular block (AV block); and atrial fibrillation.

In an embodiment, the ischemia comprises a change in an electrocardiogram (ECG) and/or increased plasma troponin T.

In an embodiment, the change in an electrocardiogram (ECG) comprises one or more of the group comprising or consisting of: ST elevations; and/or ST depressions; and/or T wave changes. One skilled in medicine would understand how to interpret those changes in an ECG, and what they represent for the diagnosis and/or prognosis of an individual.

In an embodiment, the plasma troponin T is increased by about 40% or more; for example: about 45% or more; about 50% or more; about 55% or more; about 60% or more; about 65% or more; about 70% or more; about 75% or more; about 80% or more; about 85% or more; about 90% or more; or about 95% or more; or about 100% or more; or about 110% or more; or about 120% or more; or about 130% or more; or about 150% or more; or about 200% or more; or about 250% or more; or about 300% or more or about 400% or more or about 500% or more, preferably about 100% or more.

In an embodiment, the calcium transient comprises no change in the calcium transient amplitude.

In an embodiment, the ghrelin molecule comprises one or more of the group comprising or consisting of: a modified ghrelin; and/or a ghrelin fusion molecule; and/or a ghrelin fragment; and/or a ghrelin variant; and/or a ghrelin derivative; and/or, wildtype ghrelin.

Ghrelin (also known as lenomorelin (INN)) is a 28 amino acid peptide with a molecular weight of approximately 3,371 g/mol, and in humans it is encoded by the *GHRL* gene. It is peptide hormone that is produced by ghrelinergic cells in the gastrointestinal tract, and functions as a neuropeptide in the central nervous system. Ghrelin binds to the ghrelin/growth hormone secretagogue receptor (GHS-R). The half-life of ghrelin in blood plasma is approximately 24-30 minutes. Concentrations in normal humans has variably been reported at 100-300 pmol/L (300-900 ng/L) for total ghrelin. There is limited data on acyl ghrelin but the inventors have recently measured acyl ghrelin in 41 healthy non-obese adults: fasting: mean±SD: 118±14 ng/L, 5-95th percentile 27-328 ng/L; trough 60 minutes post 260 kcal mixed meal: 80±12 ng/L, 5-95th percentile 21-293 ng/L. Ghrelin is commercially available, for example from Bachem (Bubendorf, Switzerland). A specific ghrelin product that is available from Bachem is a synthetic acylated human ghrelin, which has the brand name Clinalfa.

Ghrelin has an amino acid sequence of:

Gly-Ser-Ser(octanoyl)-Phe-Leu-Ser-Pro-Glu-His-Gln-Arg-Val-Gln-Gln-Arg-Lys-
Glu-Ser-Lys-Lys-Pro-Pro-Ala-Lys-Leu-Gln-Pro-Arg (SEQ ID NO: 1).

In an embodiment, the ghrelin molecule binds specifically to the ghrelin hormone secretagogue receptor (GHS-R) and/or activates the GHS-R.

By "modified ghrelin molecule", we include a ghrelin molecule that has been chemically modified, such as a derivatised ghrelin molecule. Chemical derivatives of one or more amino acids may be achieved by reaction with a functional side group. Such derivatised molecules include, for example, those molecules in which free amino groups have been derivatised to form amine hydrochlorides, p- toluene sulphonyl groups, carboxybenzoxy groups, f-butyloxycarbonyl groups, chloroacetyl groups or formyl groups. Free carboxyl groups may be derivatised to form salts, methyl and ethyl esters or other types of esters and hydrazides. Free hydroxyl groups may be derivatised to form O-acyl or O-alkyl derivatives. Also included as chemical derivatives are those peptides which contain naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine and ornithine for lysine. Derivatives also include peptides containing one or more additions or deletions as long as the requisite activity is maintained. Other included modifications are amidation, amino terminal acylation (e.g., acetylation or thioglycolic acid amidation), terminal carboxylamidation (e.g., with ammonia or methylamine), and the like terminal modifications.

For example, the modified ghrelin molecules described herein include not only molecules in which amino acid residues are joined by peptide (-CO-NH-) linkages but also molecules in which the peptide bond is reversed. Such retro-inverso peptidomimetics may be made using methods known in the art, for example such as those described in Meziere et al. (1997) J. Immunol. 159, 3230-3237. Such retro-inverse peptides, which contain NH-CO bonds instead of CO-NH peptide bonds, are much more resistant to proteolysis. Alternatively, the polypeptide of the invention may be a peptidomimetic compound wherein one or more of the amino acid residues are linked by a -γ(OH2NH)- bond in place of the conventional amide linkage.

It will be appreciated that the polypeptide may conveniently be blocked at its N- or C-terminus so as to help reduce susceptibility to exoproteolytic digestion, e.g., by amidation.

As discussed above, a variety of uncoded or modified amino acids such as D-amino acids and N-methyl amino acids may be used to modify the ghrelin molecule of the invention. In addition, a presumed bioactive conformation may be stabilised by a covalent modification, such as cyclisation or by incorporation of lactam or other types of bridges. Methods of synthesis of cyclic homodetic peptides and cyclic heterodetic peptides, including disulphide, sulphide and alkylene bridges, are disclosed in US 5,643,872. Other examples of cyclisation methods are discussed and disclosed in US 6,008,058. A further approach to the synthesis of cyclic stabilised peptidomimetic compounds is ring-closing metathesis (RCM).

In summary, terminal modifications are useful, as is well known, to reduce susceptibility by proteinase digestion and therefore to prolong the half-life of the peptides in solutions, particularly in biological fluids where proteases may be present. Polypeptide cyclisation is also a useful modification and is preferred because of the stable structures formed by cyclisation and in view of the biological activities observed for cyclic peptides.

An acylated ghrelin molecule is sometimes referred to as an activated ghrelin molecule or an octanoylated ghrelin molecule.

By "ghrelin fragment" we include a functional ghrelin fragment, such as a ghrelin fragment which binds specifically to the GHS-R and/or activates the GHS-R and/or binds specifically to other receptors that interact with ghrelin and/or activates other receptors that interact with ghrelin. In an embodiment, the fragment comprises about 5 amino acids or more of the wildtype ghrelin molecule; for example: about 6 amino acids or more; about 7 amino acids or more; about 8 amino acids or more; about 9 amino acids or more; about 10 amino acids or more; about 11 amino acids or more; about 12 amino acids or more; about 13 amino acids or more; about 14 amino acids or more; about 15 amino acids or more; about 16 amino acids or more; about 17 amino acids or more; about 18 amino acids or more; about 19 amino acids or more; about 20 amino acids or more; about 21 amino acids or more; about 22 amino acids or more; about 23 amino acids or more; about 24 amino acids or more; about 25 amino acids or more; about 26 amino acids or more; or about 27 amino acids of the wildtype ghrelin molecule.

By "wildtype ghrelin" we include a ghrelin molecule with an amino acid sequence of a ghrelin molecule found endogenously in an organism, such as the 28 amino acid human ghrelin molecule. In an embodiment, the wildtype ghrelin molecule is one or more from the group comprising or consisting of: a rodent (for example, a mouse, and/or a rat, and/or a hamster, and/or a guinea pig, and/or a gerbil, and/or a rabbit) wildtype ghrelin molecule; and/or a canine (for example, a dog) wildtype ghrelin molecule; and/or a feline (for example, a cat) wildtype ghrelin molecule; a primate (for example, a human; and/or a monkey; and/or an ape) wildtype ghrelin molecule; and/or an equine (for example, a horse) wildtype ghrelin molecule; and/or a bovine (for example, a cow) wildtype ghrelin molecule; and/or a porcine (for example, a pig) wildtype ghrelin molecule, preferably a human wildtype ghrelin molecule, more preferably a 28 amino acid human wildtype ghrelin molecule.

By "ghrelin fusion molecule" we include a ghrelin molecule (including a modified ghrelin, and/or a ghrelin fragment, and/or a ghrelin variant, and/or a ghrelin derivative, and/or a wildtype ghrelin) which is fused to any other polypeptide and/or protein and/or peptide. For example, the ghrelin molecule may comprise one or more additional amino acids, inserted internally and/or at the N- and/or C-termini.

By "a ghrelin variant', we include the wildtype ghrelin molecule comprising insertions, deletions and/or substitutions, either conservative or non-conservative. In particular, we include variants of the polypeptide where such changes do not substantially alter the activity of the ghrelin molecule. In particular, we also include ghrelin variants where such changes do not substantially alter the binding specificity to the GHS-R and/or activation of the GHS-R. In an embodiment, the ghrelin variant comprises about 5% identity or more to the wildtype ghrelin molecule; for example: about 10% identity or more; about 15% identity or more; about 20% identity or more; about 25% identity or more; about 30% identity or more; about 35% identity or more; about 40% identity or more; about 45% identity or more; about 50% identity or more; about 55% identity or more; about 60% identity or more; about 65% identity or more; about 70% identity or more; about 75% identity or more; about 80% identity or more; about 85% identity or more; about 86% identity or more; about 87% identity or more; about 88% identity or more; about 89% identity or more; about 90% identity or more; about 91% identity or more; about 92% identity or more; about 93% identity or more; about 94% identity or more; about 95% identity or more; about 96% identity or more; about 97% identity or more; about 98% identity or more; or about 99% identity to the wildtype ghrelin molecule; and/or comprises about 5 or more contiguous amino acids of the wildtype ghrelin molecule; for example: about 6 or more contiguous amino acids; about 7 or more contiguous amino acids; about 8 or more contiguous amino acids; about 9 or more contiguous amino acids; about 10 or more contiguous amino acids; about 11 or more contiguous amino acids; about 12 or more contiguous amino acids; about 13 or more contiguous amino acids; about 14 or more contiguous amino acids; about 15 or more contiguous amino acids; about 16 or more contiguous amino acids; about 17 or more contiguous amino acids; about 18 or more contiguous amino acids; about 19 or more contiguous amino acids; about 20 or more contiguous amino acids; about 21 or more contiguous amino acids; about 22 or more contiguous amino acids; about 23 or more contiguous amino acids; about 24 or more contiguous amino acids; about 25 or more contiguous amino acids; about 26 or more contiguous amino acids; or about 27 contiguous amino acids.

The percent sequence identity between two polypeptides may be determined using suitable computer programs, for example the GAP program of the University of Wisconsin Genetic Computing Group and it will be appreciated that percent identity is calculated in relation to polypeptides whose sequences have been aligned optimally.

The alignment may alternatively be carried out using the Clustal W program (as described in Thompson et aL, 1994, NucL Acid Res. 22:4673-4680).

Fragments and variants of the ghrelin molecule may be made using any of the methods of protein engineering, directed evolution and/or site-directed mutagenesis well known in the art (for example, see Molecular Cloning: a Laboratory Manual, 3rd edition, Sambrook & Russell, 2001, Cold Spring Harbor Laboratory Press).

By "derivative ghrelin" we include peptidomimetic compounds, which may also be useful, such as peptidomimetic compounds which bind specifically to GHS-R and/or activate the GHS-R. The term "peptidomimetic" refers to a compound that mimics the conformation and desirable features of a particular ghrelin molecule as a therapeutic agent.

In an embodiment, the acylated ghrelin molecule comprises one or more of the group comprising or consisting of: a synthetic ghrelin molecule; and/or a recombinant ghrelin molecule; and/or, an endogenous ghrelin molecule.

By "synthetic ghrelin molecule" we include a ghrelin molecule created artificially without the use of a host cell or a host organism, such as by liquid phase peptide synthesis and solid phase peptide synthesis.

In an embodiment, the synthetic ghrelin molecule is one or more from the group comprising or consisting of: a synthetic modified ghrelin; and/or a synthetic ghrelin fusion molecule; and/or a synthetic ghrelin fragment; and/or a synthetic ghrelin variant; and/or a synthetic ghrelin derivative; and/or, a synthetic wildtype ghrelin.

By "recombinant ghrelin molecule" we include a ghrelin molecule created by recombinantly expressing a gene encoding the ghrelin molecule in a recombinant host cell organism, such as a recombinant *E. coli* host, preferably followed by the recovery and purification of the ghrelin molecule therefrom. In some embodiments, the recombinant ghrelin molecule does not have an N-terminal methionine amino acid.

In an embodiment, the recombinant ghrelin molecule is one or more from the group comprising or consisting of: a recombinant modified ghrelin; and/or a recombinant ghrelin fusion molecule; and/or a recombinant ghrelin fragment; and/or a recombinant ghrelin variant; and/or a recombinant ghrelin derivative; and/or, a recombinant wildtype ghrelin.

By "endogenous ghrelin molecule" we include a ghrelin molecule created by a host organism, in which the ghrelin molecule might be a ghrelin molecule which is a wildtype ghrelin molecule for that host organism or a ghrelin molecule that is not a wildtype ghrelin molecule for that host organism.

In an embodiment, the endogenous ghrelin molecule is one or more from the group comprising or consisting of: an endogenous modified ghrelin; and/or an endogenous ghrelin fusion molecule; and/or an endogenous ghrelin fragment; and/or an endogenous ghrelin variant; and/or an endogenous ghrelin derivative; and/or, an endogenous wildtype ghrelin.

In an embodiment, the host organism is one or more from the group comprising or consisting of: a rodent (for example, a mouse, and/or a rat, and/or a hamster, and/or a guinea pig, and/or a gerbil, and/or a rabbit); and/or a canine (for example, a dog); and/or a feline (for example, a cat); a primate (for example, a human; and/or a monkey; and/or an ape); and/or an equine (for example, a horse); and/or a bovine (for example, a cow); and/or a porcine (for example, a pig).

In an embodiment, the ghrelin molecule is administered once or more each day; for example: twice or more each day; three times or more each day; four times or more each day; or five times or more each day, preferably twice each day.

In an embodiment, the ghrelin molecule is administered over a period of one day or more; for example: two days or more; three days or more; four days or more; five days or more; six days or more; one week or more; eight days or more; nine days or more; ten days or more; 11 days or more; 12 days or more; 13 days or more; two weeks or more; three weeks or more; four weeks or more; one month or more; five weeks or more; six weeks or more; seven weeks or more; eight weeks or more; two months or more; three months or more; four months or more; five months or more; six months or more; seven months or more; eight months or more; nine months or more; ten months or more; 11 months or more; or one year or more.

In an embodiment, the ghrelin molecule is administered daily, every two days, every three days, every four days, every five days, every six days, every one week, every eight days, every nine days, every ten days, every 11 days, every 12 days, every 13 days, every one week, every two weeks, every three weeks, every four weeks, every one month, every two months, every three months, every four months, every five months, every six months, every seven months, every eight months, every nine months, every ten months, every eleven months, or every one year.

In an embodiment, the ghrelin molecule is administered until it has a desired effect, such as:
(i) inhibiting AHF, for example slowing, reducing or arresting the development of AHF;
(ii) relieving AHF, for example causing regression of AHF in an individual having the AHF; and/or
(iii) curing AHF, for example returning an individual having the AHF, to a state of health in which the AHF, is no longer detectable.

In an embodiment, the ghrelin molecule is administered parenterally, and/or intravenously, and/or intra-arterially, and/or intraperitoneally, and/or intra-thecally, and/or intramuscularly, and/or subcutaneously, and/or by infusion.

In a preferred embodiment, the ghrelin molecule is administered by infusion, such as an intravenous infusion.

In an embodiment, the ghrelin molecule is administered by infusion over a period of about 10 minutes or more; for example: about 20 minutes or more; about 30 minutes or more; about 40 minutes or more; about 50 minutes or more; about one hour or more; about two hours or more; about three hours or more; about four hours or more; about five hours or more; about six hours or more; about seven hours or more; about eight hours or more; about nine hours or more; about ten hours or more; about 11 hours or more; about 12 hours or more; about 13 hours or more; about 14 hours or more; about 15 hours or more; about 16 hours or more; about 17 hours or more; about 18 hours or more; about 19 hours or more; about 20 hours or more; about 21 hours or more; about 22 hours or more; about 23 hours or more; or about 24 hours or more, preferably about six hours or more or about 24 hours or more, more preferably two hours or more.

In an embodiment, the ghrelin molecule is administered by infusion at a rate of about 0.1 ml/min or more; for example: about 0.2 ml/min or more; about 0.3 ml/min or more; about 0.4 ml/min or more; about 0.5 ml/min or more; about 0.6 ml/min or more; about 0.7 ml/min or more; about 0.8 ml/min or more; about 0.9 ml/min or more; about 1 ml/min or more; about 1.1 ml/min or more; about 1.2 ml/min or more; about 1.3 ml/min or more; about 1.4 ml/min or more; about 1.5 ml/min or more; about 1.6 ml/min or more; about 1.7 ml/min or more; about 1.8 ml/min or more; about 1.9 ml/min or more; about 2 ml/min or more; about 3 ml/min or more; about 4 ml/min or more; about 5 ml/min or more; about 6 ml/min or more; about 7 ml/min or more; about 8 ml/min or more; about 9 ml/min or more; or about 10 ml/min or more, preferably about 0.5 ml/min or more.

In an embodiment, the ghrelin molecule is administered at a dose of about 1 µg/kg body weight or more; for example: about 2 µg/kg body weight or more; about 3 µg/kg body weight or more; about 4 µg/kg body weight or more; about 5 µg/kg body weight or more; about 6 µg/kg body weight or more; about 7 µg/kg body weight or more; about 8 µg/kg body weight or more; about 9 µg/kg body weight or more; about 10 µg/kg body weight or more; about 11 µg/kg body weight or more; about 12 µg/kg body weight or more; about 13 µg/kg body weight or more; about 14 µg/kg body weight or more; about 15 µg/kg body weight or more; about 16 µg/kg body weight or more; about 17 µg/kg body weight or more; about 18 µg/kg body weight or more; about 19 µg/kg body weight or more; about 20 µg/kg body weight or more; about 25 µg/kg body weight or more; about 30 µg/kg body weight or more; about 40 µg/kg body weight or more; about 50 µg/kg body weight or more; about 60 µg/kg body weight or more; about 70 µg/kg body weight or more; about 80 µg/kg body weight or more; about 90 µg/kg body weight or more; or about 100 µg/kg body weight or more, preferably about 12 µg/kg body weight or more.

In an embodiment, the ghrelin molecule is administered at a dose of about 0.001 µg/kg body weight/min or more, for example: about 0.002 µg/kg body weight/min or more; about 0.003 µg/kg body weight/min or more; about 0.004 µg/kg body weight/min or more; about 0.005 µg/kg body weight/min or more; about 0.006 µg/kg body weight/min or more; about 0.007 µg/kg body weight/min or more; about 0.008 µg/kg body weight/min or more; about 0.009 µg/kg body weight/min or more; about 0.01 µg/kg body weight/min or more; about 0.02 µg/kg body weight/min or more; about 0.03 µg/kg body weight/min or more; about 0.04 µg/kg body weight/min or more; about 0.05 µg/kg body weight/min or more; about 0.06 µg/kg body weight/min or more; about 0.07 µg/kg body weight/min or more; about 0.08 µg/kg body weight/min or more; about 0.09 µg/kg body weight/min or more; about 0.1 µg/kg body weight/min or more; about 0.2 µg/kg body weight/min or more; about 0.3 µg/kg body weight/min or more; about 0.4 µg/kg body weight/min or more; about 0.5 µg/kg body weight/min or more; about 0.6 µg/kg body weight/min or more; about 0.7 µg/kg body weight/min or more; about 0.8 µg/kg body weight/min or more; about 0.9 µg/kg body weight/min or more; about 1 µg/kg body weight/min or more; about 1.5 µg/kg body weight/min or more; about 2 µg/kg body weight/min or more; about 2.5 µg/kg body weight/min or more; about 3 µg/kg body weight/min or more; about 3.5 µg/kg body weight/min or more; about 4 µg/kg body weight/min or more; about 4.5 µg/kg body weight/min or more; about 5 µg/kg body weight/min or more; about 5.5 µg/kg body weight/min or more; about 6 µg/kg body weight/min or more; about 6.5 µg/kg body weight/min or more; about 7 µg/kg body weight/min or more; about 7.5 µg/kg body weight/min or more; about 8 µg/kg body weight/min or more; about 8.5 µg/kg body weight/min or more; about 9 µg/kg body weight/min or more; about 9.5 µg/kg body weight/min or more; or about 10 µg/kg body weight/min or more, preferably about 0.1 µg/kg body weight/min or more.

In an embodiment, the ghrelin molecule is administered at a dose of about 1 picomol (pmol) or more, for example: about 2 pmol or more; about 3 pmol or more; about 4 pmol or more; about 5 pmol or more; about 6 pmol or more; about 7 pmol or more; about 8 pmol or more; about 9 pmol or more; about 10 pmol or more; about 15 pmol or more; about 20 pmol or more; about 25 pmol or more; about 30 pmol or more; about 35 pmol or more; about 40 pmol or more; about 45 pmol or more; about 50 pmol or more; about 55 pmol or more; about 60 pmol or more; about 65 pmol or more; about 70 pmol or more; about 75 pmol or more; about 80 pmol or more; about 85 pmol or more; about 90 pmol or more; about 95 pmol or more; about 100 pmol or more; about 110 pmol or more; about 120 pmol or more; about 130 pmol or more; about 140 pmol or more; about 150 pmol or more; about 160 pmol or more; about 170 pmol or more; about 180 pmol or more; about 190 pmol or more; about 200 pmol or more; about 250 pmol or more; about 300 pmol or more; about 350 pmol or more; about 400 pmol or more; about 450 pmol or more; or about 500 pmol or more, preferably about 30 pmol or more.

In an embodiment, the ghrelin molecule is administered at a concentration of about 1 µg/ml or more; for example: about 1.2 µg/ml or more; about 1.4 µg/ml or more; about 1.6 µg/ml or more; about 1.8 µg/ml or more; about 2 µg/ml or more; about 2.2 µg/ml or more; about 2.4 µg/ml or more; about 2.6 µg/ml or more; about 2.8 µg/ml or more; about 3 µg/ml or more; about 3.2 µg/ml or more; about 3.4 µg/ml or more; about 3.6 µg/ml or more; about 3.8 µg/ml or more; about 4 µg/ml or more; about 4.2 µg/ml or more; about 4.4 µg/ml or more; about 4.6 µg/ml or more; about 4.8 µg/ml or more; about 5 µg/ml or more; about 5.2 µg/ml or more; about 5.4 µg/ml or more; about 5.6 µg/ml or more; about 5.8 µg/ml or more; about 6 µg/ml or more; about 6.2 µg/ml or more; about 6.4 µg/ml or more; about 6.6 µg/ml or more; about 6.8 µg/ml or more; about 7 µg/ml or more; about 7.2 µg/ml or more; about 7.4 µg/ml or more; about 7.6 µg/ml or more; about 7.8 µg/ml or more; about 8 µg/ml or more; about 8.1 µg/ml or more; about 8.2 µg/ml or more; about 8.3 µg/ml or more; about 8.4 µg/ml or more; about 8.5 µg/ml or more; about 8.6 µg/ml or more; about 8.7 µg/ml or more; about 8.8 µg/ml or more; about 8.9 µg/ml or more; about 9 µg/ml or more; about 9.1 µg/ml or more; about 9.2 µg/ml or more; about 9.3 µg/ml or more; about 9.4 µg/ml or more; about 9.5 µg/ml or more; about 9.6 µg/ml or more; about 9.7 µg/ml or more; about 9.8 µg/ml or more; about 9.9 µg/ml or more; about 10 µg/ml or more; about 11 µg/ml or more; about 12 µg/ml or more; about 13 µg/ml or more; about 14 µg/ml or more; about 15 µg/ml or more; about 16 µg/ml or more; about 17 µg/ml or more; about 18 µg/ml or more; about 19 µg/ml or more; about 20 µg/ml or more; about 21 µg/ml or more; about 22 µg/ml or more; about 23 µg/ml or more; about 24 µg/ml or more; about 25 µg/ml or more; about 26 µg/ml or more; about 27 µg/ml or more; about 28 µg/ml or more; about 29 µg/ml or more; or about 30 µg/ml or more.

In a preferred embodiment, the ghrelin molecule is administered at a concentration of about 10 µg/ml to about 30 µg/ml.

In an embodiment, the amount of the ghrelin molecule administered is about 100 µg or more; for example: about 120 µg or more; about 140 µg or more; about 160 µg or more; about 180 µg or more; about 200 µg or more; about 220 µg or more; about 240 µg or more; about 260 µg or more; about 280 µg or more; about 300 µg or more; about 320 µg or more; about 340 µg or more; about 360 µg or more; about 380 µg or more; about 400 µg or more; about 420 µg or more; about 440 µg or more; about 460 µg or more; about 480 µg or more; about 500 µg or more; about 520 µg or more; about 540 µg or more; about 560 µg or more; about 580 µg or more; about 600 µg or more; about 620 µg or more; about 640 µg or more; about 660 µg or more; about 680 µg or more; about 700 µg or more; about 720 µg or more; about 740 µg or more; about 760 µg or more; about 780 µg or more; or about 800 µg or more.

In a preferred embodiment, the ghrelin molecule is administered at a dosage of about 0.1 µg / kg body weight / min and at a concentration of about 10 µg/ml to about 30 µg/ml and at an infusion rate of 0.5 ml/min.

As outlined in Table 2, below, the concentration and/or amount of ghrelin administered can be dependent on the body weight of the individual to which the ghrelin is to be administered.

In therapy, the ghrelin molecule and compositions of the invention can be administered alone but will generally be administered in admixture with a suitable pharmaceutical excipient, diluent or carrier selected with regard to the intended route of administration and standard pharmaceutical practice.

The ghrelin molecule and compositions of the invention are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-is known to those skilled in medicine.

Ghrelin molecules and compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The ghrelin molecules and compositions may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets.

The physician will determine the dosage of the ghrelin molecule and compositions which will be most suitable for any individual and it will vary with the age, weight and response of the particular individual.

In an embodiment, the individual is one or more from the group comprising or consisting of: a rodent (for example, a mouse, and/or a rat, and/or a hamster, and/or a guinea pig, and/or a gerbil, and/or a rabbit); and/or a canine (for example, a dog); and/or a feline (for example, a cat); a primate (for example, a human; and/or a monkey; and/or an ape); and/or an equine (for example, a horse); and/or a bovine (for example, a cow); and/or a porcine (for example, a pig), preferably a human.

In an embodiment, the individual is aged 18 years or older; for example: 20 years or older; 30 years or older; 40 years or older; 50 years or older; 60 years or older; 65 years or older; 70 years or older; 75 years or older; 80 years or older; 85 years or older; or 90 years or older, preferably 65 years or older.

Generally, AHF is considered to be a disease which is more prevalent in older individuals. Therefore, the ghrelin molecule of the invention is likely to be particularly effective in older individuals.

In an embodiment, the ghrelin molecule is administered before surgery, and/or during surgery, and/or after surgery.

In some circumstances AHF can be, in part, caused by surgery. Therefore, administration of the ghrelin molecule of the invention might be particularly effective before, after or during surgery.

In an embodiment, the surgery is selected from the group comprising or consisting of: heart surgery; and/or heart bypass surgery (such as contrary artery heart bypass surgery); and/or angioplasty surgery; and/or andioplasty surgery; and/or heart valve repair surgery; and/or heart transplant surgery; and/or gastrointestinal surgery; and/or orthopaedic surgery; and/or neurological surgery; and/or surgery to install a device (optionally wherein the device is a pacemaker, and/or heart pump, and/or defibrillator), preferably heart bypass surgery.

In an embodiment, the individual is administered with one or more additional therapeutic agents.

In an embodiment, the ghrelin molecule is in a composition, preferably a pharmaceutical composition.

In an embodiment, the composition comprises a pharmaceutically acceptable excipient.

In an embodiment, the composition comprises one or more from the group comprising or consisting of: a non-toxic organic acid; and/or a non-toxic inorganic acid; and/or non-toxic organic base; and/or a non-toxic inorganic base; and salts therefrom.

The chemical bases that may be used as reagents to prepare pharmaceutically acceptable base salts of the present agents that are acidic in nature are those that form non-toxic base salts with such compounds. Such non-toxic base salts include, but are not limited to those derived from such pharmacologically acceptable cations such as alkali metal cations (e.g. potassium and sodium) and alkaline earth metal cations (e.g. calcium and magnesium), ammonium or water-soluble amine addition salts such as N-methylglucamine-(meglumine), and the lower alkanolammonium and other base salts of pharmaceutically acceptable organic amines, among others

In an embodiment, the composition comprises one or more additional therapeutic agents.

In an embodiment, the one or more additional therapeutic agents are one or more additional therapeutic agents for the treatment of AHF.

In an embodiment, the one or more therapeutic agent are selected from the group comprising or consisting of: angiotensin-converting enzyme (ACE) inhibitors; and/or angiotensin II receptor blockers; and/or vasopressin receptor antagonists; and/or beta blockers; and/or an inodilator (in particular, mirinone and/or enoximone and/or dobutamine and/or levosimendan); and/or omecamtiv mecarbil; and/or renin antagonist; and/or relaxin; and/or ularitide; and/or digoxin (Lanoxin); and/or vasodilators; and/or angiotensin II receptor antagonists (such as valsartan); and/or aspirin; and/or statins; and/or antihypertensive drugs (such as sacubitril); and/or calcium sensitisers; and/or ivabradine; and/or diuretics; and/or vasopressor (such as noradrenaline, dopamine, vasopressin, and/or angiotensin II); and/or adenosine antagonists; and/or aldosterone antagonists.

In a preferred embodiment, the one or more therapeutic agents comprise valsartan and/or sacubitril.

In an alternative preferred embodiment, the one or more therapeutic agents comprise vasodilators.

In an embodiment, the calcium sensitisers comprise levosimendan and/or dobutamine.

In an embodiment, the vasodilators comprise nitrovasodilators (such as nitroglycerin, and/or nitroprusside, and/or nesiritide).

In an embodiment, the diuretics comprise one or more of the group comprising or consisting of: furosemide; and/or bumetanide; and/or thiazide; and/or metolazone; and/or metolazone.

In an embodiment, the vasopressin receptor antagonists comprise tolvaptan and/or conivaptan.

In an embodiment, the adenosine antagonists comprise BG9719 and/or rolofylline.

In an embodiment, the ghrelin molecule is used with a medical device, such as a heart pump. By "used with", we include that one or more surface of the medical device is coated with the ghrelin molecule.

In a preferred embodiment, the one or more therapeutic agents are selected from the list comprising: relaxin; and/or ularitide; and/or inodilators; and/or vasopressors; and/or vasodilators.

In a more preferred embedment, the one or more therapeutic agents comprise vasodilators (in particular, noradrenaline and/or vasopressin and/or angiotensin II). In an alternative more preferred embedment, the one or more therapeutic agents comprise inodilators (in particular, mirinone and/or enoximone and/or dobutamine and/or levosimendan).

The listing or discussion of an apparently prior published document in this specification should not necessarily be taken as an acknowledgement that the document is part of the state of the art or is common general knowledge.

The present invention will now be described with reference to one or more non-limiting figures and example.
**Figure 1****.** Consort flow chart of screened and enrolled patients.
**Figure 2****.** Flow chart of investigations and ghrelin/placebo infusion.
**Figure 3****.** Acylated ghrelin concentrations ng/L. The notation "FU" denotes follow-up at 2-5 days.
**Figure 4****.** Cardiac output (CO) prior to, during and after ghrelin/placebo infusion. (A) In the ghrelin group, CO increased with infusion and fell after stop of infusion, all pair-wise comparisons significant. In the placebo group, there was no significant change in CO. (B and C) The individual patient absolute and percent changes in CO were different in the ghrelin (increase) vs. placebo (no change) groups.
**Figure 5****.** Stroke volume (SV) prior to, during and after ghrelin/placebo infusion. (A) In the ghrelin group, SV increased with infusion and fell after stop of infusion, all pair-wise comparisons significant. In the placebo group, there was no significant change in SV. (B and C) The individual patient absolute and percent changes in SV were different in the ghrelin (increase) vs. placebo (no change) groups.
**Figure 6A**. Heart rate (HR) measured manually prior to, during and after ghrelin/placebo infusion. (A) In the ghrelin group, HR fell slightly between baseline and 120 minutes. In the placebo group, there was no significant change in HR. (B and C) There were no statistically significant differences in absolute or percent changes in HR.
**Figure 6B****.** Median heart rate by continuous monitoring with Nexfin. Median heart rate is at 70 because many patients were paced at 70 beats per minute with cardiac resynchronization therapy (bi-ventricular pacemaker). For means, see Figure 6C.
**Figure 6C****.** Average heart rate by continuous monitoring with Nexfin.
**Figure 7****.** Estimated systemic vascular resistance (SVR) prior to, during and after ghrelin/placebo infusion. (A) In the ghrelin group, SVR fell between baseline and 60 minutes. In the placebo group, there was no significant change in SVR. (B and C) The absolute and percent changes in SVR were different in the ghrelin (decrease) vs. placebo (no change) groups.
**Figures 8-12****.** Blood pressure measured by Nexfin. Systolic (Fig 8), Diastolic (Fig 9) and mean (Fig 10) arterial pressure during and after ghrelin/placebo infusion and median (Fig 11) and mean (Fig 12) measured by Nexfin continuously. There were no changes or differences in changes between or within ghrelin or placebo groups.
**Figure 13****.** Left ventricular end-diastolic diameter (LVEDD) from echocardiography. There were no changes or differences in changes between or within ghrelin or placebo groups.
**Figure 14****.** Left ventricular end-systolic diameter (LVESD). There were no changes or differences in changes between or within ghrelin or placebo groups.
**Figure 15****.** Left ventricular ejection fraction (LVEF). There were no statistical different changes or differences in changes between or within ghrelin or placebo groups although there was a trend toward a greater absolute (p=0.12) and percent change (p=0.14) in EF with ghrelin.
**Figure 16****.** Tricuspid annular plane systolic excursion (TAPSE). There was a trend toward a greater difference in change between ghrelin and placebo.
**Figure 17****.** E/e' (pulse wave Doppler diastolic mitral inflow velocity [E] / tissue Doppler diastolic mitral annulus velocity [e'], a surrogate for LV filling pressures). There were no differences in change between ghrelin and placebo.
**Figure 18****.** Stroke volume (SV) measured by echocardiography. There was a significant difference in change of SV in favor of ghrelin (A), a trend toward a difference in absolute and percent change at 60 minutes, and a significant difference in absolute and percent change at 120 minutes, in favor of ghrelin.
**Figure 19****.** Cardiac output (CO) measured by echocardiography. There were trends toward a difference in change of CO in favor of ghrelin (A), a trend toward a difference in absolute and percent change at 60 and 120 minutes in favor of ghrelin.
**Figure 20****.** Segmental strain measured by echocardiography. There were numerical differences in favor of ghrelin but no statistical significance.
**Figure 21****.** QTc during infusion (INFUSION) and at baseline vs. 2-5 days follow-up (BL vs FU).
**Figure 22****.** Survival free from HF hospitalization up to 90 days.
**Figure 23****.** Survival free from HF hospitalization or heart transplantation or left ventricular assist device up to 90 days.
**Figure 24****.** Cardiomyocyte fractional shortening. Contractility measured by percent fractional shortening (FS) in isolated cardiomyocytes from SHAM and HF mice exposed to ghrelin, placebo, D-Lys (ghrelin antagonist), and D-Lys + ghrelin. Ghrelin increases contractility in a ghrelin receptor specific fashion. Since this is ex vivo, these effects are independent of loading conditions (left ventricular preload or afterload). In this MI model, HF cardiomyocytes have greater contractility due to compensatory responses. Fractional Shortening (FS).
**Figure 25A****.** Representative Ca2+ transients obtained from electrically stimulated adult cardiomyocytes. The Ca2+ transient amplitudes were not different after ghrelin treatment of cardiomyocytes neither in SHAM nor HF mice. Left: representative line scan recordings in cardiomyocytes loaded with fluo-3. Right: representative cardiomyocyte Ca2+ transients.
**Figure 25B****.** Average Ca2+ transients from adult murine cardiomyocytes. There was no difference between ghrelin vs. placebo in SHAM or HF mice. Data is presented as mean ±SEM.

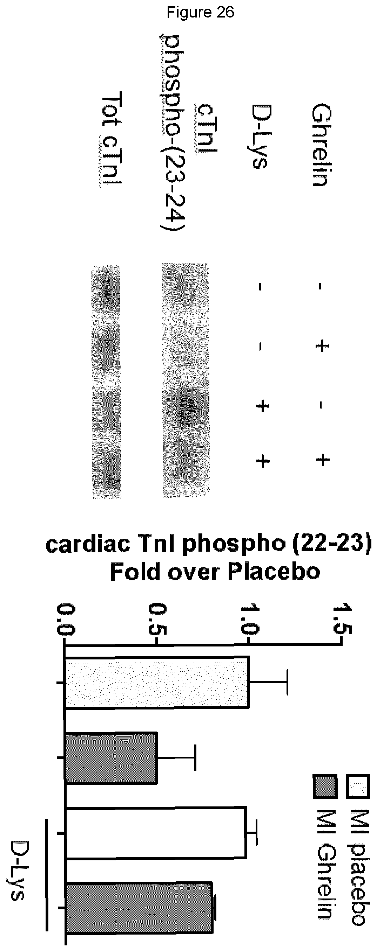

**Figure 26****.** Cardiac troponin I phosphorylation. Immunoblotting of protein lysates with an antibody against phosphorylation of serine 23-24 on troponin I. Left, example immunoblots blots of phosphorylated (ser23-24) cardiac troponin I (cTnl phospho (23-24) and total cardiac troponin I (cTnl). Ghrelin treatment of cardiomyocytes was associated with less cTnl phosphorylation (hypophosphorylation) without change in total troponin I expression. Co-incubation with the ghrelin receptor antagonist D-Lys blocked the effect of ghrelin-induced hypophosphorylation. Right, quantification of immunoblot band intensity representing phosphorylation levels of cTnl in cardiomyocytes isolated from mouse myocardial infarction heart. Two animals and one experiment per animal. In each experiment, 4 aliquots of cardiomyocytes isolated and treated as indicated. Mean±SEM of 2 experiments.
**Figure 27****.** Cardiomyocyte concentration of cAMP following Ghrelin incubation. Ghrelin reduces cAMP concentration in cardiomyocytes. The effect of ghrelin on cAMP was blocked in the presence of the ghrelin receptor antagonist D-lys 3.

### Example

The following example is included to demonstrate particular embodiments of the invention. It should be appreciated by those of skill in the art that the techniques disclosed in the example which follows represent techniques discovered by the inventor to function well in the practice of the invention, and thus can be considered to constitute preferred modes for its practice.

### Summary of the experimental work

### Introduction

Ghrelin is an endogenous appetite-stimulating peptide hormone with potential cardiovascular actions. It has long been considered that ghrelin is a potential inotrope agent, and assumed that it acts by increasing intracellular Ca²⁺ concentrations. Based on this understanding, it was widely expected that ghrelin would have a number of side effects that would be highly dangerous for a patient with acute heart failure (AHF), including increased heart arrhythmias, hypotension and ischemia (Abraham *et al.,* 2005; Cuffe *et* al., 2002; Mebasaa *et al,* 2007; and Packer *et al.,* 2013).

Contrary to that understanding, the inventors have surprisingly identified that ghrelin does not increase intracellular Ca²⁺ concentrations, but increases sensitivity to existing Ca².

This led the inventors to hypothesize that the previous assumptions of ghrelin acting as a conventional inotrope and thus causing hypotension, ischemia and arrhythmia were incorrect and, contrary to the previous misconceptions regarding ghrelin's function, ghrelin could act as a new type of inotrope and be used to treat AHF without causing hypotension, ischemia or arrhythmia.

To test this hypothesis, the inventors tested acylated ghrelin on heart failure patients. In order to avoid inadvertently causing harm to patients on which the ghrelin was tested, the inventors conducted a clinical study on patients with advanced heart failure. Advanced heart failure is similar to AHF, but it was expected that the severe side effects that would be observed in AHF in response to an inotrope would not occur to the same extent. Therefore, whilst administering ghrelin to a patient with advanced heart failure would provide a very similar indication of the safety and effectiveness of ghrelin if administered to an AHF patient, the tests could be conducted in a safer manner.

As explained below, in the clinical study the inventors confirmed that although ghrelin acted as an inotrope it did not increase Ca²⁺ concentrations. Most importantly, the inventors demonstrated that ghrelin did not cause any of the side effects that would be seriously problematic for a patient with AHF, such as increased heart arrhythmias, hypotension and ischemia. The experiments also show that treatment with acylated ghrelin leads to an improvement in signs which would also occur in AHF, demonstrating that acylated ghrelin would be effective in the treatment of that disease.

### Heart Failure And Its Forms And Outcomes

Heart failure (HF) is defined as inadequate cardiac output to meet metabolic demands or adequate CO only secondary to compensatory neurohormonal activation. Chronic HF (CHF) affects 2-3% of the population and up to 20% of the elderly (Go et al., 2014), and is the most common cause of hospitalization (acute heart failure; AHF) (Ambrosy et al., 2014 JACC 2014;63:1123-33).

AHF most often occurs when the chronic compensation is inadequate and is termed acute decompensated HF (ADHF). A majority of this is in patients with pre-existing CHF, thus the term acute decompensated chronic HF, ADCHF.

Although medical and device therapy have improved outcomes in CHF with reduced ejection fraction (HFrEF), 5-10% of patients suffer advanced (also termed severe or end-stage) CHF, characterized by reduced cardiac output (CO), progressive end organ failure, frequent repeated hospitalizations for ADCHF, and high risk of death.

In ADHF and ADCHF, in-hospital mortality ranges 5-10%, and median length of hospital stay is 5-8 days. Over 50% of patients are discharged with unresolved symptoms, and within 30 or 60 days in different studies, half have again worsening symptoms, one fourth are re-hospitalized and over 10% have died (Baker et al., 2003; Curtis et al., 2008; Gheorghiade et al., 2006; Go et al., 2014; Polanczyk et al., 2000). Mortality at 1 year in population wide registries is 25-35% (Lund, 2017). After an improvement in outcomes in the late 1990s, prognosis in CHF and ADCHD has not improved since 2000 (Baker et al., 2003; Curtis et al., 2008; Polanczyk et al., 2000; Thorvaldsen et al., 2016). Costs to society for HF are projected to increase 3-fold between 2010 and 2030 and most of this cost is related to ADCHF (Heidenreich et al., 2013).

Among hospitalizations for ADCHF, about 5% have severe hemodynamic compromise, with hypotension and shock, arrhythmias and global ischemia (even in the absence of obstructive coronary artery lesions), and the short-term mortality in this group exceeds 50%.

### Inotrope Therapy For Advanced HF And ADCHF

Most inotrope drugs work by increasing intracellular Ca²⁺ concentrations (e.g. milrinone, dobutamine); some work by increasing the sensitivity of contractile proteins to existing Ca²⁺ (levosimendan); but the former increases oxygen demand and all have multiple and complex mechanisms involving vasodilation. In clinical trials and clinical practice, existing inotropes indeed universally cause hypotension, tachy-arrhythmias and ischemia, and have neutral effect on or increase mortality (Abraham et al., 2005; Cuffe et al., 2002; Mebazaa et al., 2007; Packer et al., 2013). Three main classes of inotropes are in use: phosphodiesterase (PD) -inhibitors (e.g. milrinone); adrenergic agonists (e.g. dobutamine) and levosimendan. Specifically, in the randomized OPTIME CHF, milrinone vs. placebo caused significantly more hypotension and atrial arrhythmias and non-significantly more deaths (Cuffe et al., 2002). In the observational ADHERE Registry, both milrinone and dobutamine were independently associated with increased mortality (Abraham et al., 2005). Oral PD inhibitors were developed for longer term use but compared to placebo, caused more arrhythmias, vertigo (likely due to hypotension), cardiac death, and sudden death (Amsallem et al., 2005; Cohn et al., 1998; Packer et al., 1991; Uretsky et al., 1990). Intermittent intravenous dobutamine infusions reduced HF hospitalization (Oliva et al., 1999), but in a subsequent study that was stopped early and never published, it increased mortality (Dies et al., 1986) and in another there was no control group and only 3 of 13 patients survived the 26 week intermittent treatment study period (Krell et al., 1986). Levosimendan increases CO but also causes hypotension. It was neutral compared to dobutamine (which is harmful) in SURVIVE (Mebazaa et al., 2007) and increased hypotension and arrhythmias vs. placebo in REVIVE I and II (Packer et al., 2013). Thus, existing inotropes worsen outcomes, yet are sometimes used in severe ADCHF and advanced CHF when organ function is deteriorating, patients cannot be mobilized and discharged from hospital, when bridging to heart transplantation, and/or death is imminent.

### Ghrelin

Ghrelin ("ghre"= grow) was originally identified as a 28 amino acid peptide hormone that is the endogenous ligand for the growth hormone (GH) secretagogue receptor (GHSR), and partially acts by stimulating GH release (Kojima et al., 1999). Ghrelin has received attention mainly as a centrally acting appetite stimulant (Cummings et al., 2002). Ghrelin is released from the stomach in response to fasting and weight loss, whereas release is inhibited by food intake (Kojima et al., 1999; Kojima and Kangawa, 2005; Shiiya et al., 2002). Ghrelin is acylated ("activated") at amino-acid 3, and the acyl / acylated form is believed to be responsible for most of ghrelin's actions (Hosoda et al., 2000; Soares and Leite-Moreira, 2008).

### Ghrelin's Potential Cardiovascular Actions

Ghrelin is elevated in cachectic (Nagaya et al., 2001c) and non-cachectic CHF (Lund et al., 2009) but there appears to be resistance to the appetite stimulating effects, which resolves after heart transplantation (Lund et al., 2009). Beyond metabolic effects, ghrelin appears to have specific cardiovascular actions. Ghrelin receptors (growth hormone secreatagogue receptors; GHSR) are widely distributed in cardiac and skeletal muscle and endothelium (Papotti et al., 2000). It is possible that ghrelin is elevated in HF as a compensatory response to poor cardiac function, analogously to compensatory elevations of catecholamines and natriuretic peptides, and indeed acylation of ghrelin is increased in HF, potentially representing an adaptive compensatory response, and decreases post heart transplantation (Zabarovskaja et al., 2014).

### Previous Data On Ghrelin Treatment

In rat HF models, ghrelin increased CO and fractional shortening (Nagaya et al., 2001d); in rat myocardial infarction models, ghrelin reduced cardiac sympathetic activity and left ventricular (LV) remodeling (Schwenke et al., 2008; Soeki et al., 2008) and apoptosis (Yang et al., 2014). Small studies in human HF suggested ghrelin may improve cardiac output (Nagaya et al., 2001b) and left ventricular ejection fraction (EF), exercise capacity and muscle wasting (Nagaya et al., 2004) but these studies do not specify whether the ghrelin form used was acylated or not. The cardiovascular actions of ghrelin have been reviewed, but data are inconsistent, showing both positive and negative inotropic effects (Soares et al., 2005), and refer to variable forms of ghrelin and predominantly the non-acylated inactive form (Broglio et al., 2003b; Isgaard, 2013; Kishimoto et al., 2012; Leite-Moreira et al., 2008; Nagaya and Kangawa, 2003a, b, 2006; Nagaya et al., 2006). Furthermore, the safety, clinical efficacy and mechanisms of action of ghrelin in HF are unknown. GHSR agonists such as pralmorelin and hexarelin have fewer amino acids and no sequence similarity. Pralmorelin but not ghrelin or hexarelin improved CO in dogs with acute myocardial infarction (US patent US2004014671A1).

### Present Aims

Based on the unmet clinical need in Acute HF (AHF, ADHF, ADCHF), we conducted a randomized double-blind placebo-controlled trial of intravenous acyl ghrelin in patients with heart failure. To determine the underlying mechanism for any clinical effect, we also assessed contractility and cellular Ca²⁺ transients in response to ghrelin in isolated cardiomyocytes from healthy control and HF mice.

### Methods Human Trial

### Study Design And Setting

Between 17 February 2013 and 19 May 2015 we conducted a prospective double-blind, placebo-controlled, parallel-group, single centre randomized clinical trial with a one-time treatment of intravenous acyl ghrelin or placebo.

### Patients

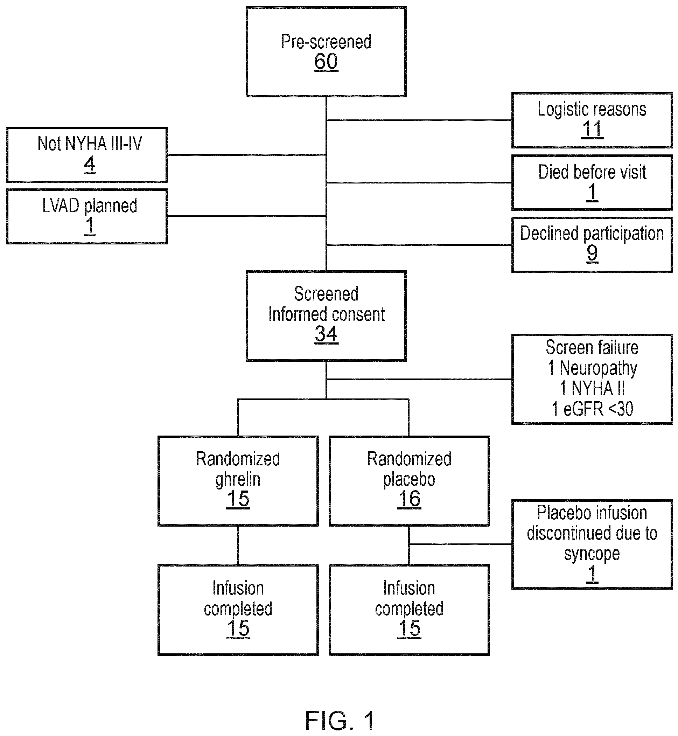

Patients were pre-screened in the Karolinska University Hospital heart failure clinic (Figure 1) and had symptoms and signs of advanced CHF (New York Heart Association [NYHA] class III-IV) and an EF of ≤40%. Detailed inclusion and exclusion criteria are listed in Table 1.

**Table 1. Inclusion and exclusion criteria**

| Inclusion criteria |
|---|
| 1. Heart failure defined as symptoms and signs of heart failure in judgement of investigator |
| 2. Left ventricular ejection fraction (EF) at screening and on day of treatment of ≤ 40% |
| 3. New York Heart Association (NYHA) class III or IV |
| 4. Written informed consent |

| Exclusion criteria |
|---|
| 1. Age < 18 |
| 2. Current smoker that cannot refrain from smoking from day of treatment |
| 3. Consumption of caffeine on day of treatment |
| 4. Current alcohol or drug abuse |
| 5. Acute coronary syndrome in last 3 months |
| 6. Received heart transplantation or left ventricular assist device ever |
| 7. Oxygen dependent lung disease |
| 8. Peripheral saturation by pulse oximetry <95% on study day |
| 9. Creatinine clearance (Cockgroft-Gault) or estimated glomerular filtration rate (eGFR, by MDRD) <30 mL/min on study day |
| 10. Dementia or inability to give informed consent |
| 11. Any solid organ transplant |
| 12. Current hormonal treatment |
| 13. Current immunosuppressive treatment other than corticosteroids |
| 14. Any inotropes within two weeks prior to study day |
| 15. Any gastrointestinal surgery except appendix, gall bladder, hernia, colon. |
| 16. Current gastrointestinal disease other than reflux disease or dyspepsia |
| 17. Known cirrhosis |
| 18. Systolic blood pressure <90 mmHg on study day |
| 19. Blood glucose >20 mmol/L after breakfast on study day that cannot be reduced to <10 with insulin |

### Preparation of Ghrelin

Ghrelin is a 28 amino acid peptide with a molecular weight of 3,371 g/mol. Half-time in plasma is 24-30 minutes. Concentrations in normal humans has variably been reported at 100-300 pmol/L (300-900 ng/L) for total ghrelin. There is limited data on acyl ghrelin but we recently measured acyl ghrelin in 41 healthy non-obese adults: fasting: mean±SD: 118±14 ng/L, 5-95th percentile 27-328 ng/L; trough 60 minutes post 260 kcal mixed meal: 80±12 ng/L, 5-95th percentile 21-293 ng/L. Synthetic acylated (active) human ghrelin (brand name Clinalfa Ghrelin (human) Acetate, product number 4071265; Bachem, Hauptstrasse 144, 4416 Bubendorf, Switzerland - hereafter referred to as ghrelin, was purchased from Bachem (Bubendorf, Switzerland) under license from Daiichi Sankyo (Tokyo, Japan). For each patient and treatment, a stock solution was prepared consisting of multiple vials of powder ghrelin (100 ug ghrelin / vial together with phosphate buffer), each vial dissolved in 1 mL sterile water for infusion (B. Braun, Germany) and visually inspected to be a clear and colorless solution, prior to adding 0.001 g (0.02 ml of 50 g/L stock) human albumin (for concentration 0.001 g/mL = 0.1%) to each vial and further mixed with normal saline (NaCl 9 mg/ml, B. Braun, Germany). The proportion of stock solution and saline were according to patient weight (Table 2), resulting in a total volume of 100 mL (to ensure the same volume infusion for all patients). The final intravenous infusion rate was 0.50 mL/min volume (total volume 60 mL), equivalent to 0.1 µg (30 pmol) /kg/min (total amount 12.0 µg / kg) of acyl ghrelin administered to all patients randomized to ghrelin. The infusion was given for 120 minutes and continued until all measurements at 120 minutes had been completed (thus delivering some additional ghrelin). The median total infusion duration was 171 minutes. Measurements were repeated 30 minutes after stopping infusion.

**Table 2. Stock solution, NaCl and final volume**

| Patient weight | Stock solution acyl ghrelin concentra tion (ug/ml) | Volume of stock solution (ml) | Amount acyl ghrelin (ug) | Volum NaCl (ml) | Infusion solution acyl ghrelin concent ration (ug/ml) | Total volume (ml) Infused over 120 min* | Total acyl ghrelin (ug) infused over 120 min |
|---|---|---|---|---|---|---|---|
| 50 | 100 | 10 | 1000 | 90 | 10 | 60 | 600 |
| 55 | 100 | 11 | 1100 | 89 | 11 | 60 | 660 |
| 60 | 100 | 12 | 1200 | 88 | 12 | 60 | 720 |
| 65 | 100 | 13 | 1300 | 87 | 13 | 60 | 780 |
| 70 | 100 | 14 | 1400 | 86 | 14 | 60 | 840 |
| 75 | 100 | 15 | 1500 | 85 | 15 | 60 | 900 |
| 80 | 100 | 16 | 1600 | 84 | 16 | 60 | 960 |
| 85 | 100 | 17 | 1700 | 83 | 17 | 60 | 1020 |
| 90 | 100 | 18 | 1800 | 82 | 18 | 60 | 1080 |
| 95 | 100 | 19 | 1900 | 81 | 19 | 60 | 1140 |
| 100 | 100 | 20 | 2000 | 80 | 20 | 60 | 1200 |
| 105 | 100 | 21 | 2100 | 79 | 21 | 60 | 1260 |
| 110 | 100 | 22 | 2200 | 78 | 22 | 60 | 1320 |
| 115 | 100 | 23 | 2300 | 77 | 23 | 60 | 1380 |
| 120 | 100 | 24 | 2400 | 76 | 24 | 60 | 1440 |
| 125 | 100 | 25 | 2500 | 75 | 25 | 60 | 1500 |
| 130 | 100 | 26 | 2600 | 74 | 26 | 60 | 1560 |
| 135 | 100 | 27 | 2700 | 73 | 27 | 60 | 1620 |
| 140 | 100 | 28 | 2800 | 72 | 28 | 60 | 1680 |
| 145 | 100 | 29 | 2900 | 71 | 29 | 60 | 1740 |
| 150 | 100 | 30 | 3000 | 70 | 30 | 60 | 1800 |

| | | | | | | | |
|---|---|---|---|---|---|---|---|
| *The last data collection during infusion occurred at 120 minutes. The infusion was stopped after all data had been collected, at a median time of 171 minutes after start of infusion. | | | | | | | |

### Preparation of Placebo

A solution of physiological normal saline (NaCl 9mg/mL, B. Braun, Germany) was infused at the same volume and rate (0.50 mL/min) and total duration as the ghrelin infusion.

### Procedures And Data Collection

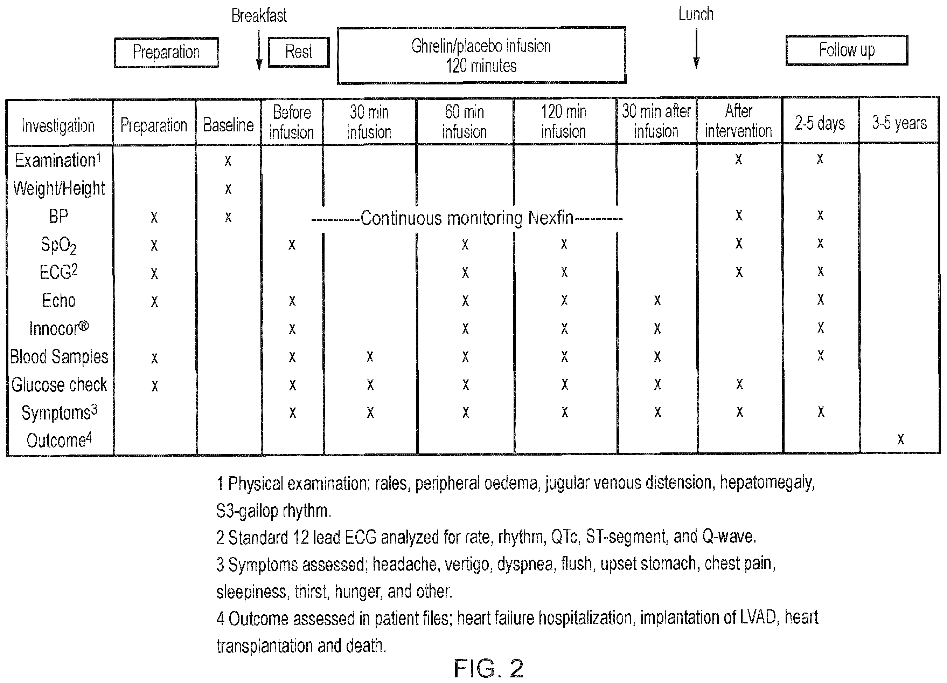

Patients potentially eligible during pre-screening and providing written informed consent reported to the laboratory at 8:00 am in the fasting state. Examinations are shown schematically in Figure 2. Manual brachial artery blood pressure and O₂ saturation (peripheral pulse oximetry) were measured, and EF ≤ 40% confirmed by echocardiography (visual estimate or Simpson's method). Two peripheral venous catheters were inserted and blood samples were collected and analyzed including estimated glomerular filtration ratio (eGFR) and plasma glucose (as part of inclusion/exclusion criteria). Patients not meeting eligibility criteria at this time, including EF ≤40%, were excluded and considered screening failures. Thereafter the patients underwent additional examinations, and then a standardized breakfast of 500 kcal (since fasting increases endogenous ghrelin levels) consumed ad lib without coffee or tea. Prior to starting infusion, blood samples and exams were repeated.

Patients were randomized in parallel by block randomization in groups of 4 using paper envelopes to ghrelin or placebo and the infusion was given by antecubital vein over 120 minutes. A dedicated study nurse prepared ghrelin and placebo for infusion and patients and all other investigators were blinded. Immediately prior to, during, and after the 120 minute infusion and also 30 minutes after stopping the infusion, blood samples were drawn and symptoms, signs, echocardiography, ECG, and non-invasive hemodynamics were assessed (Figure 2). Patients were discharged to home and returned 2-5 days later for repeat measurements. Patients were followed prospectively for morbidity and mortality outcomes.

### Pre-Specified Trial Efficacy Outcomes

The primary efficacy outcome was difference between ghrelin and placebo in change in CO from start of infusion (time 0) to end of infusion (time 120 minutes). Numerous secondary outcomes related to hemodynamics, echocardiography findings and plasma biomarkers in response to the 120 minute infusion, 30 minutes after stopping the infusion and 2-5 days after stopping the infusion were also assessed (see results).

### Pre-Specified Trial Safety Outcomes

Safety outcomes included reduction in systolic blood pressure, hypotension, and symptomatic hypotension, prolongation of the QTc, ischemia, and arrhythmia during or after infusion, and clinical outcomes.

### Data Collection Methods And Definitions

### Cardiac output and hemodynamics

Non-invasive resting CO in was assessed in duplicate at each measurement using the Innocor^{®} device (Innovision, Odense, Denmark). The Innocor is a non-invasive device that measures pulmonary blood flow using an inert gas rebreathing technique, and measures VO₂ directly. It has been validated for CO and VO₂ at rest and exercise (Gabrielsen et al., 2002; Stahlberg et al., 2009). The coefficient of variance is low (VO₂ < 2%; CO 5-7%)(Stahlberg et al., 2009), similar to the gold standard Fick method (Warburton et al., 1999) and superior to other non-invasive methods (Warburton et al., 1999). In the absence of a significant intrapulmonary shunt, pulmonary blood flow as calculated by inert gas rebreathing measured by the Innocor^{®} has been shown to provide a reliable estimate of CO (Stahlberg et al., 2009).

The Innocor^{®} directly measures pulmonary blood flow, VO₂ and SpO₂. From these variables, shunt fraction, cardiac output, SVO₂ and A-V O₂ difference was then calculated using standard formulas. Beat-by-beat ECG, blood pressure and the first derivative of the pressure signal (+ dP/dt) were measured using a Nexfin^{®} device (described below). Fifteen minutes of continuous beat-by-beat Nexfin data were averaged at the time of Innocor measurements. Estimated systemic vascular resistance (eSVR) was subsequently calculated as mean blood pressure/cardiac output x 80. Stroke volume (ml) was calculated as Innocor derived cardiac output/Nexfin derived heart rate x 1000.

### Continuous hemodynamic and EKG monitoring

A plethysmographic based (finger-cuff) approach was used to measure blood pressure (Nexfin^{®}, BMEYE B.V., Amsterdam, The Netherlands). Nexfin was also used for heart rate monitoring. Heart rate and blood pressure data were measured continuously, beat-by-beat, throughout the study protocol. The data was then averaged over 15 minutes following the Innocor measurements. In addition EKG was assessed before start of and during infusion at 60 and 120 minutes and after the *ad lib* lunch. QT intervals were measured in the precordial lead V5 in 12-lead surface ECGs, at 50mm/sec paper speed and 10mm/mV amplitude. Heart rate corrected QT intervals (QTc) were manually calculated with the Bazett formula, QTc = QT/ RR^{1/2}, using the RR interval preceding the measured QT interval. The mean QTc from 3 heart beats was recorded.

### Echocardiography

Echocardiography was performed by a technician blinded to treatment allocation and all echo images were analyzed and interpreted by one independent investigator blinded to treatment allocation and clinical history of the patients. Two dimensional images were recorded with a Vivid 7/E9 ultrasound system (GE, Horten, Norway) with a 3 MHz Doppler transducer. A detailed echocardiographic examination including dimensions, cardiac systolic and diastolic function, valve performance and systolic pulmonary artery pressure was performed at baseline (Table 5). A shorter protocol including left ventricular dimensions and function, stroke volume and cardiac output was used for comparison of changes over time (Table 9 and 10). Left ventricular end-systolic and end-diastolic volumes and left ventricular ejection fraction (LVEF) were measured using the modified biplane Simpson's method. LVEF was also measured using the Teichholz method. Because of missing data on LVEF by the Simpson's method due to low image quality, changes in LVEF over time was calculated using the Teichholz method. Left atrial volumes were calculated using the biplane area length method from the apical four- and two-chamber views and indexed to the body surface area. The E/e' ratio was calculated using the peak E-wave velocity of the mitral inflow and the averaged of septal and lateral tissue Doppler recordings. Tricuspid annular peak systolic excursion (TAPSE) was assessed with M-mode echocardiography. Speckle tracking was used for left ventricular longitudinal strain. Changes in strain over time was calculated using the average of the regional strain from septal and inferior segments. Stroke volume was derived from the left ventricular outflow tract (LVOT) area and the LVOT ventricular time integral (VTI) (LVOT area x LVOT VTI). Cardiac output was calculated as the echo derived stroke volume x heart rate.

**Table 5. Baseline echocardiography data. P values by Wilcoxon rank sum test for continuous variables and by Fischer's exact test for categorical variables. Continuous variables are presented as medians (IQR) and categorical variables as %.**

| **Variable** | **Missing n (%)** | **Ghrelin** | **Placebo** | **p value** |
|---|---|---|---|---|
| **LVEDD (mm)** | 2(7) | 69 (60-75) | 67 (61-73) | 0.878 |
| **LVESD (mm)** | 2 (7) | 63 (53-67) | 60 (51-66) | 0.983 |
| **LVESV (ml)** | 14 (45) | 145 (92-218) | 139 (50-176) | 0.242 |
| **LVEDV (ml)** | 14 (45) | 206 (163-304) | 197(100-244) | 0.329 |
| **Septum (mm)** | 2 (7) | 10 (8-12) | 11 (9-13) | 0.367 |
| **Posterior wall (mm)** | 2 (7) | 7 (7-8) | 8 (7-10) | 0.346 |
| **LV mass (g)** | 2 (7) | 248 (194-337) | 293 (231-377) | 0.183 |
| **LVEF Simpson (%)** | 14 (45) | 28 (25-41) | 30 (24-36) | 0.922 |
| **LVEF visual (%)** | 1 (3) | 30 (20-35) | 20 (20-33) | 0.192 |
| **LVEF Teicholz (%)** | 2 (6) | 29 (20-34) | 19 (13-33) | 0.169 |
| **SV (ml)** | 2 (6) | 44 (39-76) | 56 (40-67) | 0.896 |
| **CO (mL/min)** | 2 (6) | 3560 (30904660) | 3560 (2930-4340) | 0.760 |
| **LAV (ml)** | 2 (7) | 90 (70-141) | 120 (75-155) | 0.325 |
| **LAVi (ml/m²)** | 2 (7) | 47 (34-63) | 53(41-67) | 0.513 |
| **RA area (cm²)** | 1 (3) | 25 (21-30) | 26 (19-31) | 0.852 |
| **MR ≥2, n (%)** | 1 (3) | 1 (7) | 0 | 1.000 |
| **TR ≥2, n (%)** | 1 (3) | 1 (7) | 2 (13) | 0.179 |
| **AR ≥2, n (%)** | 1 (3) | 1 (7) | 0 | 0.651 |
| **MS, n** | | 0 | 0 | |
| **AS, n** | | 0 | 0 | |
| **TAPSE (mm)** | 1 (3) | 13 (10-17) | 11 (6-16) | 0.271 |
| **SPAP (mmHg)** | 8 (25) | 39 (33-50) | 30 (30-45) | 0.304 |
| **E/e' sept** | 1 (3) | 17 (13-22) | 20 (14-28) | 0.281 |
| **E/e' lateral** | 1 (3) | 13 (9-17) | 12 (8-18) | 0.917 |
| **e' septal (cm/s)** | 0 | 5 (4-6) | 5 (3-6) | 0.345 |
| **e' lateral (cm/s)** | 0 | 8 (4-9) | 8 (5-11) | 0.567 |

Abbreviations: LVEDD, left ventricular end diastolic diameter; LVESD, left ventricular end systolic diameter, LVESV, left ventricular end systolic volume; LVEDV, left ventricular end diastolic volume; LEVF, left ventricular ejection fraction; SV, stroke volume; CO, cardiac output; LV mass, left ventricular mass; LAV, left atrial volume; LAVi, left atrial volume index; RA, right atrial; MR, mitral regurgitation; TR, tricuspid regurgitation; AR, aortic regurgitation; MS, mitral stenosis; AS, aortic stenosis; TAPSE, tricuspid annular plane systolic excursion; SPAP, systolic pulmonary artery pressure; E, E-wave mitral inflow; E/e', E-wave/e' ratio; e', mitral relaxation velocity.

**Table 9. Acute echocardiography effects of ghrelin vs. placebo**

| **Ghrelin** | | | | | **Placebo** | | | | |
|---|---|---|---|---|---|---|---|---|---|
| **Variable** | **BL** | **INF + 60min** | **INF + 120 min** | **+ 30min** | **BL** | **INF + 60min** | **INF + 120min** | **+30min** | **p-interaction** |
| **LVEDD (mm)** | 66±9 | 67±9 | 67±10 | 65±9 | 69±11 | 68±11 | 68±11 | 69±11 | 0.70 |
| **LVESD (mm)** | 58±9 | 58±11 | 57±11 | 56±11 | 61±12 | 61±14 | 60±13 | 61±13 | 0.98 |
| **LVEF (%)** | 27.4±7.4 | 29.1±11.5 | 29.8±11.4 | 30.1±11.2 | 26.7±12.2 | 24.9±15.4 | 25.4±13.2 | 25.5±13.1 | 0.29 |
| **TAPSE (mm)** | 13.0±5.9 | 13.1±4.8 | 14.2±5.4 | 14.1±5.4 | 11.7±5.0 | 9.8±3.8* | 10.7±5.6 | 11.1±4.5 | 10 0.075 |
| **Strain (%)^{†}** | 11.9±6.3 | 13.4±4.6 | 13.2±5.8 | 12.46±4.1 | 11.4±8.1 | 11.6±5.8 | 11.4±7.6 | 11.8±9.3 | 0.51 |
| **E/e' (ratio)** | 15.2±5.5 | 17.1±9.5 | 16.3±8.9 | 15.3±6.6 | 18.1±6.7 | 19.8±8.2 | 18.2±5.9 | 19.8±5.6 | 0.97 |
| **SV (ml)** | 54.3±18.4 | 56.3±21.4 | 59.5±25.5 | 62.2±23.8 | 59.1±23.3 | 49.8±16.6* | 50.7±13.5* | 52.6±19.3 | 0.021₁₅ |
| **CO (L/min)** | 3.8±1.5 | 3.9±1.3 | 3.9±1.4 | 4.1±1.5 | 4.1±2.0 | 3.4±1.0 | 3.5±1.0 | 3.6±1.3 | 0.12 |

| | | | | | | | | | |
|---|---|---|---|---|---|---|---|---|---|
| *= p<0.05 vs. baseline within treatment group †= n=13 in ghrelin group and 10 in placebo group | | | | | | | | | |

**Table 10. Echocardiography at baseline and at 2-5 day follow-up**

| | **Ghrelin** | | **Placebo** | | |
|---|---|---|---|---|---|
| **Variable** | **Baseline** | **2-5 days FU** | **Baseline** | **2-5 days FU** | **p-interaction** |
| **LVEDD (mm)** | 66±9 | 66±10 | 66±9 | 69±11 | 0.82 |
| **LVESD (mm)** | 58±9 | 57±11 | 61±12 | 61±10 | 0.90 |
| **LVEF (%)** | 27.4±7.4 | 28.5±11.9 | 26.7±12.2 | 24.9±14.7 | 0.62 |
| **TAPSE (mm)** | 13.0±5.9 | 11.8±6.0 | 11.7±5.0 | 11.3±4.8 | 0.81 |
| **Strain (%)tt** | 11.9±6.3 | 10.4±6.4 | 11.4±8.1 | 10.3±5.7 | 0.93 |
| **E/e' (ratio)** | 15.2±5.5 | 16.2±8.3 | 18.1±6.7 | 16.1±2.8 | 0.37 |
| **SV (ml)** | 54.3±18.4 | 52.5±20.6 | 59.1±23.3 | 50.43±17.9 | 0.52 |
| **CO (L/min)** | 3.8±1.5 | 3.8±1.3 | 4.1±2.0 | 3.6±1.3 | 0.52 |

| | | | | | |
|---|---|---|---|---|---|
| Longer term effects comparing baseline (before infusion starts) vs. follow-up approximately 2-5 days after infusion. *=p<0.05 vs. baseline within group. †=p<0.05 ghrelin vs. placebo at same time point. ††= n=12 in ghrelin group and 10 in placebo group | | | | | |

### Symptoms and signs

The following symptoms were assessed as yes or no prior to infusion and after 30, 60, 120 min and 30 min after infusion and at the 2-5 days follow-up; headache, dizziness, dyspnea, central chest pain, flush, sleepiness, upset stomach and other symptoms. The following signs were recorded prior to start of infusion and after lunch; rales, peripheral oedema, jugular venous distension, hepatomegaly and S3 gallop.

### Blood samples

Blood samples were collected in 1) morning fasting state baseline, 2) after standardized breakfast prior to intervention, 3) after 30 min infusion, 4) after 60 min infusion and 5) after 120 min infusion, 6) 30 min after completed infusion and 7) at follow-up 2-5 days later (Figure 2). Blood was collected in ethylenediaminetetraacetic acid (EDTA) and serum tubes and immediately centrifuged and plasma and serum was aliquoted and stored in - 70 °C until analysis.

In samples dedicated for ghrelin measurements a protease inhibitor cocktail was prepared, consisting of 5.5 µl 10 mM KR-62436 (DPP4 inhibitor) in DMSO and a SIGMAFAST^{®} protease inhibitor tablet (both produced by Sigma-Aldrich Corp., St. Louis, MO, USA) dissolved in 2100 µl of distilled water (50x stock). Blood samples were drawn using 6 mL EDTA plasma tubes, immediately put on ice and 160 µl of the 50x protease inhibitor cocktail was added. Tubes were vortexed for 10 seconds and centrifuged at 4 °C, 10 min, 2500 relative centrifugal force (RCF, or g). The resulting supernatant (plasma) was then pipetted into Eppendorf tubes (450µl each) and immediately frozen at -70°C until analyzed.

### Serum and plasma biomarkers

Creatinine clearance was calculated according to the Cockcroft-Gault equation; ([140-age] * weight in kg * 1.23) / creatinine * 0.85 (if woman). Renal function was measured by creatinine and cystatin-C at Karolinska University Hospital core laboratory. NT-proBNP was analyzed by proBNPll (Roche Diagnostics, Bromma, Sweden). Plasma glucose measurements were from EDTA-containing whole venous blood tubes and analyzed with a photometric point-of-care technique, the HemoCue^{®} Glucose 201 RT (Ängelholm, Sweden). Troponin T was analyzed in the Karolinska University Hospital core laboratory.

### Acyl ghrelin and pharmacokinetics

Plasma concentrations of active (acylated) ghrelin were assayed by a custom ELISA using electrochemiluminescence detection. Plasma samples were thawed and vortexed. They were then analyzed in duplicate on 96-well multispot plates (Meso Scale Diagnostics, Rockville, MD, USA) coated with capture antibodies against acyl-ghrelin according to manufacturer instructions. The Meso Scale Diagnostics Sector Imager 2400 was used to read the plates. The coefficients of variation (CV%) calculated from plasma concentrations after curve fitting were 9.3/3.5 (intra/inter-assay CV%). The lower limit of detection was 3.2 ng/L and upper limit was set at 10,000ng/L.

### Safety outcomes

### Hypotension

Hypotension was assessed in two ways: The difference in change in blood pressure between the groups was quantified, and hypotension in any one patient was defined as a drop of systolic blood pressure of > 20 mm Hg or to < 80 mm Hg.

### Arrhythmias

The difference in change in heart rate between the groups was quantified. Bradycardia was defined as a reduction in heart rate of >15 beats per minute, to a heart rate <40 beats per minute, an increase in PQ time by > 20 ms, or AV block II-III. In individual patients, tachycardia was defined as an increase in heart rate by > 10 beats per minute or heart rate > 110 beats per minute. Ventricular extraystoles were quantified and ventricular tachycardia was defined as non-sustained if ≥ 3 beats in a row up to ≤ 30 seconds, and sustained if >30 seconds. Supra-ventricular arrhythmia was defined as ectopic atrial tachycardia, atrial flutter or atrial fibrillation, or AV-nodal tachycardia of > 3 seconds duration. Ghrelin may activate hERG channels and as such cause a prolongation of the QTc interval on the EKG, which theoretically could predispose to arrhythmia. Therefor we monitored QTc.

### Ischemia

Ischemia was assessed in two ways: The difference in change in troponin T between the groups was quantified, and ischemia in any one patient was defined as symptoms of ECG changes consistent with ischemia, or a rise in troponin T of >50%.

### Clinical outcomes

Patients were followed in the medical record until 30 April 2017. Outcomes were freedom from all-cause death; all cause death or HF hospitalization; all cause death or heart transplantation or left ventricular assist device, and a combination of all these.

### Statistics

For baseline characteristics, continuous variable were compared by the Wilcoxon rank sum test for continuous variables and by Fischer's exact test for categorical variables.

In order to compare the effect of ghrelin vs. placebo on continuous variable over time, a 2-way repeated measures ANOVA (2W-RM-ANOVA) was used to evaluate treatment effect within treatment groups. The 2W-RM-ANOVA has two factors: treatment (2 levels: ghrelin or placebo) and time (3 levels: baseline, after 60 minutes infusion, and after 120minutes infusion). The main analysis to be considered in this statistical model is the interaction (treatment x time) where we test the significance of the null-hypothesis that the differences between treatments are the same at all time points. Time factor makes little sense in this analysis since we expect a time effect. Treatment factor makes little sense in this analysis since we do not expect a ghrelin vs. placebo group difference at baseline. Post-hoc testing, Tukey adjusted p-values for repeated measures within each treatment group and Sidak between treatment groups were performed to test for significant changes. We also assessed differences between the treatment groups at 60 and 120 minutes using unpaired t-tests of absolute and % delta values at 60 and 120 min time points after infusion start within groups by Wilcoxon sign-rank test and between groups by Wilcoxon rank sum test

To compare times to outcomes, survival and event-free survival was charted with Kaplan-Meier curves and compared by the log-rank test.

### Sample size

The power calculation was based on difference in change of CO and conducted as described in Table 3. With a power of 80%, 2-sided alpha of 0.05 and an assumed 10% minimal treatment difference the sample size required 10 patients in each group. Accounting for potential missing data measurements and additional margins, sample size was set to 15 patients in each group.

**Table 3: Sample size calculation**

| **Assumptions:** | | | |
|---|---|---|---|
| Standard deviation of difference in change of CO from baseline to 120 minutes (L/min) | 0.4 | | |
| Mean CO (L/min) | 4.2 | | |
| 5 % increase from 4.2 L/min | 0.21 | | |
| 10 % increase from 4.2 L/min | 0.42 | | |
| 15 % increase from 4.2 L/min | 0.63 | | |
| Significance set to 0.05 (2-sided) | | | |
| power: 0.80 | | | |
| **Minimal detectable difference (treatment effect)** | **Treated n =** | **total n=** | **including additional 10 % for anticipated missing data** |
| 5% | 31 | 62 | 68 |
| 10% | 10 | 20 | 22 |
| 15% | 6 | 12 | 13 |
| **Decision: 10% with margins -->** | **15** | **30** | **30** |

### Ethics

Ghrelin has been used previously in studies in healthy humans and patients. The trial was conducted according to International Conference on Harmonization and Good Clinical Practice guidelines and the Declaration of Helsinki. The trial was approved by the local (Stockholm) ethics committee (number 2008/1:12 and 2008/1695-31). In this physiological study, the ethics committee waived the need for medical products agency (MPA) approval, based on a previous waiver for the same treatment in another study of gastrointestinal effects (MPA waiver number 159:2007/16373; ethics Stockholm number 2007/119-31/1). All patients provided written informed consent.

### Methods of the Rodent Studies

### Mouse Model Of Myocardial Infarction-Induced Heart Failure

Twelve-week old C57BL6 mice were anesthetized with gas mixture of oxygen and isoflurane (2-3 %). Myocardial infarction (MI) was induced in mice by permanent ligation of the left coronary artery, as previously described (Perrino et al., 2013). Briefly, mice underwent thoracotomy and subsequent left coronary ligation; ~80% survived during the follow-up period (4-6 weeks). SHAM-operated animals underwent the same procedure without coronary artery ligation (SHAM). At study termination, after sedation, mice were euthanized through cervical dislocation.

### Mouse Transthoracic Echocardiography

Heart function was noninvasively monitored in both groups by transthoracic echocardiography using the Philips HDI 5000 imaging system before the termination. Echocardiograms were performed with a 7-15 MHz CL 15-7 scanning head. Heart contractility was measured as % left ventricular fractional shortening using the following formula: LVd-LVs/LVd *100, where LVd and LVs stand for left ventricular diastolic and systolic dimensions respectively.

### Mouse Cardiomyocyte Isolation

After euthanasia, single cardiomyocytes were isolated from the left and right ventricles (mouse hearts are dominated by left ventricles) and atria were excluded, following the protocols developed by the Alliance for Cellular Signalling (AfCS Procedure Protocol ID PP00000 125) as previously described (Pironti et al., 2016).

### Cytosolic [Ca2+]l And Cell Shortening In Response To Ghrelin And Placebo

Mouse cardiomyocytes were incubated with a cell permeable form of fluorescent indicators Fluo-3 AM followed by washing for >5 min as previously described (Andersson et al., 2011). Cardiomyocytes were plated on laminin coated glass bottom dishes. The dishes were placed in a custom built perfusion/stimulation chamber and continuously perfused with O₂/CO₂ (95/5%) bubbled Tyrode solution with the following composition (in mM): NaCl 121, KCI 5.0, CaCl₂ 1.8, MgCl₂ 0.5, NaH2PO₄ 0.4, NaHCO₃ 24, EDTA 0.1, glucose 5.5. The cardiomyocytes were stimulated to contract using an electrical field between two platinum electrodes attached to the perfusion/stimulation chamber. Measurements were only performed in cardiomyocytes that contracted upon electrical stimulation and displayed normal morphology (e.g. striated, "brick shaped"). Cells that displayed spontaneous contractions were not measured. Fluorescence was measured using a confocal microscope Bio-Rad MRC 1024 unit attached to a Nikon Diaphot inverted microscope (40-60x oil immersion lenses). Confocal images were analyzed off line using ImageJ (National Institutes of Health; available at http://rsb.info.nih.gov/ij). Line scan confocal images were obtained from paced cardiomyocytes with the line scan running along the long axis of the cell. Changes in the emitted fluorescence, representing changes in free cytoplasmic Ca²⁺, were quantified. The amplitude of Ca²⁺ transients was measured as the change in the fluo-3 fluorescence signal (F) divided by the fluorescence immediately before a stimulation pulse given under control conditions (F0). Ca²⁺ transient decay time constant (Tau) was quantified by fitting the decay to an exponential decay function in the GraphPad Prism software (La Jolla, Ca, USA). Fractional cell shortening was calculated from the line scan images as the fractional change in the cell length at rest and during maximal contraction.

The myocytes were superfused with physiological buffer (Tyrode solution) or for the pharmacological experiments Tyrode + Ghrelin (100 nM) or D-Lys 3 (D-Lys 3 GHS-R1a antagonist 3 µM). All cells were perfused for 15 min with respective solution before measurements, except in some experiments where D-Lys 3 (3 µM) was introduced in perfusion system 10 minutes before adding Ghrelin. All experiments were performed at room temperature (~24°C).

### Ghrelin Treatment Of Cardiomyocytes

The myocytes were superfused with physiological buffer (Tyrode solution) with or without Ghrelin (Bachem, Bubendorf, Switzerland) (100 nM). All cardiomyocytes were superfused for 15 min with respective solution before measurements. An antagonist against the ghrelin receptor GHS-r1a (D-Lys 3; 3 µM), was used in some experiments. In those experiments, D-Lys 3 was introduced in superfusion system 10 minutes before adding Ghrelin.

### Mouse Cardiomyocyte Protein Immunoblotting

Isolated cardiomyocytes were treated for 15 min in different conditions (Placebo, Ghrelin, D-Lys 3 GHS-R1a, D-Lys 3 GHS-R1a + Ghrelin). Thereafter cardiomyocytes were pelleted and homogenated. Protein lysates were separated by electrophoresis and transferred onto membranes. Membranes were incubated with primary antibody: rabbit phospho-troponin I (cardiac) (Ser 23/23) antibody (Cell Signaling #4004), mouse troponin 1 (Millipore MAB1691A). Then infrared-labelled secondary antibodies (IRDye 680 and IRDye 800, 1:5000, Licor) were used. Immunoreactive bands were analyzed using the Odyssey Infrared Imaging System. Band densities were quantified with Image J, normalized to GAPDH and final data expressed as fold increase compared to Placebo group.

### cAMP Measurement

The intracellular cAMP concentration was measured using the cAMP direct Immunoassay kit (Abcam ab65355). Briefly, frozen cardiomyocytes pellets from groups described above (Placebo, Ghrelin, D-Lys 3 GHS-R1a + Ghrelin) were homogenized on ice using volume of 0.1 M HCl to obtain a protein concentration of 1 mg/ml. Acetylated supernatants of samples were used for the incubation with antibodies in ELISA plates according to the kit manufacturer. Finally, the optic density absorbance at 450 nm was analyzed using a microplate reader (Biotek, Synergy 2). The concentration of cAMP was normalized, dividing the resulting readout (pmol/ml) by the total protein concentration (mg/ml) for each sample. The experiments were conducted by an operator blinded regarding the treatment of the animals.

### Statistics

Statistical comparison between 2 groups were performed using students t-test (unpaired). For comparison between >2 groups, ANOVA was used. A p < 0.05 was used as definition for statistical significance. Average data was presented as mean ± standard error of the mean (SEM).

### Ethical Approval

All animal experiments were performed under the ethics approvals N19/15, N273/15.

### Results of the Human Trial

### Patients

Thirty-four patients were identified from Karolinska University Hospital heart failure clinic and consented to the trial. On the morning of study, one patient was excluded because unable to lie still due to neuropathy and leg pain, one patient because of creatinine clearance 26 mL/min (< 30 mL/min was exclusion criterion), and one patient because of NYHA class II (NYHA III-IV was inclusion criterion) and EF 43% (EF ≤40% was inclusion criterion). Thus thirty-one patients were randomized. One patient (placebo) experienced repeated urinary urgency and dizziness early after start of infusion and infusion was interrupted and patient excluded. Thirty patients remained for analysis (15 ghrelin; 15 placebo).

### BASELINE CHARACTERISTICS

Baseline characteristics are shown in Table 4 and were similar between the treatment and placebo groups. Median age was 71 and 70, respectively, and 13% were women in both groups. Baseline echocardiography data are shown in Table 5. Median EF (Simpson) was 30% and 28%, respectively.

**Table 4. Baseline clinical characteristics. Continuous variables are presented as medians (IQR) and categorical variables as n and %. p values were assessed by Wilcoxon rank sum test for continuous variables and by Fischer's exact test for categorical variables. Additional biomarkers are shown in Table 12.**

| **Variable** | | | **Missing n (%)** | **Ghrelin n=15 (50%)** | **Placebo n=15 (50%)** | **p value** |
|---|---|---|---|---|---|---|
| **Demographics** | | | | | | |
| Age (years) | | | | 70 (62-76) | 71 (55-75) | 0.787 |
| Male sex, n (%) | | | | 13 (87) | 13 (87) | 1.000 |
| BMI (kg/m²) | | | | 29 (25-30) | 30 (28-32) | 0.097 |
| **Vital parameters** | | | | | | |
| SBP (mmHg) | | | | 105 (95-120) | 100 (100-120) | 0.738 |
| DBP (mmHg) | | | | 70 (65-80) | 70 (65-80) | 0.655 |
| SpO₂ (%) | | | | 97 (95-98) | 96 (95-97) | 0.421 |
| **HF related** | | | | | | |
| Duration of HF (years) | | | | 8 (2-16) | 9 (5-13) | 0.967 |
| NYHA class III, n (%) | | | | 14 (93) | 14 (93) | 1.000 |
| NYHA class IV | | | | 1 (7) | 1 (7%) | 1.000 |
| Ischemic cardiomyopathy, n (%) | | | | 7 (47) | 11 (73) | 0.264 |
| **Biochemistry** | | | | | | |
| P-acyl ghrelin fasting (ng/L) (normal fasting 5%-95% range 27-328 ng/L*) | | | | 70 (32-144) | 82(30-97) | 0.724 |
| **Medical history n (%)** | | | | | | |
| Previous cardiovascular disease | | | | 11 (73) | 11 (73) | 1.000 |
| Whereof | | | | | | |
| | Previous MI | | | 6 (55) | 11 (100) | 0.035 |
| | Previous CABG | | | 3 (27) | 7 (63) | 0.198 |
| | Previous PCI | | | 5 (45) | 4 (36) | 1.000 |
| | Previous Stroke/TIA | | | 2 (18) | 2 (18) | 1.000 |
| Claudication | | | | 3 (27) | 2 (18) | 1.000 |
| Atrial fibrillation or flutter | | | | 12 (80) | 10 (67) | 0.682 |
| Previous valve disease, at least moderate | | | | 4 (27) | 5 (33) | 1.000 |
| Hypertension | | | | 11(73) | 8 (53) | 0.450 |
| Hyperlipidemia | | | | 10 (67) | 1 (73) | 1.000 |
| Pulmonary disease | | | | 4 (27) | 3 (20) | 1.000 |
| Previous or current malignancies | | | | 5 (33) | 2 (13) | 0.390 |
| Inflammatory disease (polymyalgia reumatica, rheumatoid arthritis) | | | 1 (3) | 2 (14) | 1 (7) | 0.598 |
| Smoking ever | | | | 10(67) | 11 (73) | 1.000 |
| Smoking current | | | | 2 (13) | 3 (20) | 1.000 |
| Diabetes Mellitus | | | | 7(47) | 6 (40) | 1.000 |

| **Treatment, n (%)** | | | | | | |
|---|---|---|---|---|---|---|
| ACEi/ARB | | | | 15 (100) | 15 (100) | 1.000 |
| | Percent dose | | | | | |
| | | <50% | | 2 (13) | 2 (13) | 1.000 |
| | | 50-99% | | 3 (20) | 3 (20) | |
| | | ≥100% | | 10 (67) | 10 (67) | |
| | | | | | | |
| Beta blockers | | | | 15 (100) | 15 (100) | 1.000 |
| | | <50% | | 1 (7) | 0 | 0.222 |
| | | 50-99% | | 5 (33) | 2 (13) | |
| | | ≥100% | | 9 (60) | 13 (87) | |
| MRA | | | | 11 (73) | 14 (93) | 0.330 |
| Loop diuretics | | | 1 (3) | 12 (86) | 14 (93) | 0.598 |
| Thiazide diuretics | | | | 0 | 0 | |
| Levosimendan ever | | | | 3 (20) | 3 (20) | 1.000 |
| Other inotropes ever | | | | 0(7) | 1 (7) | 1.000 |
| Non-dihydropyridine Calcium channel blocker | | | 1 (3.3) | 0 (0) | 1 (7) | 0.483 |
| Aspirin | | | 2 (6.7) | 3 (23) | 3 (20) | 1.000 |
| Other anti-platelets | | | | 1 (7) | 1 (7) | 1.000 |
| Warfarin | | | 2 (6.7) | 10 (77) | 11 (73) | 1.000 |
| Low molecular heparin | | | 1 (3.3) | 0 | 1 (7) | 1.000 |
| Digoxin | | | | 1 (7) | 3 (20) | 0.598 |
| Amidarone | | | | 2 (13) | 3 (20) | 1.000 |
| Statins | | | | 9 (60) | 10 (66) | 1.000 |
| Allopurinol | | | | 3 (20) | 3 (20) | 1.000 |
| Insulin | | | | 2 (13) | 6 (40) | 0.333 |
| Oral glucose lowering treatment | | | | 3 (20) | 6 (40) | 0.427 |
| CRT | | | | 8 (53) | 10 (67) | 0.710 |
| ICD | | | | 14 (93) | 11 (73) | 0.330 |
| | Secondary prevention | | | 5 (33) | 2 (13) | 0.390 |
| | Primary prevention | | | 9 (60) | 10 (66) | 1.000 |

| **Tests** | | | | | | |
|---|---|---|---|---|---|---|
| Previous VO₂ max test, n (%) | | | | 8 (57) | 9 (60) | 1.000 |
| Most recent VO₂ max, | | | | 13.4 (11.6, 16.4) | 12.2 (9.1, 14.1) | 0.5625 |
| Previous 6 minutes' walk test, n (%) | | | | 5 (33) | 3 (20) | 0.682 |

| **Variable** | | | **Missing n (%)** | **Ghrelin n=15 (50%)** | **Placebo n=15 (50%)** | **p value** |
|---|---|---|---|---|---|---|
| Most recent 6 minutes' walk test (m) | | | | 426 (421-450) | 330 (330-355) | 0.1771 |

| **Symptoms the week prior to inclusion, n (%)** | | | | | | |
|---|---|---|---|---|---|---|
| Paroxysmal nocturnal dyspnea | | | 0 | 2 (13) | 1 (7) | 1.000 |
| Orthopnea | | | 0 | 4 (27) | 4 (27) | 1.000 |
| Able to walk one block | | | 0 | 11 (73) | 9 (60) | 0.700 |
| Dyspnea at rest | | | 0 | 0(0) | 3 (20) | 0.224 |
| Unspecific chest pain, any cause | | | 0 | 1 (7) | 3 (20) | 0.598 |

| **ECG, n (%)** | | | | | | |
|---|---|---|---|---|---|---|
| Sinus rhythm | | | 0 | 5 (33) | 7 (47) | 0.710 |
| Atrial fibrillation/flutter | | | 0 | 5 (33) | 7 (47) | 0.710 |
| Atrial pace | | | 0 | 5 (33) | 1 (7) | 0.169 |
| Ventricular pace | | | 0 | 7 (47) | 10 (67) | 0.462 |

| | | | | | | |
|---|---|---|---|---|---|---|
| Abbreviations: BMI, body mass index; SBP, systolic blood pressure; DBP, diastolic blood pressure; SpO2, peripheral saturation by pulse oximetry, HF, heart failure; NYHA class, New York Heart Association class; INR, international normalized ratio; NT-proBNP, N-terminal pro brain natriuretic peptide; hsCRP, high sensitive C reactive protein; MI, myocardial infarction; AP, angina pectoris; CABG, coronary artery bypass grafting; PCI, percutaneous coronary intervention; TIA, transient ischemic attack, CRT, cardiac resynchronization therapy; ICD, implantable cardioverter defibrillator; MRA, mineralocorticoid receptor antagonist; ACEi, angiotensin converting enzyme inhibitor; ARB, angiotensin receptor blocker. *D Webb, data on file from 41 healthy non-obese adults | | | | | | |

**Table 12. Biomarkers at baseline and 2-5 days follow up**

| | **Ghrelin** | | **Placebo** | | |
|---|---|---|---|---|---|
| **Variable** | **Baseline** | **2-5 days FU** | **Baseline** | **2-5 days FU** | **P value interaction** |
| **NT-proBNP [ng/L]** | 2080 [1080-3980] | 2450* [942-4920] | 2380 [787-4060] | 2020 [762-2870] | 0.01 |
| **Troponin T [ng/L]** | 21±10 | 31±38 | 22±13 | 24±15 | 0.36 |
| **Creatinine [ukat/L]** | 112±34 | 123±35* | 118±33 | 124±37 | 0.28 |
| **Cystatin C [mg/L]** | 1.45±0.44 | 1.59±0.52* | 1.45±0.42 | 1.62±0.50* | 0.57 |
| **hsCRP [mg/L]** | 2.7 [0.9-7.7] | 3.0 [1.3-8.0] | 2.4 [1.1-7.8] | 3.0 [1.0-5.6] | 0.63 |
| **Insulin (non-fasting) [mIE/L]** | 36 [20-55] | 30 [12-59] | 39 [27-49] | 28 [20-61] | 0.70 |
| **ASAT [ukat/L]** | 0.41 [0.33-0.47] | 0.42 [0.33-0.50] | 0.37 [0.27-0.45] | 0.40 [0.28-0.47] | 0.41 |
| **ALAT [ukat/L]** | 0.31 [0.24-0.38] | 0.31 [0.26-0.41] | 0.35 [0.25-0.48] | 0.35 [0.23-0.43] | 0.70 |
| **GT [ukat/L]** | 0.77 [0.65-1.40] | 0.83 [0.66-1.30] | 0.97 [0.47-1.50] | 1.00 [0.47-1.60] | 0.60 |
| **Triglycerides [mmol/L]** | 1.4 [0.9-1.48] | 1.6 [1.1-2.7]* | 1.6 [0.9-1.9] | 1.6 [1.3-2.8]* | 0.94 |
| **INR among warfarin treated** | 2.2 [2.0-2.8] | 2.6 [2.2-2.9] | 2.6 [2.4-2.8] | 2.5 [2.3-2.5] | 0.02 |
| **INR among non-warfarin treated** | 1.1 [1.0-2.3] | 1.0 [1.0-2.7] | 1.1 [1.0-1.2] | 1.0 [0.9-1.1] | 0.19 |
| **Potassium [mmol/L]** | 4.1±0.6 | 4.3±0.4 | 4.1±0.4 | 4.2±0.4 | 0.88 |
| **Sodium [mmol/L]** | 139±3 | 138±2 | 138±3 | 138±2 | 0.16 |
| **Haemoglobin [g/dL]** | 13.5±14 | 13.5±17 | 12.9±17 | 13.1±17 | 0.20 |
| **Haematocrit [%]** | 40±4 | 41±5 | 38±4 | 39±4 | 0.96 |
| **White blood cell count (109/L)** | 7.4±1.9 | 7.4±2.0 | 7.0±1.7 | 7.3±1.3 | 0.23 |

| | | | | | |
|---|---|---|---|---|---|
| *= p<0.05 vs. baseline within the same group (treatment or placebo) | | | | | |

### Patient Outcomes

### Plasma acyl ghrelin pharmacokinetics

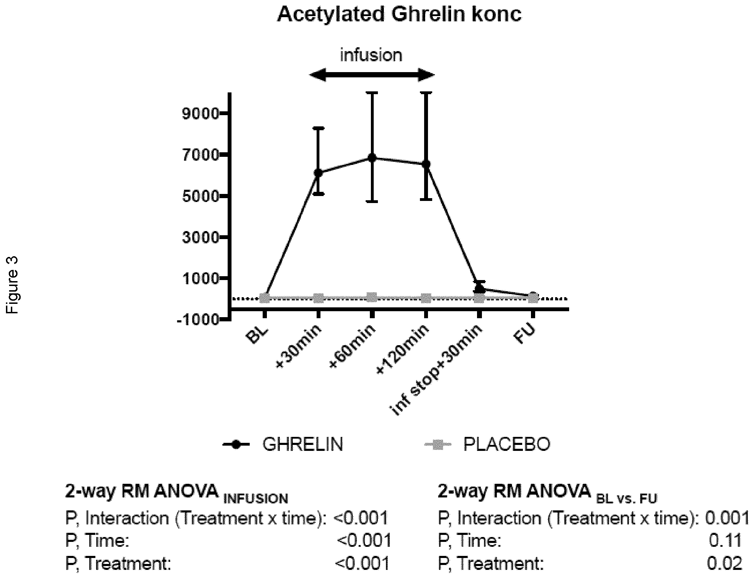

Plasma acyl ghrelin concentrations at baseline and during and after ghrelin infusion are shown in Table 6 and Figure 3. In the treatment group, median concentrations of acyl ghrelin quickly reached near the maximum of the assay. Acyl ghrelin was near normalized 30 minutes after infusion and normalized at 2-5 days follow-up.

**Table 6. Ghrelin pharmacokinetics. Measured plasma acyl ghrelin in placebo and treatment group (median, interquartile range). The lower limit of detection was as 3.2 ng/L. P for trend 30 min post infusion to 2-5 days post infusion, within ghrelin group p<0.001 and within placebo group p=0.0254.**

| **Time** | **Ghrelin, acyl ghrelin (ng/L)** | **Placebo, acyl ghrelin (ng/L)** | **P value** |
|---|---|---|---|
| **Fasting** | 70(32-144) | 82(30-97) | 0.724 |
| **Prior to infusion** | 76(42-131) | 60(40-78) | 0.361 |
| **Infusion 30 min** | 6114(5102-8267) | 62(35-90) | <0.001 |
| **Infusion 60 min** | 6841(4755-11 392) | 64(32-85) | <0.001 |
| **Infusion 120 min** | 6536(4835-10 078) | 59(37-73) | <0.001 |
| **30 min post infusion** | 503(372-833) | 58(37-89) | <0.001 |
| **2-5 days post infusion** | 139 (117-161) | 55(31-69) | 0.001 |
| **P trend 0 to 120 min within groups** | <0.0001 | 0.7893 | |
| **P trend 0 to 120 min between groups** | <0.0001 | | |

### Primary efficacy outcome: cardiac output

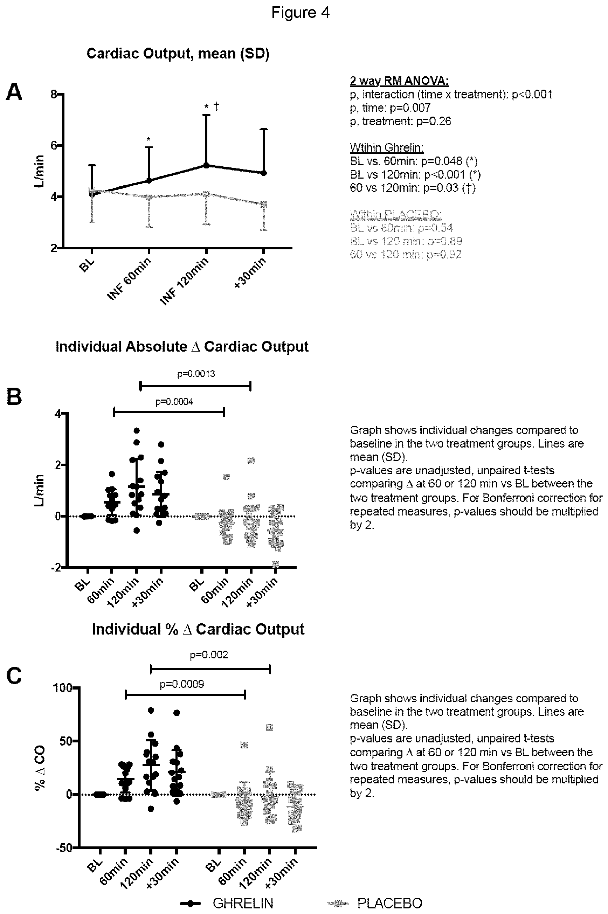

Tables 7 and 8 and Figure 4 show CO at baseline and responses to ghrelin and placebo. In the ghrelin group, CO increased with infusion and fell after stop of infusion, all pair-wise comparisons significant. In the placebo group, there was no significant change in CO, p interaction <0.0001. The absolute and percent changes in CO were different in the ghrelin vs. placebo groups. At 2-5 days follow-up there was still a significant increase in CO in the ghrelin compared to placebo group (Table 8).

**Table 7. Acute hemodynamic effects of ghrelin vs. placebo infusion**

| | **Ghrelin** | | | | **Placebo** | | | | |
|---|---|---|---|---|---|---|---|---|---|
| **Variable** | **Baseline** | **Infusion +60 min** | **Infusion + 120 min** | **30 min after stop infusion** | **Baseline** | **Infusion +60 min** | **Infusion + 120 min** | **30 min after stop infusion** | **P value interacti on** |
| **CO [L/min]** | 4.08±1.15 | 4.64±1.31 * | 5.23±1.98* | 4.94±1.70 | 4.26±1.23 | 3.99±1.16 | 4.11±1.99 | 3.70±0.99 | <0.001 |
| **SV [ml]** | 59.10±17.82 | 69.64±22.75* | 79.83±30.30* | 74.31±26.09 | 62.0±15.7 | 58.9±17.7 | 60.7±15.1 | 55.1±15.9 | <0.001 |
| **HR [bpm]** | 70.7±11.3 | 68.5±11.2 | 67.1±11.1 | 68.2±11.4 | 68.7±7.6 | 68.6±8.1 | 67.6±9.5 | 68.1±8.6 | 0.15 |
| **VO₂ [L/min]** | 0.20±0.07 | 0.19±0.07 | 0.19±0.08 | 0.19±0.08 | 0.20±0.08 | 0.20±0.09 | 0.19±0.08 | 0.18±0.08 | 0.88 |
| **A-V O₂ diff [%]** | 27.6±14.3 | 23.6±12.9* | 20.9±8.5* | 21.6±12.1 | 26.4±9.0 | 28.5±11.3 | 26.6±10.7 | 29.0±15.1 | 0.0049 |
| **SpO₂ [%]** | 95.2±3.4 | 93.5±3.5* | 93.4±3.6* | 94.7±3.9 | 95.7±1.9 | 95.2±2.0 | 95.7±1.8 | 95.9±2.3 | 0.0054 |
| **Shunt [%]** | 9.9±10.6 | 16.8±11.8 * | 18.0±12.3* | 12.6±14.3 | 9.5±6.7 | 11.7±10.1 | 10.5±8.6 | 10.3±10.7 | 0.0083 |
| **SvO₂ [%]** | 67.9±14.4 | 70.0±13.8 | 71.7±10.1 | 73.3±12.8 | 69.3±8.6 | 66.8±10.5 | 69.1±9.9 | 67.1±14.6 | 0.094 |
| **PBF [L/min]** | 3.61±0.70 | 3.76±0.76 | 4.08±0.90* | 4.14±0.97 | 3.82±1.06 | 3.52±1.08‡ | 3.66±1.12 | 3.30±1.02 | 0.008 |
| **dBP [mmHg]** | 58.6±8.4 | 56.5±8.7 | 56.1±10.3 | 58.9±7.5 | 63.5±10.1 | 66.5±8.0 | 63.4±8.2 | 68.4±10.1 | 0.11 |
| **sBP [mmHg]** | 104.7±18.4 | 99.0±17.6 | 99.0±21.1 | 105.7±20.5 | 120.2±24.4 | 118.2±21.3 | 115.5±22.3 | 123.6±25.2* | 0.83 |
| **mBP [mmHg]** | 74.9±11.3 | 71.4±10.2 | 70.9±13.1 | 75.5±12.0 | 82.2±14.1 | 84.1±11.9 | 81.1±12.1 | 87.0±14.5* | 0.23 |
| **dP/dt [mmHg/s]** | 742±267 | 535±213 | 531±191 | 602±252 | 875±305 | 675±256 | 699±265 | 776±314 | 0.81 |
| **eSVR [dyn*s/cm-1]]** | 1567±468 | 1311±365* | 1200±387* | 1354±480 | 1656±512 | 1820±574 | 1693±484 | 2013±658 | 0.0004 |

| | | | | | | | | | |
|---|---|---|---|---|---|---|---|---|---|
| *= p<0.05 vs. baseline within the same group (treatment or placebo) †=p<0.05 vs. infusion 60min within the same group (treatment or placebo) ‡=0.05<p>0.10 vs. baseline within the same group (treatment or placebo) Measured data: VO2, SpO2, HR, PBF, dBP, sBP Calculated data: CO, SV, A-V O2 diff, Shunt, SvO2, mBP, eSVR | | | | | | | | | |

**Table 8. Hemodynamics at baseline and 2-5 day follow-up visit**

| | **Ghrelin** | | **Placebo** | | |
|---|---|---|---|---|---|
| **Variable** | **Baseline** | **Follow-up** | **Baseline** | **Follow-up** | **P value interaction** |
| **CO [L/min** ] | 4.08±1.15 | 4.21±1.27 | 4.26±1.23 | 3.98±1.05^{†} | 0.017 |
| **SV [ml ]** | 59.9±19.0 | 59.9±18.5 | 61.9±15.2 | 57.4±14.3^{†} | 0.16 |
| **HR [bpm** ] | 69.6±9.1 | 71.6±10.8 | 68.8±7.6 | 69.8±9.8 | 0.53 |
| **VO₂ [L/min ]** | 0.2±0.07 | 0.22±0.06 | 0.20±0.09 | 0.23±0.09 | 0.97 |
| **SpO₂ [% ]** | 95.2±3.4 | 94.0±3.9^{†} | 95.4±1.7 | 95.6±1.3 | 0.028 |
| **SvO₂ [%]** | 67.7±14.3 | 66.4±18.4^{†} | 69.9±8.5 | 64.9±8.6^{†} | 0.82 |
| **A-V O₂-diff [%** ] | 24.4±7.6 | 27.4±10.0‡ | 25.6±8.7 | 30.7±9.0^{†} | 0.41 |
| **Shunt [% ]** | 9.9±10.6 | 15.7±16.7 | 10.2±6.4 | 8.6±5.1 | 0.09 |
| **PBF [L/min ]** | 3.61±0.70 | 3.58±0.74 | 3.88±1.08 | 3.64±0.87‡ | 0.30 |

| | | | | | |
|---|---|---|---|---|---|
| †= p<0.05 vs. baseline in the same group (GHSRA or placebo) ‡ = p=0.05-0.10 vs. baseline in the same group (GHSRA or placebo) The ghrelin molecule is "GHSRA". | | | | | |

### Secondary efficacy and safety outcomes: hemodynamics

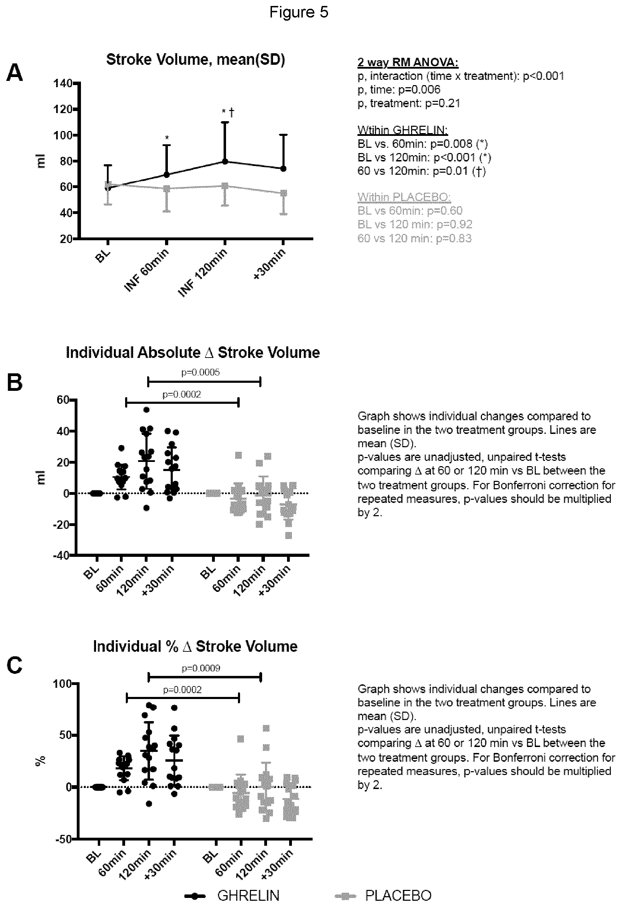

Tables 7 and 8 and Figure 5 show calculated stroke volume (SV) prior to, during and after ghrelin/placebo infusion. In the ghrelin group, SV increased with infusion and fell after stop of infusion, all pair-wise comparisons significant. In the placebo group, there was no significant change in SV. The absolute and percent changes in SV were different in the ghrelin vs. placebo groups.

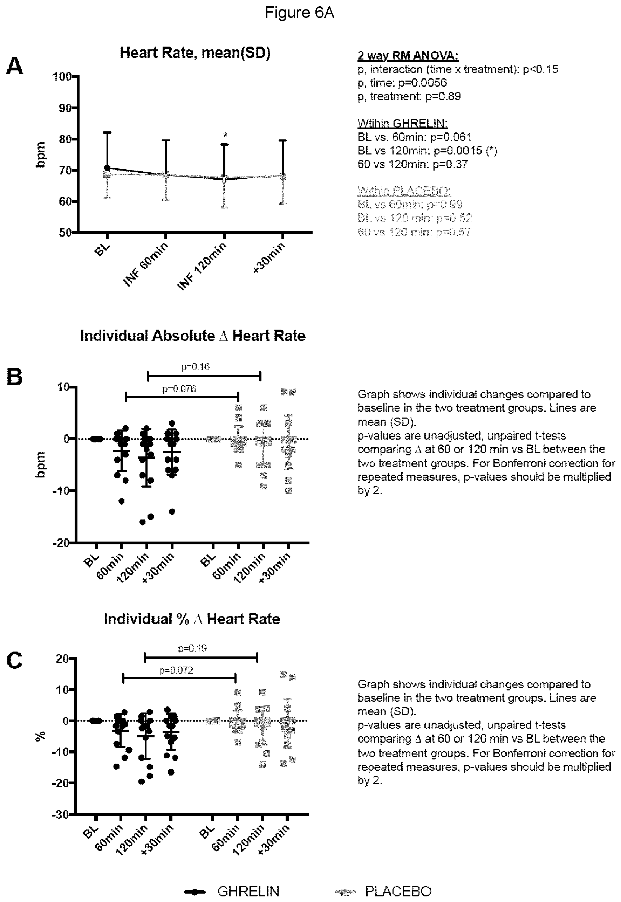

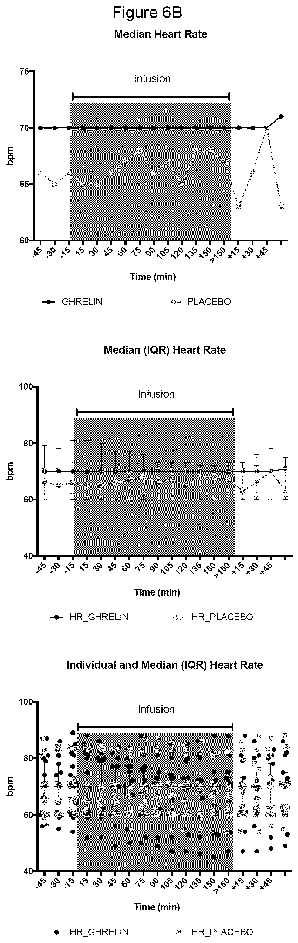

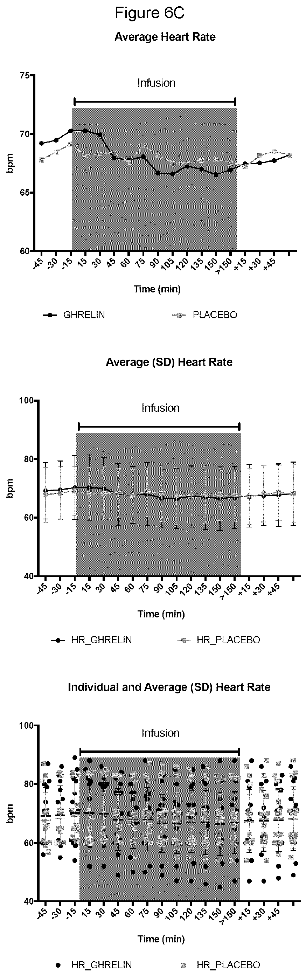

Tables 7 and 8 and Figure 6 show heart rate (HR) at select time points measured manually and continuously recorded by Nexfin. In the ghrelin group, HR fell slightly between baseline and 120 minutes. In the placebo group, there was no significant change in HR. There were no statistically significant differences in absolute or percent changes in HR.

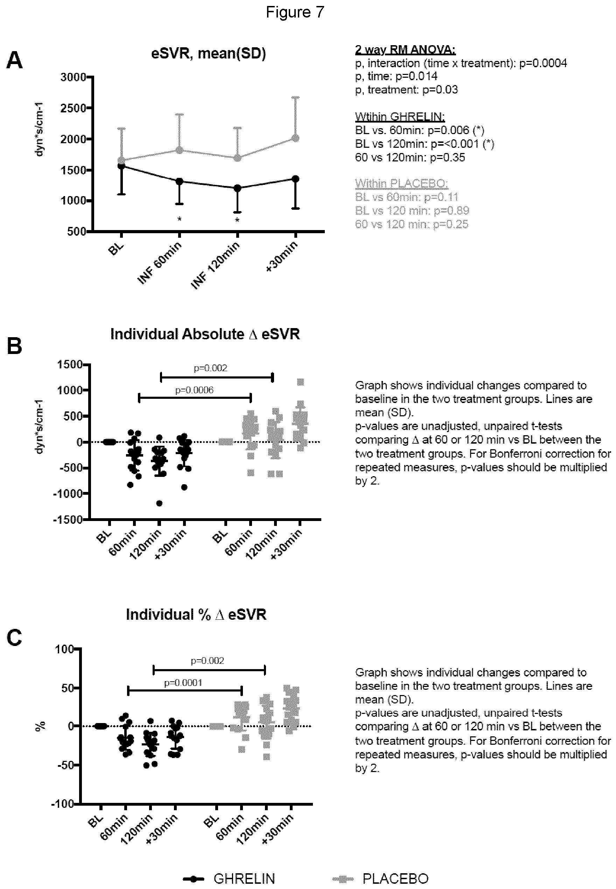

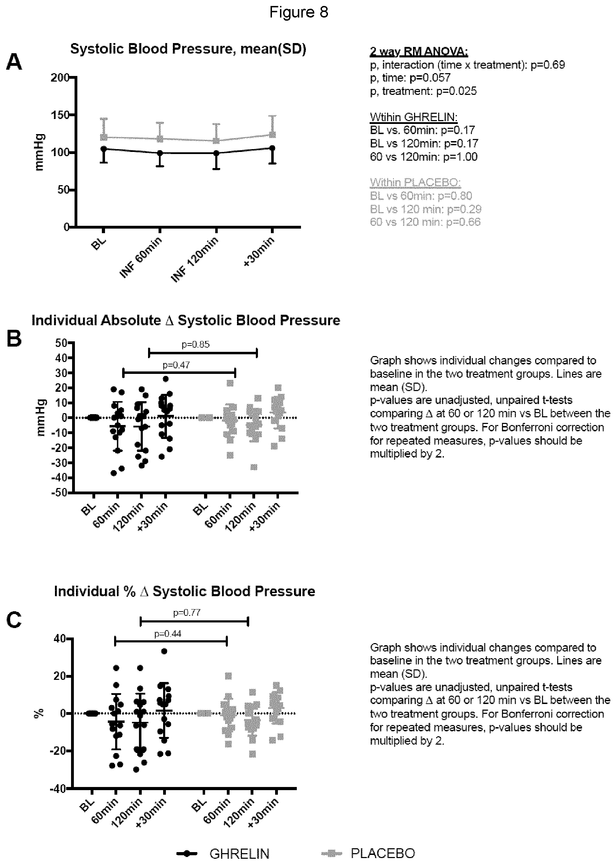

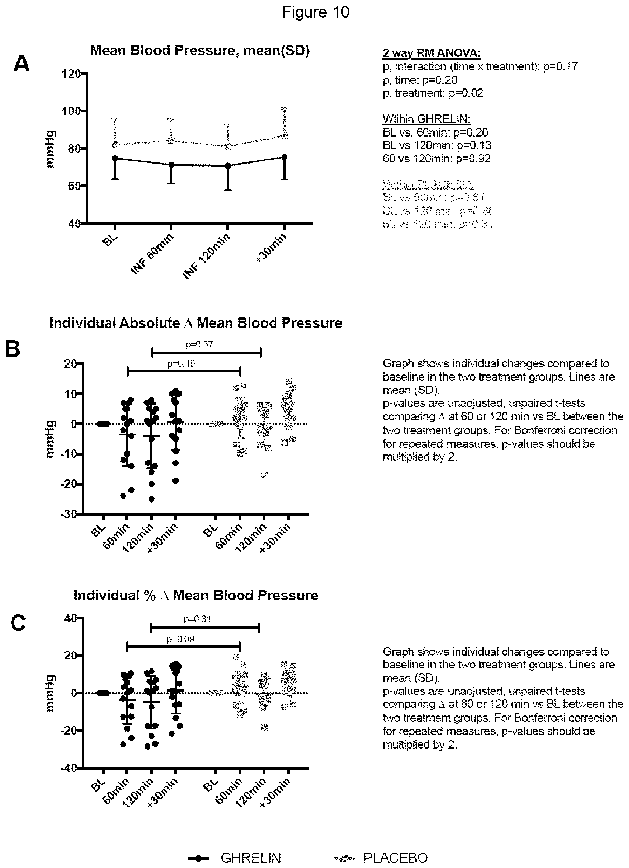

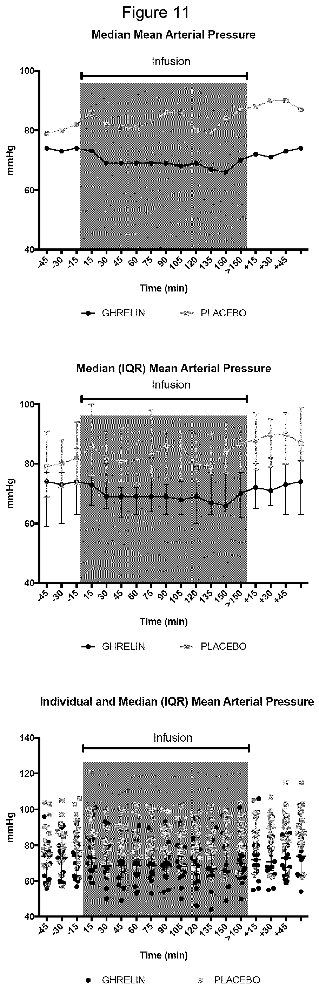

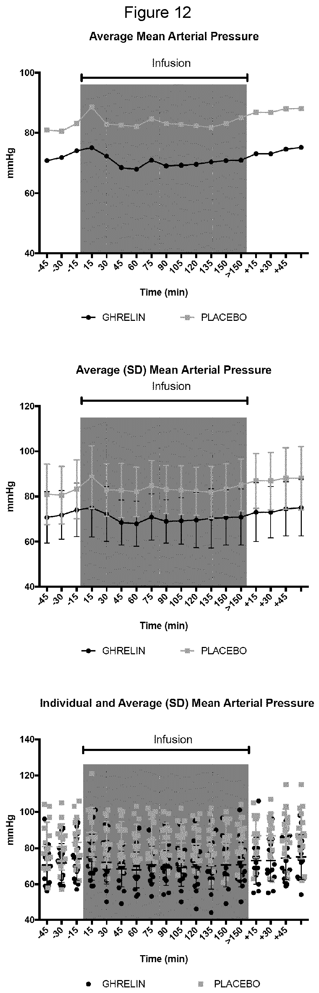

Tables 7 and 8 and Figure 7 shows estimated systemic vascular resistance (SVR) and Figures 8-12 show systolic, diastolic and mean arterial blood pressure. Although there was a decrease in calculated SVR commensurate with the increase in CO with ghrelin, there were no changes in blood pressure and no incidence of hypotension.

Tables 7 and 8 show additional hemodynamic data. There was no change in oxygen consumption (VO₂), suggesting that neither ghrelin nor placebo affected metabolism (oxygen demand). Pulmonary blood flow increased in the ghrelin groups, consistent with the increase in CO.

### Secondary outcomes: echocardiography

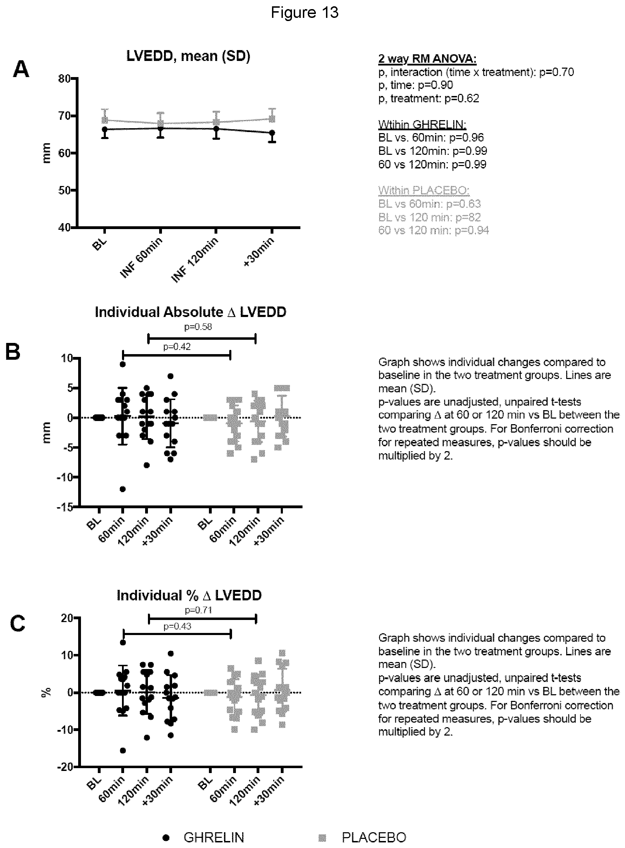

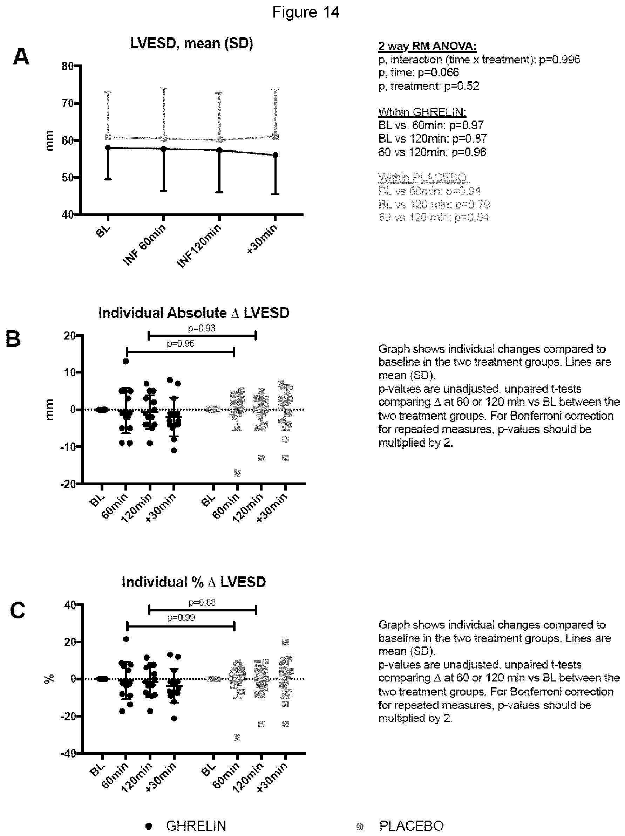

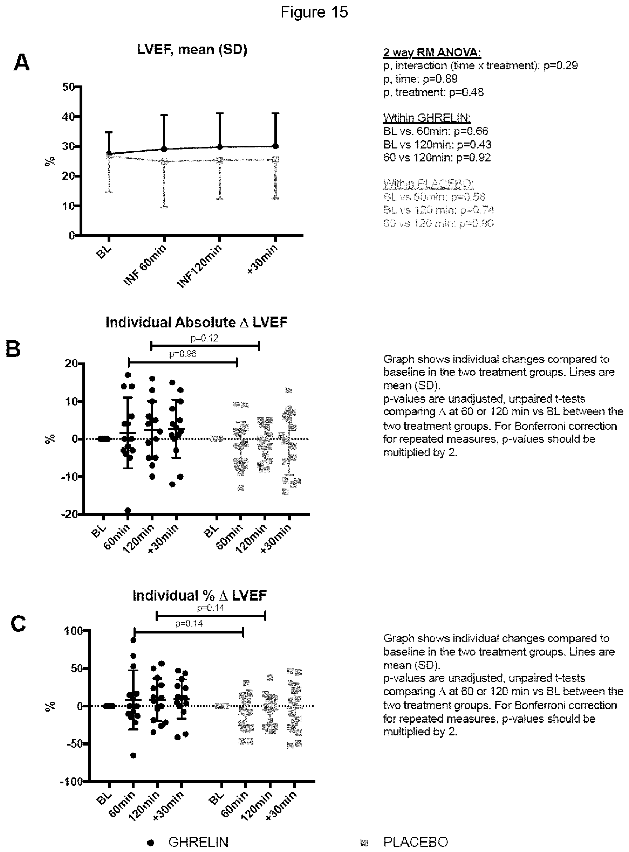

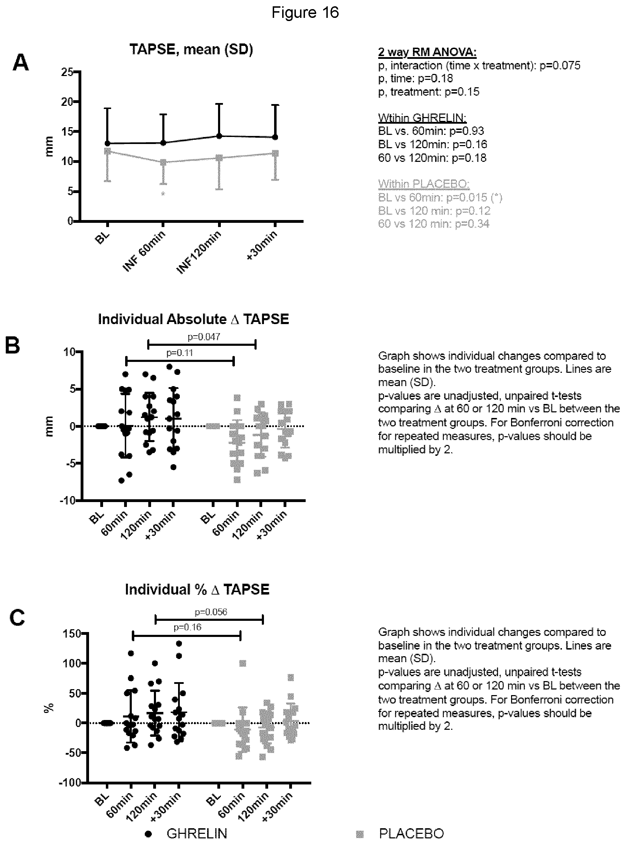

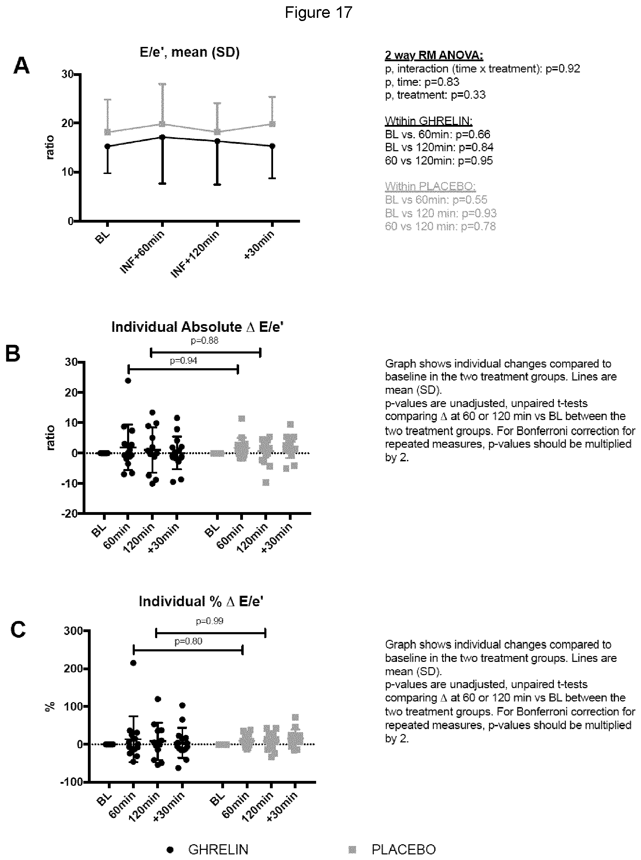

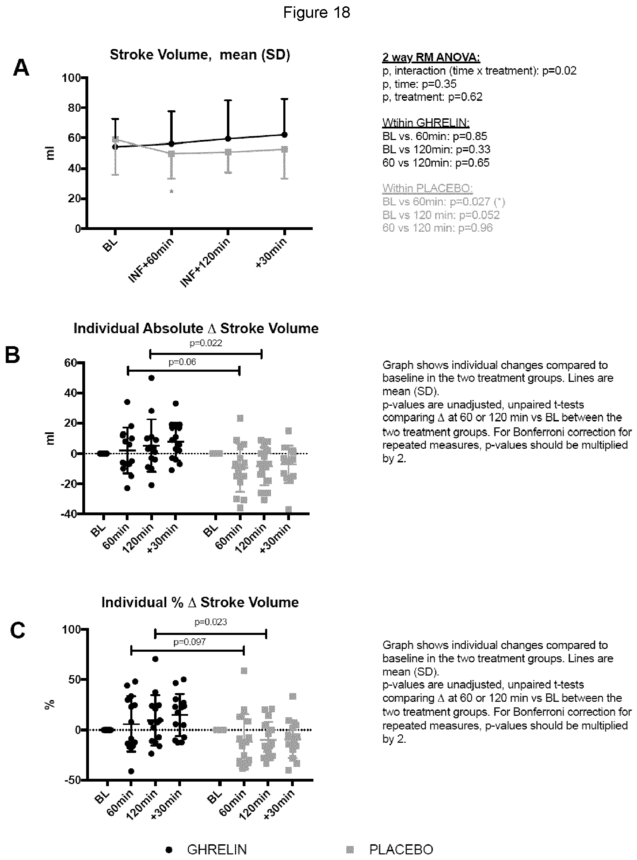

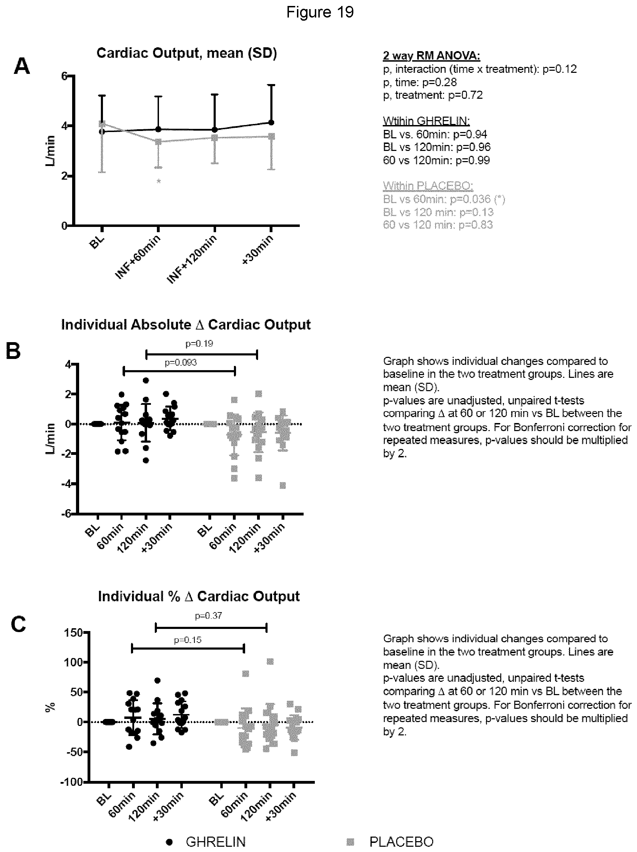

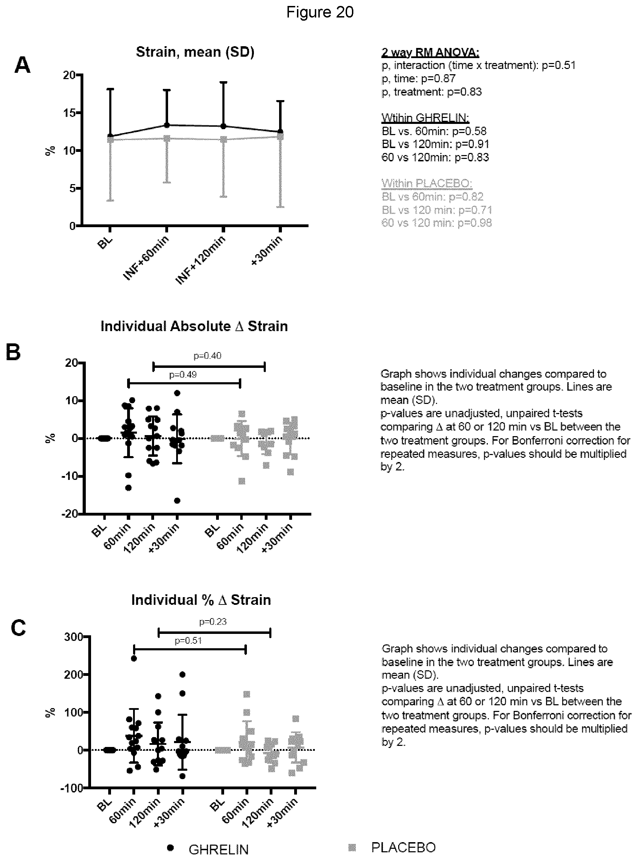

Tables 9 and 10 and Figures 13-20 show echocardiography parameters prior to, during, and after infusion. There was no change or differences in change in left ventricular end-diastolic diameter (LVEDD) (Figure 13) or left ventricular end systolic diameter (LVESD) (Figure 14). For EF, representing left ventricular function, there was a nominal interaction suggesting improvement in EF with ghrelin, and a trend toward a difference in change in EF at 60 and 120 minutes (Figure 15). For tricuspid annular plane systolic excursion (TAPSE), representing right ventricular function, there was a trend toward an interaction suggesting improvement in TAPSE, trends toward differences in changes at 60 and 120 minutes, and a significant difference in absolute change at 120 minutes (Figure 16). There was no interaction with or differences in change in E/e', a surrogate for LV filling pressures (Figure 17), suggesting the increased CO was not accompanied by adverse increases in filling pressure. For stroke volume (SV) measured by echocardiography, there was a significant interaction suggesting improvement with ghrelin and consistent although of less magnitude than the SV measured be noni-invasive hemodynamics. There was also difference in change in SV although this was significant only at 120 minutes but not 60 minutes (Figure 18). For cardiac output (CO) measured by echocardiography, there was a nominal interaction suggesting improvement with ghrelin but no significant differences in change (Figure 19). The same was true for systolic strain, a surrogate for cardiac contractility (Figure 20).

### Biomarkers

Table 11 shows biomarkers before, during infusion, and 30 minutes after infusion. There was no interaction with treatment for any biomarker. There was no interaction with or change in troponin T. NT-proBNP increased in both groups, which is likely due to prolonged supine state and not taking diuretics during the infusion. Cystatin C increased in both groups. Table 12 shows biomarkers at 2-5 days after infusion. NT-proBNP remained elevated in the ghrelin group. This may be due to a stimulating effect of ghrelin leading to increased activity after infusion or different times since infusion. With multiple testing, it may also be due to chance.

### Safety outcomes during and immediately after infusion and 2-5 days after infusion

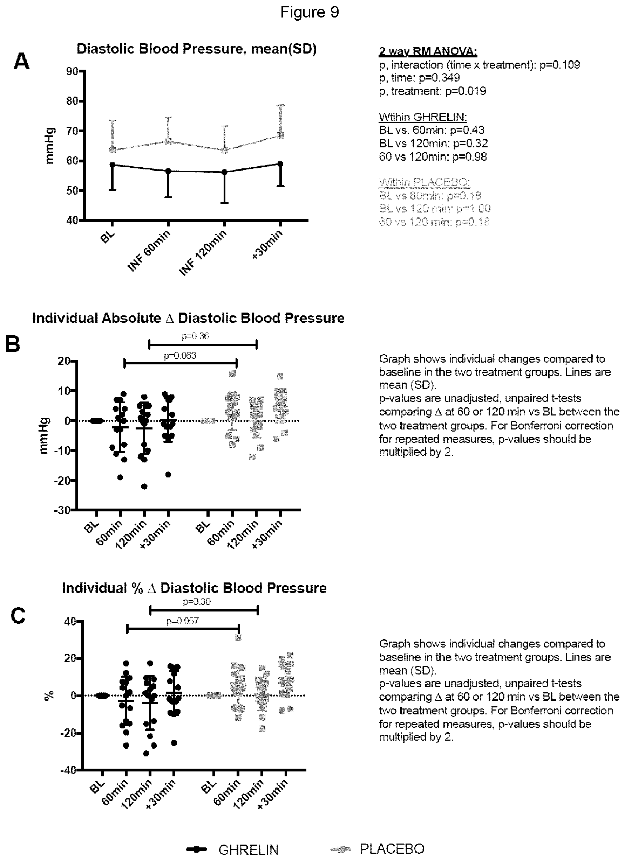

There was no effect of ghrelin or placebo on heart rate (Figure 6) or the incidence of ventricular tachy or brady arrhythmias (not shown). There was no effect of ghrelin or placebo on systolic (Figure 7), diastolic (Figure 8) or mean arterial (Figure 9) blood pressure. There were no events of hypotension or symptomatic hypotension in either group (not shown), except, one patient (placebo) experienced repeated urinary urgency and dizziness early after start of placebo infusion and infusion was interrupted and patient excluded. There was no effect on troponin T (p interaction infusion, 0.67 [Table 11] and p interaction 2-5 days follow-up, 0.36 [Table 12]). There were no symptoms or EKG changes consistent with ischemia. One patient in the ghrelin group had a >50% increase in troponin T (baseline; 31, during infusion 34, 38, 42, 43 and at FU 164 ng/L but no symptoms of chest pain or ECG changes).

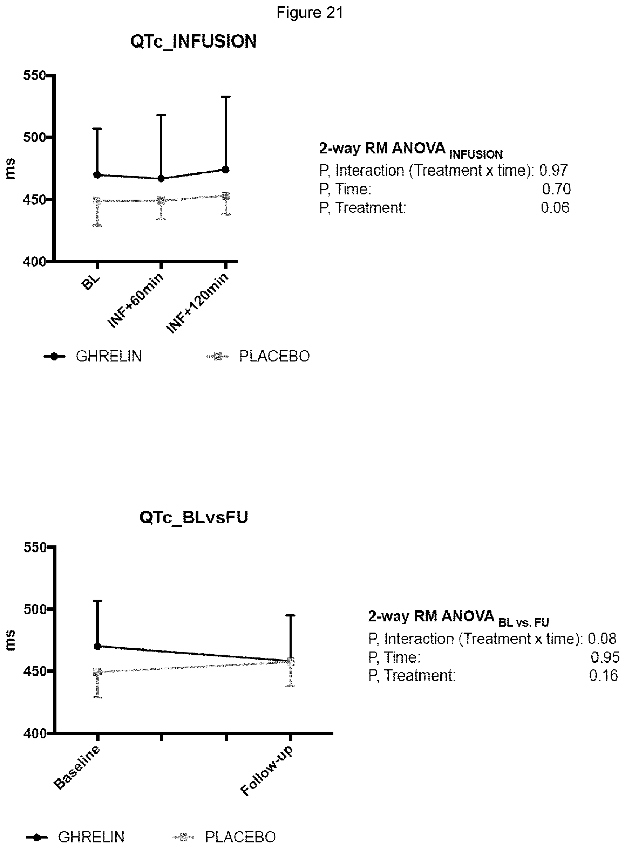

Among symptoms (headache, dizziness, dyspnea, central chest pain, flush, sleepiness, upset stomach and other symptoms) assessed as yes or no, flush at 60 min was more frequent in the intervention group compared to placebo (7 vs. 0; p=0.006) There was a nominal but not significant increase in QTc (Table 13 and Figure 21).

**Table 11. Acute effects of ghrelin vs. placebo on biomarkers**

| | **Ghrelin** | | | | | **Placebo** | | | | | |
|---|---|---|---|---|---|---|---|---|---|---|---|
| **Variable** | **Baselin e** | **Infusion + 30'** | **Infusio n + 60'** | **Infusion + 120'** | **30' after infusion** | **Baseline** | **Infusi on + 30'** | **Infusio n + 60'** | **Infusion + 120'** | **30' after infusio** | **p-interacti on** |
| **NT-proBNP [ng/L]** | 2080 [1080-3980] | 2220 [1080-4030] | 2340* [1100-4580] | 2630* [1220-4840] | 2820 [1280-5170] | 2380 [787-4060] | 2410 [754-4000] | 2490* [918-4770] | 2750* [860-4900] | 2820 [968-4690] | 0.63 |
| **Troponin T [ng/L]** | 21±9 | 21±9 | 22±10 | 21±10 | 21±10 | 22±13 | 21±1 3 | 22±13 | 22±13 | 23±13 | 0.67 |
| **Creatinine [ukat/L]** | 112±34 | 110±31 | 112±30 | 114±29 | 113±27 | 118±33 | 114± 32* | 117±32 | 118±32 | 119±32 | 0.58 |
| **Cystatin C [mg/L]** | 1.45±0. 44 | 1.46±0.4 4 | 1.55±0. 47* | 1.57±0.4 3* | 1.54±0.4 1 | 1.45±0.4 2 | 1.42± 0.40 | 1.49±0. 37 | 1.51±0.3 8* | 1.57±0. 40 | 0.48 |
| **hsCRP [mg/L]** | 2.7 [0.9-7.7] | 3.0 [1.1-7.6] | 3.0 [1.1-7.6] | 3.3 [1.1-7.5] | 3.5 [1.1-7.8] | 2.4 [1.1-7.8] | 2.3 [1.0-7.6] | 2.4 [0.9-7.5] | 2.3 [1.1-7.8] | 2.4 [1.0-8.1] | 0.75 |
| **ASAT [ukat/L]** | 0.41 [0.33-0.47] | 0.40 [0.34-0.43] | 0.44 [0.36-0.47] | 0.41 [0.33-0.46] | 0.43 [0.36-0.49] | 0.37 [0.27-0.45] | 0.33 [0.28-0.43] | 0.40 [0.30-0.44] | 0.37 [0.24-0.45] | 0.34 [0.30-0.50] | 0.97 |
| **ALAT [ukat/L]** | 0.31 [0.24-0.38] | 0.31 [0.23-0.39] | 0.31 [0.31-0.40] | 0.31 [0.21-0.39] | 0.32 [0.23-0.41] | 0.35 [0.25-0.48] | 0.34 [0.23-0.48] | 0.35 [0.23-0.49] | 0.34 [0.23-0.48] | 0.33 [0.22-0.47] | 0.58 |
| **GT [ukat/L]** | 0.77 [0.65-1.40] | 0.78 [0.65-1.40] | 0.79 [0.62-1.40] | 0.80 [0.62-1.30] | 0.82 [0.62-1.40] | 0.97 [0.47-1.50] | 0.93 [0.47-1.50] | 0.94 [0.46-1.50] | 0.95 [0.48-1.50] | 0.98 [0.48-1.50] | 0.51 |

| | | | | | | | | | | | |
|---|---|---|---|---|---|---|---|---|---|---|---|
| *= p<0.05 vs. baseline within the same group (treatment or placebo) | | | | | | | | | | | |

**Table 13. Effects of ghrelin and placebo on QTc interval**

| | | During infusion | | After infusion | |
|---|---|---|---|---|---|
| | | 60 minutes | 120 minutes | Prior to discharge | 2-5 days after |
| Median (interquartile range) changes in QTc (ms) from baseline | Placebo | -3 (-8 to +14) | -4 (-10 to +11) | +4 (-3 to +14) | +2 (-16 to +20) |
| | Ghrelin | +4 (-7 to +21) | +8 (-4 to +26) | +18 (-10 to +30) | -4 (-34 to +4) |
| | P placebo vs ghrelin | 0.418 | 0.191 | 0.295 | 0.135 |
| Maximal QTc prolongation (ms) from baseline | Placebo | +60 | +80 | +96 | +103 |
| | Ghrelin | +36 | +45 | +47 | +47 |

### Clinical Safety Outcomes over 90 days of follow-up

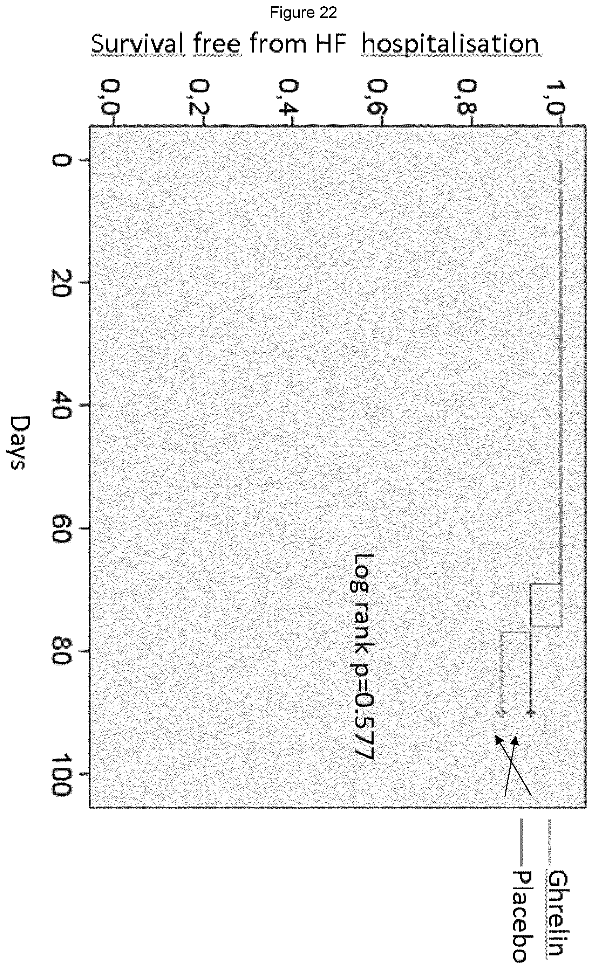

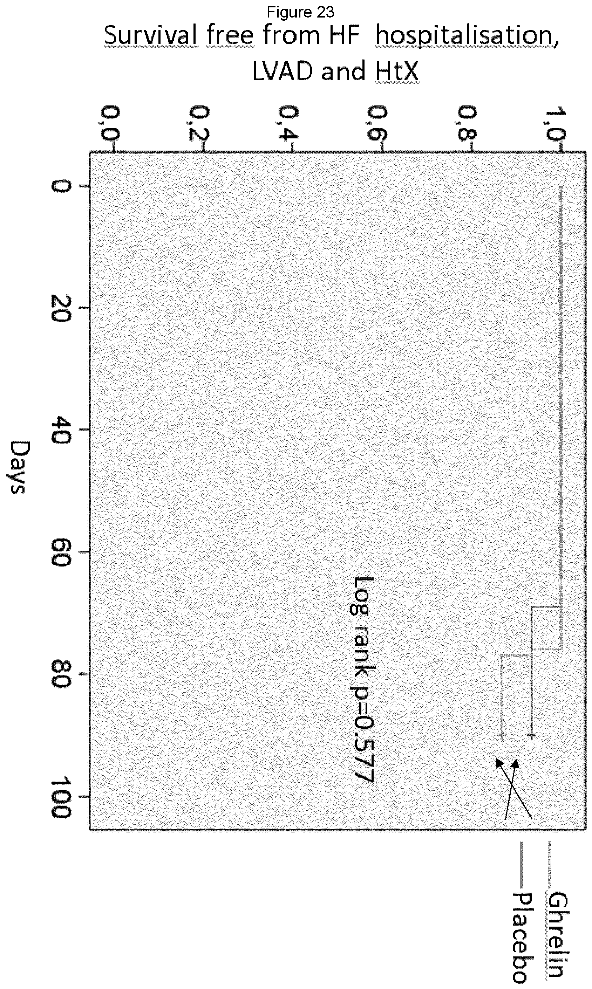

There we no differences in clinical events over 90 days follow-up (Figures 22 and 23).

### Results Ex Vivo Rodent Cardiomyocyte Studies

### Inotropic effect of Ghrelin on mouse healthy and heart failure cardiomyocytes

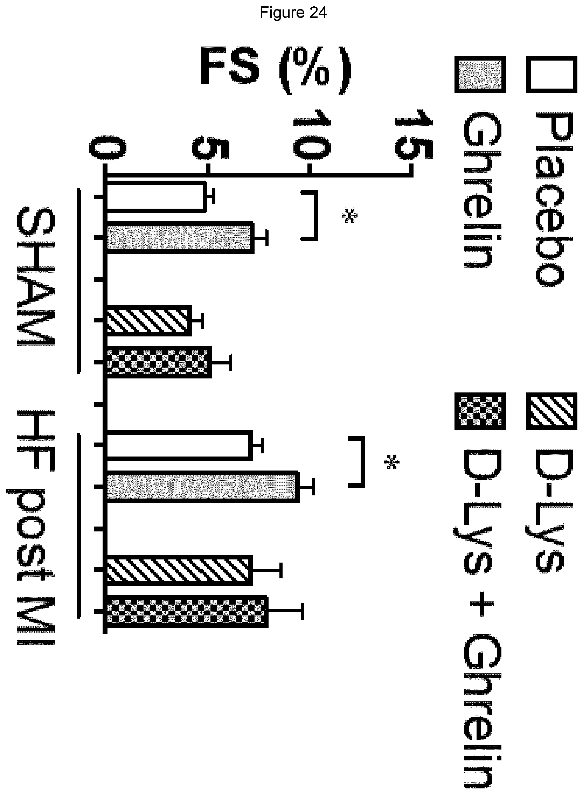

Following 15 min ghrelin exposure, cardiomyocyte fractional shortening (FS) increased compared to placebo. The ghrelin-induced increase in contraction was seen in cardiomyocytes from both sham and post-MI HF mice (Figure 24). The ghrelin effect on FS was blocked by pretreatment with GHS-receptor 1a antagonist (D-Lys 3; Sigma). This suggests that the inotropic effect of ghrelin in cardiomyocytes is mediated by GHS-R1a.

### Inotropic effect of ghrelin is through Ca²⁺ sensitization

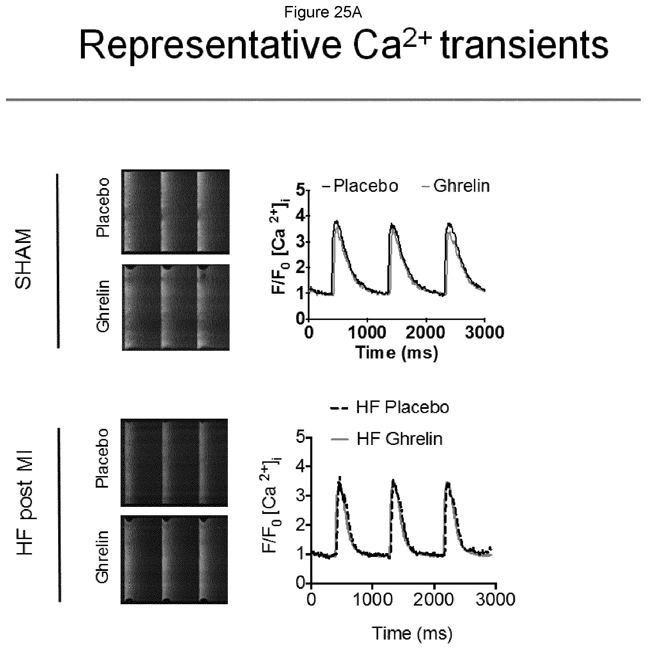

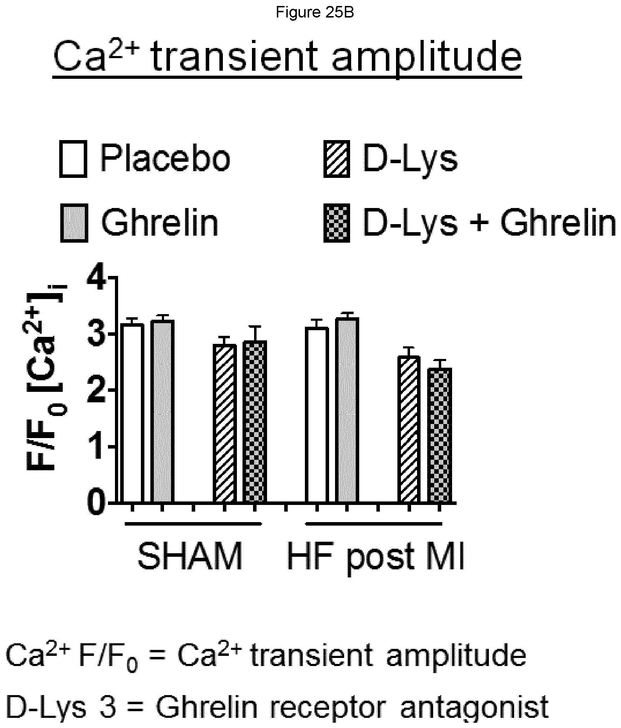

Cardiomyocyte Ca²⁺ transients did not differ with the different treatments (Placebo, Ghrelin, D-Lys3 or D-Lys 3 + Ghrelin) (Figure 25A and B). Moreover, ghrelin treatment did not affect the kinetics of Ca²⁺ transients since ROI (rate of increase of Ca²⁺ transients) and Tau (decay time constant) was similar between groups (Figure 25A). Taken together, these data suggests that the contraction potentiating effects of ghrelin is not mediated via increase in intracellular Ca²⁺, but may be mediated through increase in the myofilament Ca²⁺ sensitivity.

### Ca²⁺ sensitization by ghrelin is associated with reduced phosphorylation of troponin I

Post-translational modifications of myofibrillar proteins, e.g. troponin, can regulate the myofibril Ca²⁺ sensitivity. For instance, phosphorylation of serine 23-24 on troponin I has been linked to reduced myofibrillar Ca²⁺ sensitivity (Hasenfuss and Teerlink, 2011; Layland et al., 2005). We used antibodies targeting phosphorylated Serine 23-24 residues on troponin I to test if ghrelin changed the protein phosphorylation. Protein lysate from healthy cardiomyocytes treated with placebo, ghrelin, D-Lys 3 + ghrelin was used. In the presence of ghrelin the phosphorylation signal was lower compared to placebo (Figure 26). Pretreatment of cardiomyocytes with the ghrelin receptor antagonist D-Lys 3 prevented the reduction in troponin serine 23-24 phosphorylation (Figure 26). This indicates that the inotropic effect of ghrelin could involve post-translational modifications of troponin.

### Ghrelin reduces cardiomyocyte cAMP concentration and lowers troponin I phosphorylation

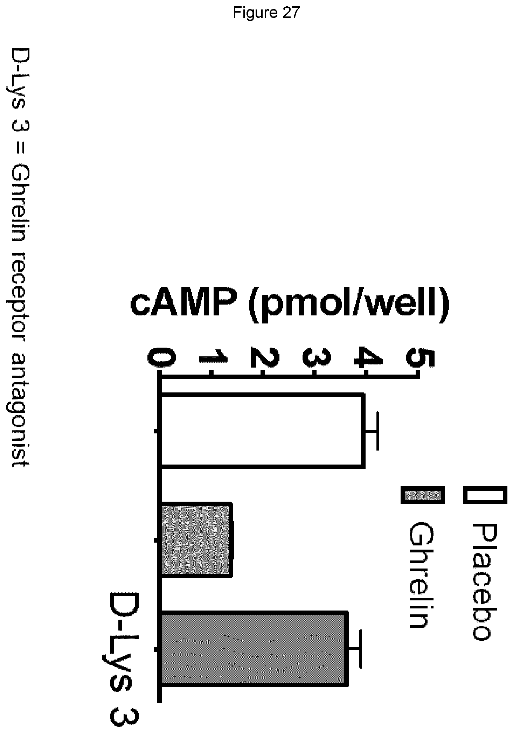

cAMP, is a key molecule for intracellular signal transduction between plasma membrane receptors (e.g. β-adrenergic receptor) and regulation of myocyte contractility. cAMP activates protein kinase A (PKA) that in turn can phosphorylate downstream proteins, including troponin. We found that in presence of ghrelin, the cAMP concentration in cardiomyocytes was lower compared to placebo (Figure 27 bars 1 and 2). Pretreatment of cardiomyocytes with GHS-R1a antagonist (D-Lys 3) prevented the reduction in cAMP concentration, which remained similar to placebo treatment (Figure 27 bar 3). These results are consistent with ghrelin-mediated reduction in troponin I serine 23-24 phosphorylation and suggest that a phosphokinase-dependent on cAMP could be responsible for serine 23-24 phosphorylation.

### Discussion

In this randomized placebo-controlled trial of intravenous acylated ghrelin vs. placebo in patients with advanced CHF and reduced EF, the primary outcome of change in CO was met. Ghrelin increased CO without adverse effects on hypotension, arrhythmia or ischemia. This was due to a significant increase in SV since HR was unchanged. The increased CO was associated with a calculated unaltered resting VO₂, decreased AVO₂-difference, increased SvO₂ and small but significant decrease in SpO₂, suggest improved O₂ delivery to metabolizing tissues through increased CO (despite a small reduction in arterial O₂ content measured as SpO₂). The ex vivo data suggested that the increased CO may be secondary to a novel mechanism involving inhibition of cAMP production, reduced troponin I phosphorylation, and calcium sensitization. This mechanism is different from that of conventional inotropes (which have harmful adverse effects) and may explain the absence of adverse effects with ghrelin.

### Physiology And Cardiovascular Actions Of Ghrelin

Concentrations of and the effects of ghrelin have been studied in normal human subjects (Akamizu et al., 2004; Arvat et al., 2001; Broglio et al., 2001; Broglio et al., 2003a; Broglio et al., 2003c; Broglio F., 2001; Enomoto et al., 2003; Falken et al., 2010; Levin et al., 2006; Nagaya et al., 2001a; Okumura et al., 2002; Peino et al., 2000; Vestergaard et al., 2007a; Vestergaard et al., 2007b; Vestergaard et al., 2008; Vestergaard et al., 2007c) and patients with cancer cachexia (Garcia et al., 2013; Garcia et al., 2005), chronic obstructive pulmonary disease cachexia (Miki et al., 2012), growth hormone deficiency (Janssen et al., 2004a; Janssen et al., 2004b), obesity (Tassone et al., 2003), the metabolic syndrome (Tesauro et al., 2005), and Cushing's syndrome (Leal-Cerro et al., 2002). Effects were variable and inconsistent. Small human studies suggested ghrelin may improve cardiac output (Nagaya et al., 2001b) and left ventricular EF (Nagaya et al., 2004) but these do not specify whether the ghrelin form used was acylated or not, and do not relate to the specific condition of AHF. It is also unclear which form of ghrelin is responsible for the observed actions in these studies, since acyl and des-acyl has different binding and effect in cardiomyocytes (Lear et al., 2010).

### Potential underlying mechanisms

As with many drugs in use or under development in chronic and acute heart failure (e.g. beta-blockers, angiotensin-receptor neprilysin inhibitors, levosimendan, serelaxin, ularatide, omecamtiv mecarbil, nitroxyl donors, istaroxime and others), the mechanisms of action and mechanism(s) responsible for the clinical benefit are unknown and probably multifactorial.

The growth hormone secreatagogue receptor (GHSR) is a 7-transmembrane G-protein coupled receptor with numerous downstream signaling pathways. It heterodimerizes with other G-protein coupled receptors, which may result in both Gs, Gi, and Gq effects. There are 4 (and possibly 5) different ghrelin receptors in the heart and vasculature with poorly understood function.

The previously described ghrelin signaling and potential mechanisms responsible for the observed inotropic (contractile) effect of ghrelin include the following:
1. GHSR-1a → couples to Gaq11 → phopsholipase C (PLC) → inositol triphosphate (IP3) and diacylglycerol (DAG) → IP3 receptor on endoplasmic (and potentially sarcoplasmic) reticulum → intracellular calcium release (Albarran-Zeckler and Smith, 2013)
2. DAG → Activates protein kinas C (PKC) → activate voltage gated L-type Ca channel → increased intracellular calcium (Sun et al., 2010b)
3. GHSR → via PLC and protein kinase S (or PKC?)→ inhibit potassium release → prolong action potential → activation L-type calcium channels (Sun et al., 2010a)

However, these mechanisms all result in increased intracellular calcium concentrations which we did not observe. It is the increase in intracellular calcium concentrations that leads to severe side effects in AHF patients. Instead, we observed increased contractility and fractional shortening secondary to increased calcium sensitivity.

### Our data support a novel mechanism as follows:

4. GHSR → Gi → reduces cAMP → reduced PKA activity → reduced phosphorylation of proteins (e.g. troponin I) → altered Ca²⁺ sensitivity.

If ghrelin works via increased calcium concentrations it would potentially be associated with adverse effects such as for beta adrenoreceptor agonists and phosphodiesterase inhibitors, although this may be offset by ghrelin also producing a central sympatholytic effect (Nagaya et al., 2001b). However, as stated, our data support a model of increased contractility through calcium sensitization. Thus, if ghrelin works as an inotrope, then we would expect side effects and adverse events. We did not observe any. This is novel and unexpected and explained by our ex vivo experiments.

Ghrelin also has many additional potentially cardioprotective effects, such as reduced inflammation (Li et al., 2004) and apoptosis (Baldanzi et al., 2002), improved endothelial function through improved nitric oxide bioavailability (Tesauro et al., 2005), and increased lean body mass (Nagaya et al., 2004). Furthermore, ghrelin has also been observed to have vasodilatory effects, and this is often a limitation of inotropic drugs, leading to hypotension. A major potential benefit of ghrelin observed in the present study was the absence of effect on blood pressure and lack of hypotension.

### Conclusion

In patients with advanced HF and reduced EF, 120 minutes of intravenous acylated ghrelin compared to placebo improved cardiac output without causing adverse hypotension, arrhythmias, tachycardia or ischemia. Our *ex vivo* murine cardiomyocyte studies suggested that the mechanism may be related to calcium sensitization. These experiments demonstrate that acylated ghrelin would be effective and safe to use for treating AHF.

### REFERENCES

Abraham, W.T., Adams, K.F., Fonarow, G.C., Costanzo, M.R., Berkowitz, R.L., LeJemtel, T.H., Cheng, M.L., Wynne, J., Committee, A.S.A., Investigators, et al. (2005). In-hospital mortality in patients with acute decompensated heart failure requiring intravenous vasoactive medications: an analysis from the Acute Decompensated Heart Failure National Registry (ADHERE). J Am Coll Cardiol 46, 57-64.
Akamizu, T., Takaya, K., Irako, T., Hosoda, H., Teramukai, S., Matsuyama, A., Tada, H., Miura, K., Shimizu, A., Fukushima, M., et al. (2004). Pharmacokinetics, safety, and endocrine and appetite effects of ghrelin administration in young healthy subjects. European journal of endocrinology / European Federation of Endocrine Societies 150, 447-455.
Albarran-Zeckler, R.G., and Smith, R.G. (2013). The ghrelin receptors (GHS-R1a and GHS-R1b). Endocrine development 25, 5-15.
Ambrosy, A.P., Fonarow, G.C., Butler, J., Chioncel, O., Greene, S.J., Vaduganathan, M., Nodari, S., Lam, C.S., Sato, N., Shah, A.N., et al. (2014). The global health and economic burden of hospitalizations for heart failure: lessons learned from hospitalized heart failure registries. J Am Coll Cardiol 63, 1123-1133.
Amsallem, E., Kasparian, C., Haddour, G., Boissel, J.P., and Nony, P. (2005). Phosphodiesterase III inhibitors for heart failure. Cochrane Database Syst Rev, CD002230.
Andersson, D.C., Fauconnier, J., Yamada, T., Lacampagne, A., Zhang, S.J., Katz, A., and Westerblad, H. (2011). Mitochondrial production of reactive oxygen species contributes to the beta-adrenergic stimulation of mouse cardiomycytes. J Physiol 589, 1791-1801.
Arvat, E., Maccario, M., Di Vito, L., Broglio, F., Benso, A., Gottero, C., Papotti, M., Muccioli, G., Dieguez, C., Casanueva, F.F., et al. (2001). Endocrine activities of ghrelin, a natural growth hormone secretagogue (GHS), in humans: comparison and interactions with hexarelin, a nonnatural peptidyl GHS, and GH-releasing hormone. J Clin Endocrinol Metab 86, 1169-1174.
Baker, D.W., Einstadter, D., Thomas, C., and Cebul, R.D. (2003). Mortality trends for 23,505 Medicare patients hospitalized with heart failure in Northeast Ohio, 1991 to 1997. American heart journal 146, 258-264.
Baldanzi, G., Filigheddu, N., Cutrupi, S., Catapano, F., Bonissoni, S., Fubini, A., Malan, D., Baj, G., Granata, R., Broglio, F., et al. (2002). Ghrelin and des-acyl ghrelin inhibit cell death in cardiomyocytes and endothelial cells through ERK1/2 and PI 3-kinase/AKT. J Cell Biol 159, 1029-1037.
Broglio, F., Arvat, E., Benso, A., Gottero, C., Muccioli, G., Papotti, M., van der Lely, A.J., Deghenghi, R., and Ghigo, E. (2001). Ghrelin, a natural GH secretagogue produced by the stomach, induces hyperglycemia and reduces insulin secretion in humans. J Clin Endocrinol Metab 86, 5083-5086.
Broglio, F., Benso, A., Gottero, C., Prodam, F., Gauna, C., Filtri, L., Arvat, E., van der Lely, A.J., Deghenghi, R., and Ghigo, E. (2003a). Non-acylated ghrelin does not possess the pituitaric and pancreatic endocrine activity of acylated ghrelin in humans. Journal of endocrinological investigation 26, 192-196.
Broglio, F., Gottero, C., Arvat, E., and Ghigo, E. (2003b). Endocrine and non-endocrine actions of ghrelin. Hormone research 59, 109-117.
Broglio, F., Gottero, C., Benso, A., Prodam, F., Destefanis, S., Gauna, C., Maccario, M., Deghenghi, R., van der Lely, A.J., and Ghigo, E. (2003c). Effects of ghrelin on the insulin and glycemic responses to glucose, arginine, or free fatty acids load in humans. J Clin Endocrinol Metab 88, 4268-4272.
Broglio F., A.E., Benso A., Gottero C., Muccioloi G., Papottu M., van der Lely A. J., Deghendhi R., Ghigo E. (2001). Ghrelin, a natural GH secretagogue produced by the stomach, induces hyperglycemia and reduces insulin secretion in humans. J Clin Endocrinol Metab 86, 5083-5086.
Cohn, J.N., Goldstein, S.O., Greenberg, B.H., Lorell, B.H., Bourge, R.C., Jaski, B.E., Gottlieb, S.O., McGrew, F., 3rd, DeMets, D.L., and White, B.G. (1998). A dose-dependent increase in mortality with vesnarinone among patients with severe heart failure. Vesnarinone Trial Investigators. N Engl J Med 339, 1810-1816.
Cuffe, M.S., Califf, R.M., Adams, K.F., Jr., Benza, R., Bourge, R., Colucci, W.S., Massie, B.M., O'Connor, C.M., Pina, I., Quigg, R., et al. (2002). Short-term intravenous milrinone for acute exacerbation of chronic heart failure: a randomized controlled trial. Jama 287, 1541-1547.
Cummings, D.E., Weigle, D.S., Frayo, R.S., Breen, P.A., Ma, M.K., Dellinger, E.P., and Purnell, J.Q. (2002). Plasma ghrelin levels after diet-induced weight loss or gastric bypass surgery. N Engl J Med 346, 1623-1630.
Curtis, L.H., Greiner, M.A., Hammill, B.G., Kramer, J.M., Whellan, D.J., Schulman, K.A., and Hernandez, A.F. (2008). Early and long-term outcomes of heart failure in elderly persons, 2001-2005. Archives of internal medicine 168, 2481-2488.
Dies, F., Krell, M.J., and Whitlow, P. (1986). Intermittent dobutamine in ambulatory outpatients with chronic cardiac failure. Circulation 74, (Suppl II):38.
Enomoto, M., Nagaya, N., Uematsu, M., Okumura, H., Nakagawa, E., Ono, F., Hosoda, H., Oya, H., Kojima, M., Kanmatsuse, K., et al. (2003). Cardiovascular and hormonal effects of subcutaneous administration of ghrelin, a novel growth hormone-releasing peptide, in healthy humans. Clinical science 105, 431-435.
Falken, Y., Hellstrom, P.M., Sanger, G.J., Dewit, O., Dukes, G., Gryback, P., Holst, J.J., and Naslund, E. (2010). Actions of prolonged ghrelin infusion on gastrointestinal transit and glucose homeostasis in humans. Neurogastroenterology and motility : the official journal of the European Gastrointestinal Motility Society 22, e192-200.
Gabrielsen, A., Videbaek, R., Schou, M., Damgaard, M., Kastrup, J., and Norsk, P. (2002). Non-invasive measurement of cardiac output in heart failure patients using a new foreign gas rebreathing technique. Clinical science 102, 247-252.
Garcia, J.M., Friend, J., and Allen, S. (2013). Therapeutic potential of anamorelin, a novel, oral ghrelin mimetic, in patients with cancer-related cachexia: a multicenter, randomized, double-blind, crossover, pilot study. Supportive care in cancer : official journal of the Multinational Association of Supportive Care in Cancer 21, 129-137.
Garcia, J.M., Garcia-Touza, M., Hijazi, R.A., Taffet, G., Epner, D., Mann, D., Smith, R.G., Cunningham, G.R., and Marcelli, M. (2005). Active ghrelin levels and active to total ghrelin ratio in cancer-induced cachexia. J Clin Endocrinol Metab 90, 2920-2926.
Gheorghiade, M., Filippatos, G., De Luca, L., and Burnett, J. (2006). Congestion in acute heart failure syndromes: an essential target of evaluation and treatment. Am J Med 119, S3-S10.
Go, A.S., Mozaffarian, D., Roger, V.L., Benjamin, E.J., Berry, J.D., Blaha, M.J., Dai, S., Ford, E.S., Fox, C.S., Franco, S., et al. (2014). Heart disease and stroke statistics--2014 update: a report from the American Heart Association. Circulation 129, e28-e292.
Hasenfuss, G., and Teerlink, J.R. (2011). Cardiac inotropes: current agents and future directions. Eur Heart J 32, 1838-1845.
Heidenreich, P.A., Albert, N.M., Allen, L.A., Bluemke, D.A., Butler, J., Fonarow, G.C., Ikonomidis, J.S., Khavjou, O., Konstam, M.A., Maddox, T.M., et al. (2013). Forecasting the impact of heart failure in the United States: a policy statement from the American Heart Association. Circ Heart Fail 6, 606-619.
Hosoda, H., Kojima, M., Matsuo, H., and Kangawa, K. (2000). Ghrelin and des-acyl ghrelin: two major forms of rat ghrelin peptide in gastrointestinal tissue. Biochemical and biophysical research communications 279, 909-913.
Isgaard, J. (2013). Ghrelin and the cardiovascular system. Endocrine development 25, 83-90.
Janssen, J.A., Poldermans, D., Hofland, L.J., Vourvouri, E.C., Muller, A.F., Bax, J.J., Deghenghi, R., Broglio, F., Ghigo, E., and van der Lely, A.J. (2004a). There are no acute cardiac effects of a single iv dose of human ghrelin in severe growth hormone deficient patients. Journal of endocrinological investigation 27, 659-664.
Janssen, J.A., van der Lely, A.J., and Lamberts, S.W. (2004b). Is there a role of ghrelin in preventing catabolism? Journal of endocrinological investigation 27, 400-403.
Kishimoto, I., Tokudome, T., Hosoda, H., Miyazato, M., and Kangawa, K. (2012). Ghrelin and cardiovascular diseases. Journal of cardiology 59, 8-13.
Kojima, M., Hosoda, H., Date, Y., Nakazato, M., Matsuo, H., and Kangawa, K. (1999). Ghrelin is a growth-hormone-releasing acylated peptide from stomach. Nature 402, 656-660.
Kojima, M., and Kangawa, K. (2005). Ghrelin: structure and function. Physiol Rev 85, 495-522.
Krell, M.J., Kline, E.M., Bates, E.R., Hodgson, J.M., Dilworth, L.R., Laufer, N., Vogel, R.A., and Pitt, B. (1986). Intermittent, ambulatory dobutamine infusions in patients with severe congestive heart failure. Am Heart J 112, 787-791.
Kurmani S, Squire I. Acute Heart Failure: Definition, Classification and Epidemiology. Curr Heart Fail Rep. 2017 Oct;14(5):385-392.
Layland, J., Solaro, R.J., and Shah, A.M. (2005). Regulation of cardiac contractile function by troponin I phosphorylation. Cardiovasc Res 66, 12-21.
Leal-Cerro, A., Torres, E., Soto, A., Dios, E., Deghenghi, R., Arvat, E., Ghigo, E., Dieguez, C., and Casanueva, F.F. (2002). Ghrelin is no longer able to stimulate growth hormone secretion in patients with Cushing's syndrome but instead induces exaggerated corticotropin and cortisol responses. Neuroendocrinology 76, 390-396.
Lear, P.V., Iglesias, M.J., Feijoo-Bandin, S., Rodriguez-Penas, D., Mosquera-Leal, A., Garcia-Rua, V., Gualillo, O., Ghe, C., Arnoletti, E., Muccioli, G., et al. (2010). Des-acyl ghrelin has specific binding sites and different metabolic effects from ghrelin in cardiomyocytes. Endocrinology 151, 3286-3298.
Leite-Moreira, A.F., Rocha-Sousa, A., and Henriques-Coelho, T. (2008). Cardiac, skeletal, and smooth muscle regulation by ghrelin. Vitamins and hormones 77, 207-238.
Levin, F., Edholm, T., Schmidt, P.T., Gryback, P., Jacobsson, H., Degerblad, M., Hoybye, C., Holst, J.J., Rehfeld, J.F., Hellstrom, P.M., et al. (2006). Ghrelin stimulates gastric emptying and hunger in normal-weight humans. J Clin Endocrinol Metab 91, 3296-3302. Li, W.G., Gavrila, D., Liu, X., Wang, L., Gunnlaugsson, S., Stoll, L.L., McCormick, M.L., Sigmund, C.D., Tang, C., and Weintraub, N.L. (2004). Ghrelin inhibits proinflammatory responses and nuclear factor-kappaB activation in human endothelial cells. Circulation 109, 2221-2226.
Lund, L.H., Carrero, J.J., Farahmand, B., Henriksson, K.M., Jonsson, Å., Jernberg, T., Dahlström, U. (2017). Association between enrolment in a heart failure quality registry and subsequent mortality - a nationwide cohort study. Eur J Heart Fail in press*.*
Lund, L.H., Williams, J.J., Freda, P., LaManca, J.J., LeJemtel, T.H., and Mancini, D.M. (2009). Ghrelin resistance occurs in severe heart failure and resolves after heart transplantation. Eur J Heart Fail 11, 789-794.
Mebazaa, A., Nieminen, M.S., Packer, M., Cohen-Solal, A., Kleber, F.X., Pocock, S.J., Thakkar, R., Padley, R.J., Poder, P., and Kivikko, M. (2007). Levosimendan vs dobutamine for patients with acute decompensated heart failure: the SURVIVE Randomized Trial. Jama 297, 1883-1891.
Miki, K., Maekura, R., Nagaya, N., Nakazato, M., Kimura, H., Murakami, S., Ohnishi, S., Hiraga, T., Miki, M., Kitada, S., et al. (2012). Ghrelin treatment of cachectic patients with chronic obstructive pulmonary disease: a multicenter, randomized, double-blind, placebo-controlled trial. PloS one 7, e35708.
Nagaya, N., and Kangawa, K. (2003a). Ghrelin improves left ventricular dysfunction and cardiac cachexia in heart failure. Curr Opin Pharmacol 3, 146-151.
Nagaya, N., and Kangawa, K. (2003b). Ghrelin, a novel growth hormone-releasing peptide, in the treatment of chronic heart failure. Regul Pept 114, 71-77.
Nagaya, N., and Kangawa, K. (2006). Therapeutic potential of ghrelin in the treatment of heart failure. Drugs 66, 439-448.
Nagaya, N., Kojima, M., and Kangawa, K. (2006). Ghrelin, a novel growth hormone-releasing peptide, in the treatment of cardiopulmonary-associated cachexia. Intern Med 45, 127-134.
Nagaya, N., Kojima, M., Uematsu, M., Yamagishi, M., Hosoda, H., Oya, H., Hayashi, Y., and Kangawa, K. (2001a). Hemodynamic and hormonal effects of human ghrelin in healthy volunteers. American journal of physiology Regulatory, integrative and comparative physiology 280, R1483-1487.
Nagaya, N., Miyatake, K., Uematsu, M., Oya, H., Shimizu, W., Hosoda, H., Kojima, M., Nakanishi, N., Mori, H., and Kangawa, K. (2001b). Hemodynamic, renal, and hormonal effects of ghrelin infusion in patients with chronic heart failure. J Clin Endocrinol Metab 86, 5854-5859.
Nagaya, N., Moriya, J., Yasumura, Y., Uematsu, M., Ono, F., Shimizu, W., Ueno, K., Kitakaze, M., Miyatake, K., and Kangawa, K. (2004). Effects of ghrelin administration on left ventricular function, exercise capacity, and muscle wasting in patients with chronic heart failure. Circulation 110, 3674-3679.
Nagaya, N., Uematsu, M., Kojima, M., Date, Y., Nakazato, M., Okumura, H., Hosoda, H., Shimizu, W., Yamagishi, M., Oya, H., et al. (2001c). Elevated circulating level of ghrelin in cachexia associated with chronic heart failure: relationships between ghrelin and anabolic/catabolic factors. Circulation 104, 2034-2038.
Nagaya, N., Uematsu, M., Kojima, M., Ikeda, Y., Yoshihara, F., Shimizu, W., Hosoda, H., Hirota, Y., Ishida, H., Mori, H., et al. (2001d). Chronic administration of ghrelin improves left ventricular dysfunction and attenuates development of cardiac cachexia in rats with heart failure. Circulation 104, 1430-1435.
Okumura, H., Nagaya, N., Enomoto, M., Nakagawa, E., Oya, H., and Kangawa, K. (2002). Vasodilatory effect of ghrelin, an endogenous peptide from the stomach. Journal of cardiovascular pharmacology 39, 779-783.
Oliva, F., Latini, R., Politi, A., Staszewsky, L., Maggioni, A.P., Nicolis, E., and Mauri, F. (1999). Intermittent 6-month low-dose dobutamine infusion in severe heart failure: DICE multicenter trial. Am Heart J 138, 247-253.
Packer, M., Carver, J.R., Rodeheffer, R.J., Ivanhoe, R.J., DiBianco, R., Zeldis, S.M., Hendrix, G.H., Bommer, W.J., Elkayam, U., Kukin, M.L., et al. (1991). Effect of oral milrinone on mortality in severe chronic heart failure. The PROMISE Study Research Group. N Engl J Med 325, 1468-1475.
Packer, M., Colucci, W., Fisher, L., Massie, B.M., Teerlink, J.R., Young, J., Padley, R.J., Thakkar, R., Delgado-Herrera, L., Salon, J., et al. (2013). Effect of Levosimendan on the Short-Term Clinical Course of Patients With Acutely Decompensated Heart Failure. JACC Heart Failure 1, 103-111.
Papotti, M., Ghe, C., Cassoni, P., Catapano, F., Deghenghi, R., Ghigo, E., and Muccioli, G. (2000). Growth hormone secretagogue binding sites in peripheral human tissues. J Clin Endocrinol Metab 85, 3803-3807.
Pei XM, Yung BY, Yip SP, Chan LW, Wong CS, Ying M, Siu PM. Protective effects of desacyl ghrelin on diabetic cardiomyopathy. Acta Diabetol. 2015 Apr;52(2):293-306.
Peino, R., Baldelli, R., Rodriguez-Garcia, J., Rodriguez-Segade, S., Kojima, M., Kangawa, K., Arvat, E., Ghigo, E., Dieguez, C., and Casanueva, F.F. (2000). Ghrelin-induced growth hormone secretion in humans. European journal of endocrinology / European Federation of Endocrine Societies 143, R11-14.
Perrino, C., Schiattarella, G.G., Sannino, A., Pironti, G., Petretta, M.P., Cannavo, A., Gargiulo, G., Ilardi, F., Magliulo, F., Franzone, A., et al. (2013). Genetic deletion of uncoupling protein 3 exaggerates apoptotic cell death in the ischemic heart leading to heart failure. J Am Heart Assoc 2, e000086.
Pironti, G., Ivarsson, N., Yang, J., Farinotti, A.B., Jonsson, W., Zhang, S.J., Bas, D., Svensson, C.I., Westerblad, H., Weitzberg, E., et al. (2016). Dietary nitrate improves cardiac contractility via enhanced cellular Ca(2)(+) signaling. Basic research in cardiology 111, 34.
Polanczyk, C.A., Rohde, L.E., Dec, G.W., and DiSalvo, T. (2000). Ten-year trends in hospital care for congestive heart failure: improved outcomes and increased use of resources. Arch Intern Med 160, 325-332.
Schwenke, D.O., Tokudome, T., Kishimoto, I., Horio, T., Shirai, M., Cragg, P.A., and Kangawa, K. (2008). Early ghrelin treatment following myocardial infarction prevents an increase in cardiac sympathetic tone and reduces mortality. Endocrinology.
Shiiya, T., Nakazato, M., Mizuta, M., Date, Y., Mondal, M.S., Tanaka, M., Nozoe, S., Hosoda, H., Kangawa, K., and Matsukura, S. (2002). Plasma ghrelin levels in lean and obese humans and the effect of glucose on ghrelin secretion. J Clin Endocrinol Metab 87, 240-244.
Soares, J.B., and Leite-Moreira, A.F. (2008). Ghrelin, des-acyl ghrelin and obestatin: three pieces of the same puzzle. Peptides 29, 1255-1270.
Soares, J.B., Rocha-Sousa, A., Castro-Chaves, P., Henriques-Coelho, T., Lourenco, A.P., Roncon-Albuquerque, R., Jr., and Leite-Moreira, A.F. (2005). Contractile effects of Ghrelin and expression of its receptor GHS-R1a in normal and hypertrophic myocardium. Revista portuguesa de cardiologia : orgao oficial da Sociedade Portuguesa de Cardiologia = Portuguese journal of cardiology : an official journal of the Portuguese Society of Cardiology 24, 1235-1242.
Soeki, T., Kishimoto, I., Schwenke, D.O., Tokudome, T., Horio, T., Yoshida, M., Hosoda, H., and Kangawa, K. (2008). Ghrelin suppresses cardiac sympathetic activity and prevents early left ventricular remodeling in rats with myocardial infarction. Am J Physiol Heart Circ Physiol 294, H426-432.
Stahlberg, M., Damgaard, M., Norsk, P., Gabrielsen, A., Sahlen, A., Linde, C., and Braunschweig, F. (2009). Cardiac output response to changes of the atrioventricular delay in different body positions and during exercise in patients receiving cardiac resynchronization therapy. Europace 11, 1160-1167.
Sun, Q., Ma, Y., Zhang, L., Zhao, Y.F., Zang, W.J., and Chen, C. (2010a). Effects of GH secretagogues on contractility and Ca2+ homeostasis of isolated adult rat ventricular myocytes. Endocrinology 151, 4446-4454.
Sun, Q., Zang, W.J., and Chen, C. (2010b). Growth hormone secretagogues reduce transient outward K+ current via phospholipase C/protein kinase C signaling pathway in rat ventricular myocytes. Endocrinology 151, 1228-1235.
Tassone, F., Broglio, F., Destefanis, S., Rovere, S., Benso, A., Gottero, C., Prodam, F., Rossetto, R., Gauna, C., van der Lely, A.J., et al. (2003). Neuroendocrine and metabolic effects of acute ghrelin administration in human obesity. J Clin Endocrinol Metab 88, 5478-5483.
Tesauro, M., Schinzari, F., lantorno, M., Rizza, S., Melina, D., Lauro, D., and Cardillo, C. (2005). Ghrelin improves endothelial function in patients with metabolic syndrome. Circulation 112, 2986-2992.
Thorvaldsen, T., Benson, L., Dahlstrom, U., Edner, M., and Lund, L.H. (2016). Use of evidence-based therapy and survival in heart failure in Sweden 2003-2012. Eur J Heart Fail 18, 503-511.
Uretsky, B.F., Jessup, M., Konstam, M.A., Dec, G.W., Leier, C.V., Benotti, J., Murali, S., Herrmann, H.C., and Sandberg, J.A. (1990). Multicenter trial of oral enoximone in patients with moderate to moderately severe congestive heart failure. Lack of benefit compared with placebo. Enoximone Multicenter Trial Group. Circulation 82, 774-780.
Warburton, D.E., Haykowsky, M.J., Quinney, H.A., Humen, D.P., and Teo, K.K. (1999). Reliability and validity of measures of cardiac output during incremental to maximal aerobic exercise. Part I: Conventional techniques. Sports Med 27, 23-41.
Vestergaard, E.T., Andersen, N.H., Hansen, T.K., Rasmussen, L.M., Moller, N., Sorensen, K.E., Sloth, E., and Jorgensen, J.O. (2007a). Cardiovascular effects of intravenous ghrelin infusion in healthy young men. Am J Physiol Heart Circ Physiol 293, H3020-3026.
Vestergaard, E.T., Dall, R., Lange, K.H., Kjaer, M., Christiansen, J.S., and Jorgensen, J.O. (2007b). The ghrelin response to exercise before and after growth hormone administration. J Clin Endocrinol Metab 92, 297-303.
Vestergaard, E.T., Djurhuus, C.B., Gjedsted, J., Nielsen, S., Moller, N., Holst, J.J., Jorgensen, J.O., and Schmitz, O. (2008). Acute effects of ghrelin administration on glucose and lipid metabolism. J Clin Endocrinol Metab 93, 438-444.
Vestergaard, E.T., Hansen, T.K., Gormsen, L.C., Jakobsen, P., Moller, N., Christiansen, J.S., and Jorgensen, J.O. (2007c). Constant intravenous ghrelin infusion in healthy young men: clinical pharmacokinetics and metabolic effects. American journal of physiology Endocrinology and metabolism 292, E1829-1836.
Yang, C., Liu, Z., Liu, K., and Yang, P. (2014). Mechanisms of Ghrelin anti-heart failure: inhibition of Ang II-induced cardiomyocyte apoptosis by down-regulating AT1R expression. PloS one 9, e85785.
Zabarovskaja, S., Freda, P., Williams, J.J., Kunavarapu, C., Lamanca, J., Mancini, D., and Lund, L.H. (2014). Acylation of ghrelin is increased in heart failure and decreases post heart transplantation. Scandinavian cardiovascular journal : SCJ 48, 343-348.

## Claims

1. An acylated ghrelin molecule for use in the treatment of Acute Heart Failure (AHF) in an individual.

2. The acylated ghrelin molecule for use according to Claim 1, wherein the AHF is selected from the group comprising: Acute Decompensated Heart Failure (ADHF), for example Acute Decompensated Chronic Heart Failure (ADCHF) or congestive ADHF, preferably Acute Decompensated Chronic Heart Failure (ADCHF); AHF associated with hypertension; tachycardia-mediated AHF; AHF associated with pulmonary edema; cardiogenic shock AHF; or severe cardiogenic shock AHF.

3. The acylated ghrelin molecule for use according to any one of Claims 1-2, wherein the AHF is not associated with a myocardial infarction and/or wherein the AHF is not *de novo* AHF.

4. The acylated ghrelin molecule for use according to any one of Claims 1-3, for use in further achieving one or more parameters from the group comprising: an increased heart output; and/or an increased heart contraction; and/or an increased heart stroke volume; and/or an increased ventricular function; and/or an increased ventricular ejection fraction; and/or reduced troponin I phosphorylation; and/or an increased calcium sensitivity; and/or reduced intracellular cAMP; and/or improved renal function; and/or improved estimated glomerular filtration rate (eGFR); and/or improved dyspnea; and/or improved edema; and/or reduced biomarkers; and/or reduced hypotension; and/or resolution of cardiogenic shock; and/or reduced dizziness; and/or reduced light-headedness; and/or a reduced arterio venous oxygen (AVO2) difference; and/or an increase in pulmonary blood flow (PBF); and/or a reduced estimated systemic vascular resistance (eSVR); and/or reduced pulmonary capillary wedge pressure; and/or reduced left ventricular end-diastolic pressure; and/or reduced left ventricular end-diastolic volume; and/or reduced pulmonary artery pressure; and/or reduced central venous pressure.

5. The acylated ghrelin molecule for use according to Claim 4, for use in further achieving one or more parameters from the group comprising: an increased heart output; and/or a reduced arterio venous oxygen (AVO2) difference; and/or an increase in pulmonary blood flow (PBF); and/or a reduced estimated systemic vascular resistance (eSVR).

6. The acylated ghrelin molecule for use according to any one of Claims 1-5, wherein the AHF is associated with one or more factors of the group comprising: spontaneous worsening of chronic heart failure; an infection; and/or an allergic reaction; and/or a blood clot; and/or surgery; and/or cardiovascular disease; and/or lung disease; and/or cardiomyopathy; and/or sleep apnea; and/or alcohol consumption; and/or recreational drug consumption; and/or anaemia; and/or dyslipidemia; and/or an overactive thyroid; and/or Paget's disease; and/or hypertension (such as pulmonary hypertension); and/or prescription medication consumption; and/or smoking; and/or high blood pressure; and/or kidney dysfunction; and/or diabetes; and/or congenital heart defects; and/or lifestyle choices; and/or an irregular heartbeat; and/or rapid heartbeat; and/or slow heartbeat; and/or inflammation; and/or toxins; and/or autoimmune disease; and/or infiltrative disease; and/or connective tissue disease; and/or metabolic disease; and/or endocrine disease; and old age; and/or hereditary genetic mutations; and/or pregnancy.

7. The acylated ghrelin molecule for use according to any one of Claims 1-6, wherein the AHF comprises one or more of the symptoms of the group comprising: chest pain; and/or a cough; and/or shock (such as, cardiogenic shock); and/or high blood pressure; and/or oliguria; and/or anuria; and/or fatigue; and/or shortness of breath (dyspnea); and/or hypoxemia; and/or rapid breathing (tachypnea); and/or tachycardia; and/or ischemia; and/or edema (such as, a worsening edema); and/or impaired renal function (such as, worsening renal function); and/or low blood pressure; and/or organ failure (such as liver failure and/or kidney failure); and/or cold extremities; and/or numb extremities; and/or muscle fatigue; and/or nausea; and/or vomiting; and/or weight loss (such as anorexia); and/or pulmonary edema; and/or discomfort of the lower body; and/or peripheral swelling; and/or hypoperfusion; and/or swelling of the lower body; and/or swelling of the heart; and/or weight gain (such as, sudden weight gain); and/or weight loss; and/or cachexia; and/or elevated neck blood vessels (such as an elevated neck blood vein and/or jugular venous distension); and/or hepatomegaly; and/or dizziness; and/or fainting (also known as syncope); and/or an altered mental state (for example, anxiety and/or confusion and/or depression); and/or loss of appetite; hypotension; and/or arrhythmia; and/or difficulty sleeping; and/or discomfort when lying flat; and/or sleep apnea.

8. The acylated ghrelin molecule for use according to any one of Claims 1-7, wherein the individual is diagnosed as having AHF using one or more of the procedures of the group comprising: an X-ray; and/or a blood test; and/or an electrocardiogram (ECG); and/or based on the medical history of the individual; and/or a positron emission tomography (PET) scan; and/or multigated acquisition (MUGA) scan; and/or scintigraphy; and/or an echocardiogram; and/or an angiogram; and/or hemodynamic measurement; and/or a computerised tomography (CT) scan; and/or a physical examination of symptoms; and/or measuring biomarkers (such as serum natriuretic peptides and/or plasma natriuretic peptides); and/or, a magnetic resonance imaging (MRI) scan.

9. The acylated ghrelin molecule for use according to any one of Claims 1-8, wherein after administration of the ghrelin molecule the individual does not exhibit one or more parameters of the group comprising: an increased heart rate; and/or tachycardia; and/or a decreased blood pressure; and/or hypotension; and/or an increased oxygen demand; and/or ischemia; and/or increased plasma troponin T; and/or heart arrhythmias; and/or affected calcium transients.

10. The acylated ghrelin molecule for use according to any one of Claims 1-9, wherein the acylated ghrelin molecule comprises wildtype ghrelin.

11. The acylated ghrelin molecule for use according to any one of Claims 1-10, wherein the acylated ghrelin molecule comprises one or more of the group comprising: a synthetic ghrelin molecule; and/or a recombinant ghrelin molecule; and/or, an endogenous ghrelin molecule.

12. The acylated ghrelin molecule for use according to any one of Claims 1-11, wherein the ghrelin molecule is administered once or more each day, preferably twice each day.

13. The acylated ghrelin molecule for use according to any one of Claims 1-12, wherein the ghrelin molecule is administered by infusion.

14. The acylated ghrelin molecule for use according to any one of Claims 1-13, wherein the individual is aged 18 years or older, preferably 65 years or older.

15. The acylated ghrelin molecule for use according to any one of Claims 1-14, wherein the ghrelin molecule is administered before surgery, and/or during surgery, and/or after surgery.

16. The acylated ghrelin molecule for use according to any one of Claims 1-15, wherein the individual is administered with one or more additional therapeutic agents.

17. The acylated ghrelin molecule for use according to any one of Claims 1-16, wherein the ghrelin molecule is in a composition, preferably a pharmaceutical composition.

18. The acylated ghrelin molecule for use according to Claim 17, wherein the composition comprises one or more additional therapeutic agents.

19. The acylated ghrelin molecule for use according to any one of Claims 16 and 18, wherein the therapeutic agent is one or more therapeutic agent selected from the group comprising: angiotensin-converting enzyme (ACE) inhibitors; and/or angiotensin II receptor blockers; and/or vasopressin receptor antagonists; and/or beta blockers; and/or an inodilator (in particular, mirinone and/or enoximone and/or dobutamine and/or levosimendan); and/or omecamtiv mecarbil; and/or renin antagonist; and/or relaxin; and/or ularitide; and/or digoxin (Lanoxin); and/or vasodilators; and/or angiotensin II receptor antagonists (such as valsartan); and/or aspirin; and/or statins; and/or antihypertensive drugs (such as sacubitril); and/or calcium sensitisers; and/or ivabradine; and/or diuretics; and/or vasopressor (such as noradrenaline, dopamine, vasopressin, and/or angiotensin II); and/or adenosine antagonists; and/or aldosterone antagonists.

## Patentansprüche

1. Acyliertes Ghrelinmolekül zur Verwendung bei der Behandlung von akuter Herzinsuffizienz (AHF) bei einer Person.

2. Acyliertes Ghrelinmolekül zur Verwendung gemäß Anspruch 1, wobei die AHF ausgewählt ist aus der Gruppe, die Folgendes umfasst: Akute dekompensierte Herzinsuffizienz (ADHF), beispielsweise akute dekompensierte chronische Herzinsuffizienz (ADCHF) oder kongestive ADHF, vorzugsweise akute dekompensierte chronische Herzinsuffizienz (ADCHF), AHF in Verbindung mit Bluthochdruck, tachykardievermittelte AHF, AHF in Verbindung mit Lungenödem, AHF mit kardiogenem Schock oder schwere AHF mit kardiogenem Schock.

3. Acyliertes Ghrelinmolekül zur Verwendung gemäß einem der Ansprüche 1 bis 2, wobei die AHF nicht mit einem Myokardinfarkt verbunden ist und/oder wobei es sich bei der AHF nicht um eine *de-novo-AHF* handelt.

4. Acyliertes Ghrelinmolekül zur Verwendung gemäß einem der Ansprüche 1 bis 3, zur Verwendung beim weiteren Erzielen eines oder mehrerer Parameter aus der Gruppe, umfassend eine gesteigerte Herzleistung und/oder eine gesteigerte Herzkontraktion und/oder ein gesteigertes Herzschlagvolumen und/oder eine gesteigerte ventrikuläre Funktion und/oder eine gesteigerte ventrikuläre Auswurffraktion und/oder eine verringerte Troponin I-Phosphorylierung und/oder eine gesteigerte Kalziumempfindlichkeit und/oder verringertes intrazelluläres cAMP und/oder eine verbesserte Nierenfunktion und/oder eine verbesserte geschätzte glomeruläre Filtrationsrate (eGFR) und/oder eine verringerte Dyspnoe und/oder ein verringertes Ödem und/oder verringerte Biomarker und/oder eine verringerte Hypotonie und/oder Auflösung eines kardiogenen Schocks und/oder verringerter Schwindel und/oder verringerte Benommenheit und/oder eine verringerte arterio-venöse Sauerstoffdifferenz (AVO2) und/oder ein Anstieg des pulmonalen Blutflusses (PBF) und/oder ein verringerter geschätzter systemischer Gefäßwiderstand (eSVR) und/oder ein verringerter pulmonaler kapillarer Verschlussdruck und/oder ein verringerter linksventrikulärer enddiastolischer Druck und/oder ein verringertes linksventrikuläres enddiastolisches Volumen und/oder ein verringerter Pulmonalarteriendruck und/oder ein verringerter zentraler Venendruck.

5. Acyliertes Ghrelinmolekül zur Verwendung gemäß Anspruch 4, zur Verwendung beim weiteren Erzielen eines oder mehrerer Parameter aus der Gruppe umfassend: eine erhöhte Herzleistung und/oder eine verringerte arterio-venöse Sauerstoffdifferenz (AVO2) und/oder eine Erhöhung des pulmonalen Blutflusses (PBF) und/oder einen verringerten geschätzten systemischen Gefäßwiderstand (eSVR).

6. Acyliertes Ghrelinmolekül zur Verwendung gemäß einem der Ansprüche 1 bis 5, wobei die AHF verbunden ist mit einem oder mehreren Faktoren aus der Gruppe, umfassend: spontane Verschlimmerung einer chronischen Herzinsuffizienz, einer Infektion und/oder einer allergischen Reaktion und/oder einem Blutgerinnsel und/oder einer Operation und/oder einer kardiovaskulären Erkrankung und/oder einer Lungenerkrankung und/oder einer Kardiomyopathie und/oder einer Schlafapnoe und/oder Alkoholkonsum und/oder Konsum von Partydrogen und/oder einer Anämie und/oder einer Dyslipidämie und/oder einer Schilddrüsenüberfunktion und/oder der Paget-Krankheit und/oder Bluthochdruck (wie etwa einer pulmonalen Hypertonie) und/oder dem Konsum von verschreibungspflichtigen Medikamenten und/oder Rauchen und/oder Bluthochdruck und/oder Nierenfunktionsstörungen und/oder Diabetes und/oder angeborenem Herzfehler und/oder Änderungen in der Lebensführung und/oder Herzrhythmusstörung und/oder Herzrasen und/oder Bradykardie und/oder Entzündungen und/oder Toxine und/oder Autoimmunerkrankungen und/oder infiltrative Erkrankungen und/oder Bindegewebserkrankungen und/oder Stoffwechselerkrankungen und/oder endokrine Erkrankungen und hohes Alter und/oder erbliche Genmutationen und/oder Schwangerschaft.

7. Acyliertes Ghrelinmolekül zur Verwendung gemäß einem der Ansprüche 1 bis 6, wobei die AHF eines oder mehrere der Symptome aus der Gruppe umfasst, umfassend: Brustschmerzen und/oder Husten und/oder Schock (wie etwa kardiogener Schock) und/oder Bluthochdruck und/oder Oligurie und/oder Anurie und/oder Erschöpfung und/oder Kurzatmigkeit (Dyspnoe) und/oder Hypoxämie und/oder schnelle Atmung (Tachypnoe) und/oder Tachykardie und/oder Ischämie und/oder Ödeme (wie etwa ein sich verschlimmerndes Ödem) und/oder beeinträchtigte Nierenfunktion (wie etwa eine sich verschlechternde Nierenfunktion) und/oder niedrigen Blutdruck und/oder Organversagen (wie etwa Leberversagen und/oder Nierenversagen) und/oder kalte Extremitäten und/oder taube Extremitäten und/oder Muskelermüdung und/oder Übelkeit und/oder Erbrechen und/oder Gewichtsverlust (wie etwa Anorexie); und/oder Lungenödem und/oder Beschwerden im Unterkörper und/oder periphere Schwellungen und/oder Hypoperfusion und/oder Schwellungen im Unterkörper und/oder Schwellungen des Herzens und/oder Gewichtszunahme (wie etwa plötzliche Gewichtszunahme) und/oder Gewichtsverlust und/oder Kachexie und/oder erhabene Halsblutgefäße (wie etwa erhabene Halsvenen und/oder juguläre Venenvergrößerung) und/oder Hepatomegalie und/oder Schwindel und/oder Ohnmacht (auch bekannt als Synkope) und/oder eine veränderte psychische Verfassung (beispielsweise Angstzustände und/oder Verwirrung und/oder Depressionen) und/oder Appetitlosigkeit, Hypotonie und/oder Arrhythmie und/oder Schlafstörungen und/oder Beschwerden beim Liegen und/oder Schlafapnoe.

8. Acyliertes Ghrelinmolekül zur Verwendung gemäß einem der Ansprüche 1 bis 7, wobei bei der Person AHF diagnostiziert wird mithilfe eines oder mehrerer Verfahren aus der Gruppe, umfassend: eine Röntgenaufnahme und/oder eine Blutuntersuchung und/oder ein Elektrokardiogramm (EKG) und/oder basierend auf der Grundlage der Krankengeschichte der Person und/oder eine Positronen-Emissions-Tomographie (PET) und/oder ein MUGA-Scan (Multigated-Acquisition-Scan) und/oder eine Szintigraphie und/oder ein Echokardiogramm und/oder ein Angiogramm und/oder eine hämodynamische Messung und/oder eine Computertomographie (CT) und/oder eine körperliche Untersuchung der Symptome und/oder die Messung von Biomarkern (wie etwa Serum-Natriuretische Peptide und/oder Plasma-Natriuretische Peptide) und/oder eine Magnetresonanztomographie (MRT).

9. Acyliertes Ghrelinmolekül zur Verwendung gemäß einem der Ansprüche 1 bis 8, wobei die Person nach der Verabreichung des Ghrelinmoleküls nicht einen oder mehrere Parameter der Gruppe aufweist, umfassend: eine erhöhte Herzfrequenz und/oder Tachykardie und/oder einen verringerten Blutdruck und/oder Hypotonie und/oder einen erhöhten Sauerstoffbedarf und/oder Ischämie und/oder erhöhtes Plasmatroponin T und/oder Herzrhythmusstörungen und/oder beeinträchtigte Kalziumtransienten.

10. Acyliertes Ghrelinmolekül zur Verwendung gemäß einem der Ansprüche 1 bis 9, wobei das acylierte Ghrelinmolekül Ghrelin vom Wildtyp umfasst.

11. Acyliertes Ghrelinmolekül zur Verwendung gemäß einem der Ansprüche 1 bis 10, wobei das acylierte Ghrelinmolekül eines oder mehrere aus der folgenden Gruppe umfasst: ein synthetisches Ghrelinmolekül und/oder ein rekombinantes Ghrelinmolekül und/oder ein endogenes Ghrelinmolekül.

12. Acyliertes Ghrelinmolekül zur Verwendung gemäß einem der Ansprüche 1 bis 11, wobei das Ghrelinmolekül einmal oder mehrmals pro Tag, vorzugsweise zweimal pro Tag, verabreicht wird.

13. Acyliertes Ghrelinmolekül zur Verwendung gemäß einem der Ansprüche 1 bis 12, wobei das Ghrelinmolekül durch Infusion verabreicht wird.

14. Acyliertes Ghrelinmolekül zur Verwendung gemäß einem der Ansprüche 1 bis 13, wobei die Person 18 Jahre oder älter, vorzugsweise 65 Jahre oder älter ist.

15. Acyliertes Ghrelinmolekül zur Verwendung gemäß einem der Ansprüche 1 bis 14, wobei das Ghrelinmolekül vor einer Operation und/oder während einer Operation und/oder nach einer Operation verabreicht wird.

16. Acyliertes Ghrelinmolekül zur Verwendung gemäß einem der Ansprüche 1 bis 15, wobei es der Person mit einem oder mehreren zusätzlichen therapeutischen Wirkstoffen verabreicht wird.

17. Acyliertes Ghrelinmolekül zur Verwendung gemäß einem der Ansprüche 1 bis 16, wobei das Ghrelinmolekül in einer Zusammensetzung, vorzugsweise einer pharmazeutischen Zusammensetzung, vorliegt.

18. Acyliertes Ghrelinmolekül zur Verwendung gemäß Anspruch 17, wobei die Zusammensetzung einen oder mehrere zusätzliche therapeutische Wirkstoffe umfasst.

19. Acyliertes Ghrelinmolekül zur Verwendung gemäß einem der Ansprüche 16 und 18, wobei es sich bei dem therapeutischen Wirkstoff um einen oder mehrere therapeutische Wirkstoffe handelt, ausgewählt aus der Gruppe, umfassend: Angiotensin-Converting-Enzym-Hemmer (ACE-Hemmer) und/oder Angiotensin-II-Rezeptorblocker und/oder Vasopressin-Rezeptorantagonisten und/oder Betablocker und/oder einen Inodilator (insbesondere Mirinon und/oder Enoximon und/oder Dobutamin und/oder Levosimendan) und/oder Omecamtiv-Mecarbil und/oder Renin-Antagonisten und/oder Relaxin und/oder Ularitid und/oder Digoxin (Lanoxin) und/oder Vasodilatatoren und/oder Angiotensin-II-Rezeptorantagonisten (wie etwa Valsartan) und/oder Aspirin und/oder Statine und/oder blutdrucksenkende Arzneimittel (wie etwa Sacubitril) und/oder Calcium-Sensibilisatoren und/oder Ivabradin und/oder Diuretika und/oder Vasopressoren (wie etwa Noradrenalin, Dopamin, Vasopressin und/oder Angiotensin II) und/oder Adenosin-Antagonisten und/oder Aldosteron-Antagonisten.

## Revendications

1. Molécule de ghréline acylée destinée à être utilisée dans le traitement de l'insuffisance cardiaque aiguë (AHF) chez un individu.

2. Molécule de ghréline acylée destinée à être utilisée selon la revendication 1, dans laquelle l'AHF est choisie dans le groupe comprenant : insuffisance cardiaque décompensée aiguë (ADHF), par exemple insuffisance cardiaque chronique décompensée aiguë (ADCHF) ou ADHF congestive, de préférence insuffisance cardiaque chronique décompensée aiguë (ADCHF) ; AHF associée à l'hypertension ; AHF médiée par la tachycardie ; AHF associée à un œdème pulmonaire ; choc cardiogénique AHF ; ou choc cardiogénique sévère AHF.

3. Molécule de ghréline acylée destinée à être utilisée selon l'une quelconque des revendications 1 et 2, dans laquelle l'AHF n'est pas associée à un infarctus du myocarde et/ou dans laquelle l'AHF n'est pas AHF *de novo.*

4. Molécule de ghréline acylée destinée à être utilisée selon l'une quelconque des revendications 1 à 3, destinée à être utilisée pour obtenir en outre un ou plusieurs paramètres du groupe comprenant : un débit cardiaque accru ; et/ou une contraction cardiaque accrue ; et/ou un volume accru d'AVC ; et/ou une fonction ventriculaire accrue ; et/ou une fraction d'éjection ventriculaire accrue ; et/ou une phosphorylation réduite de troponine I ; et/ou une sensibilité au calcium accrue ; et/ou une réduction du AMPc intracellulaire ; et/ou une amélioration de la fonction rénale ; et/ou une amélioration du taux estimé de filtration glomérulaire (RFGe) ; et/ou une amélioration de la dyspnée ; et/ou un œdème amélioré ; et/ou une diminution des biomarqueurs ; et/ou une hypotension réduite ; et/ou une résolution du choc cardiogénique ; et/ou étourdissements réduits ; et/ou vertige réduit ; et/ou différence réduite d'oxygène veineux artériel (AVO2) ; et/ou augmentation du débit sanguin pulmonaire (PBF) ; et/ou diminution de la résistance vasculaire systémique estimée (eSVR) ; et/ou réduction de la pression capillaire pulmonaire bloquée ; et/ou diminution de la pression ventriculaire gauche en fin de diastole ; et/ou diminution du volume ventriculaire gauche en fin de diastole ; et/ou diminution de la pression artérielle pulmonaire ; et/ou diminution de la pression veineuse centrale.

5. Molécule de ghréline acylée destinée à être utilisée selon la revendication 4, destinée à être utilisée pour obtenir en outre un ou plusieurs paramètres du groupe comprenant : une augmentation du débit cardiaque ; et/ou une réduction de la différence d'oxygène veineux artériel (AVO2) ; et/ou une augmentation du débit sanguin pulmonaire (PBF) ; et/ou une diminution de la résistance vasculaire systémique estimée (eSVR).

6. Molécule de ghréline acylée destinée à être utilisée selon l'une quelconque des revendications 1 à 5, dans laquelle l'AHF est associée à un ou plusieurs facteurs du groupe comprenant : aggravation spontanée d'une insuffisance cardiaque chronique ; une infection ; ou une réaction allergique ; et/ou un caillot sanguin ; et/ou une intervention chirurgicale ; et/ou une maladie cardiovasculaire ; et/ou une maladie pulmonaire ; et/ou une cardiomyopathie ; et/ou une apnée du sommeil ; et/ou une consommation d'alcool ; et/ou une consommation de drogues à des fins récréatives ; et/ou une anémie ; et/ou une dyslipidémie ; et/ou une hyperthyroïdie ; et/ou la maladie de Paget ; et/ou l'hypertension (comme l'hypertension pulmonaire) ; et/ou une consommation de médicaments sur ordonnance ; et/ou le tabagisme ; et/ou l'hypertension ; et/ou un dysfonctionnement rénal ; et/ou un diabète ; et/ou des malformations cardiaques congénitales ; et/ou des choix de mode de vie ; et/ou un rythme cardiaque irrégulier ; et/ou un rythme cardiaque rapide ; et/ou un rythme cardiaque lent ; et/ou une inflammation ; et/ou des toxines ; et/ou une maladie auto-immune ; et/ou une maladie infiltrative ; et/ou une maladie des tissus conjonctifs ; et/ou une maladie métabolique ; et/ou une maladie endocrinienne ; et la vieillesse ; et/ou des mutations génétiques héréditaires ; et/ou une grossesse.

7. Molécule de ghréline acylée destinée à être utilisée selon l'une quelconque des revendications 1 à 6, dans laquelle l'AHF comprend un ou plusieurs des symptômes du groupe comprenant : douleur thoracique ; et/ou toux ; et/ou choc (tel qu'un choc cardiogénique) ; et/ou hypertension artérielle ; et/ou oligurie ; et/ou anurie ; et/ou fatigue ; et/ou essoufflement (dyspnée) ; et/ou hypoxémie ; et/ou respiration rapide (tachypnée) ; et/ou tachycardie ; et/ou ischémie ; et/ou œdème (tel qu'une aggravation de l'œdème) ; et/ou altération de la fonction rénale (telle qu'une aggravation de la fonction rénale) ; et/ou hypotension artérielle ; et/ou défaillance d'organe (telle qu'une insuffisance hépatique et/ou une insuffisance rénale) ; et/ou extrémités froides ; et/ou extrémités engourdies ; et/ou fatigue musculaire ; et/ou nausées ; et/ou vomissements ; et/ou perte de poids (telle qu'anorexie) ; et/ou œdème pulmonaire ; et/ou gêne du bas du corps ; et/ou gonflement périphérique ; et/ou hypoperfusion ; et/ou gonflement du bas du corps ; et/ou gonflement du cœur ; et/ou prise de poids (telle qu'une prise de poids soudaine) ; et/ou perte de poids ; et/ou cachexie ; et/ou élévation des vaisseaux sanguins du cou (telle qu'une veine sanguine du cou élevée et/ou une distension veineuse jugulaire) ; et/ou hépatomégalie ; et/ou étourdissements ; et/ou évanouissement (également connu sous le nom de syncope) ; et/ou un état mental altéré (par exemple, anxiété et/ou confusion et/ou dépression) ; et/ou perte d'appétit ; hypotension ; et/ou arythmie ; et/ou difficulté à dormir ; et/ou inconfort en position allongée ; et/ou apnée du sommeil.

8. Molécule de ghréline acylée destinée à être utilisée selon l'une quelconque des revendications 1 à 7, dans laquelle l'individu est diagnostiqué comme ayant AHF en utilisant un ou plusieurs des procédés du groupe comprenant : une radiographie ; et/ou un test sanguin ; et/ou un électrocardiogramme (ECG) ; et/ou en fonction des antécédents médicaux de l'individu ; et/ou une tomographie à positons (TEP) ; et/ou une scintigraphie par acquisition multi-synchronisée (MUGA) ; et/ou une scintigraphie ; et/ou un échocardiogramme ; et/ou un angiogramme ; et/ou une mesure hémodynamique ; et/ou une tomodensitométrie (TDM) ; et/ou un examen physique des symptômes ; et/ou la mesure de biomarqueurs (tels que les peptides natriurétiques sériques et/ou les peptides natriurétiques plasmatiques) ; et/ou une imagerie par résonance magnétique (IRM).

9. Molécule de ghréline acylée destinée à être utilisée selon l'une quelconque des revendications 1 à 8, dans laquelle, après administration de la molécule de ghréline, l'individu ne présente pas un ou plusieurs paramètres du groupe comprenant : une augmentation du rythme cardiaque ; et/ou une tachycardie ; et/ou une diminution de la pression artérielle ; et/ou une hypotension ; et/ou une augmentation de la demande en oxygène ; et/ou une ischémie ; et/ou une augmentation de la troponine T plasmatique ; et/ou des arythmies cardiaques ; et/ou des transitoires calciques affectés.

10. Molécule de ghréline acylée destinée à être utilisée selon l'une quelconque des revendications 1 à 9, dans laquelle la molécule de ghréline acylée comprend une ghréline de type sauvage.

11. Molécule de ghréline acylée destinée à être utilisée selon l'une quelconque des revendications 1 à 10, dans laquelle la molécule de ghréline acylée comprend un ou plusieurs du groupe comprenant : une molécule de ghréline synthétique ; et/ou une molécule de ghréline recombinante ; et/ou une molécule de ghréline endogène.

12. Molécule de ghréline acylée destinée à être utilisée selon l'une quelconque des revendications 1 à 11, dans laquelle la molécule de ghréline est administrée une ou plusieurs fois par jour, de préférence deux fois par jour.

13. Molécule de ghréline acylée destinée à être utilisée selon l'une quelconque des revendications 1 à 12, dans laquelle la molécule de ghréline est administrée par perfusion.

14. Molécule de ghréline acylée destinée à être utilisée selon l'une quelconque des revendications 1 à 13, dans laquelle l'individu est âgé de 18 ans ou plus, de préférence de 65 ans ou plus.

15. Molécule de ghréline acylée destinée à être utilisée selon l'une quelconque des revendications 1 à 14, dans laquelle la molécule de ghréline est administrée avant une intervention chirurgicale, et/ou pendant une intervention chirurgicale, et/ou après une intervention chirurgicale.

16. Molécule de ghréline acylée destinée à être utilisée selon l'une quelconque des revendications 1 à 15, dans laquelle on administre un ou plusieurs agents thérapeutiques supplémentaires à l'individu.

17. Molécule de ghréline acylée pour utilisation selon l'une quelconque des revendications 1 à 16, dans laquelle la molécule de ghréline est dans une composition, de préférence une composition pharmaceutique.

18. Molécule de ghréline acylée destinée à être utilisée selon la revendication 17, dans laquelle la composition comprend un ou plusieurs agents thérapeutiques supplémentaires.

19. Molécule de ghréline acylée destinée à être utilisée selon l'une quelconque des revendications 16 et 18, dans laquelle l'agent thérapeutique est un ou plusieurs agents thérapeutiques choisis dans le groupe comprenant : inhibiteurs de l'enzyme de conversion de l'angiotensine (ACE) ; et/ou antagonistes des récepteurs de l'angiotensine II ; et/ou antagonistes des récepteurs de la vasopressine ; et/ou bêtabloquants ; et/ou un inodilatateur (en particulier, mirinone et/ou énoximone et/ou dobutamine et/ou lévosimendan) ; et/ou omecamtiv mécarbil ; et/ou antagoniste de la rénine ; et/ou relaxine ; et/ou ularitide ; et/ou digoxine (Lanoxin) ; et/ou vasodilatateurs ; et/ou antagonistes des récepteurs de l'angiotensine II (tels que le valsartan) ; et/ou aspirine ; et/ou statines ; et/ou médicaments antihypertenseurs (tels que le sacubitril) ; et/ou sensibilisateurs calciques ; et/ou ivabradine ; et/ou diurétiques ; et/ou vasopresseur (tel que la noradrénaline, la dopamine, la vasopressine et/ou l'angiotensine II) ; et/ou des antagonistes de l'adénosine ; et/ou des antagonistes de l'aldostérone.
